# EUROPEAN PATENT SPECIFICATION

(11) **EP 2 321 279 B1**
(45) Date of publication and mention of the grant of the patent: **02.07.2014**
(21) Application number: 09777654.6
(22) Date of filing: 05.08.2009
(51) Int. Cl.: C07D 213/81, A61K 31/44, C07B 59/00

(54) **DAA-PYRIDINE AS PERIPHERAL BENZODIAZEPINE RECEPTOR LIGAND FOR DIAGNOSTIC IMAGING AND PHARMACEUTICAL TREATMENT**
DAA-PYRIDIN ALS LIGAND FÜR DEN PERIPHEREN BENZODIAZEPINREZEPTOR ZUR DIAGNOSTISCHEN BILDDARSTELLUNG UND PHARMAZEUTISCHEN BEHANDLUNG
DAA-PYRIDINE COMME LIGAND DES RÉCEPTEURS PÉRIPHÉRIQUES DES BENZODIAZÉPINES POUR L'IMAGERIE DE DIAGNOSTIC ET LE TRAITEMENT PHARMACEUTIQUE

(30) Priority: 06.08.2008 EP 08161903
(43) Date of publication of application: 18.05.2011
(73) Proprietor: Piramal Imaging SA, 1753 Matran (CH)
(72) Inventor: LEHMANN, Lutz, 10115 Berlin (DE); THIELE, Andrea, 13125 Berlin (DE); HEINRICH, Tobias, 13507 Berlin (DE); VOLLMER, Sonja, 14352 Kleinmachnow (DE)
(74) Representative: Kilger, Ute
(86) International application number: PCT/EP2009/005651
(87) International publication number: WO 2010/015387

(56) References cited:
- WO-A-2007/060517
- JP-A- 2000 001 476
- US-A1- 2004 138 310
- WILSON ET AL: "Radiosynthesis and initial evaluation of [<18>F]-FEPPA for PET imaging of peripheral benzodiazepine receptors" NUCLEAR MEDICINE AND BIOLOGY, ELSEVIER, NY, US, vol. 35, no. 3, 17 March 2008 (2008-03-17), pages 305-314, XP022537631 ISSN: 0969-8051

## Description

### Field of Invention

This invention relates to novel compounds suitable for labelling or already labelled by ¹⁸F, methods of preparing such a compound, compositions comprising such compounds, kits comprising such compounds or compositions and uses of such compounds, compositions or kits for therapy and diagnostic imaging by positron emission tomography (PET).

### Background art

Molecular imaging has the potential to detect disease progression or therapeutic effectiveness earlier than most conventional methods in the fields of oncology, neurology and cardiology. Of the several promising molecular imaging technologies having been developed such as optical imaging, MRI, SPECT and PET, PET is of particular interest for drug development because of its high sensitivity and ability to provide quantitative and kinetic data.

For example positron emitting isotopes include carbon, iodine, fluorine, nitrogen, and oxygen. These isotopes can replace their non-radioactive counterparts in target compounds to produce tracers that function biologically and are chemically identical to the original molecules for PET imaging. Among these isotopes ¹⁸F is the most convenient labelling isotope due to its relatively long half life (111 min) which permits the preparation of diagnostic tracers and subsequent study of biochemical processes. In addition, its low β+ energy (634 keV) is also advantageous.

The nucleophilic aromatic and aliphatic [¹⁸F]-fluoro-fluorination reaction is of great importance for [¹⁸F]-fluoro-labelled radiopharmaceuticals which are used as in vivo imaging agents targeting and visualizing diseases, e.g. solid tumours or diseases of brain. A very important technical goal in using [¹⁸F]-fluoro-labelled radiopharmaceuticals is the quick preparation and administration of the radioactive compound due to the fact that the ¹⁸F isotopes have a half-life of about only 111 minutes.

A couple of methods are known to introduce F-18 to an aromatic ring (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). One of the later discoveries is the replacement of an iodonium leaving group with [¹⁸F]fluoride, compare e.g. WO2005061415(A1), WO2005097713(A1), WO2007010534(A2), WO2007073200(A1) and WO2007141529(A1).

Peripheral benzodiazepine receptor (PBR) is expressed in most organs and its expression is reported to be increased in activated microglia in the brain which are the smallest type of glial cells acting as the immune cells of the central nervous system (CNS). Microglia are related to other phagocytic cells including macrophages and dendritic cells. Microglia are thought to be highly mobile cells that play numerous important roles in protecting the nervous system. They are also thought to play a role in neurodegenerative disorders such as Alzheimer's disease, dementia, multiple sclerosis and Amyotrophic lateral sclerosis. Microglia are responsible for producing an inflammatory reaction to insults (J. Neuroinflammation, 2004, Jul 30;1(1):14.).

It is an important goal for the design of a sufficient CNS-PET tracer that the pharmacokinetics in the brain is optimized. Thus, the PET ligand should enter the brain rapidly in sufficient amount. A high fraction of these molecues should then bind tightly to the target. Subsequently those molecules which have not bound should be eliminated from the surrounding area ("wash-out" from the brain) in order to achieve an image with a high signal to background ratio.

The C-11 isotope labeled version of PK11195 **(1a)** has been widely used for the in vivo imaging of neuroinflamation and PBRs, but its signal in the brain was not high enough for stable quantitative analysis.

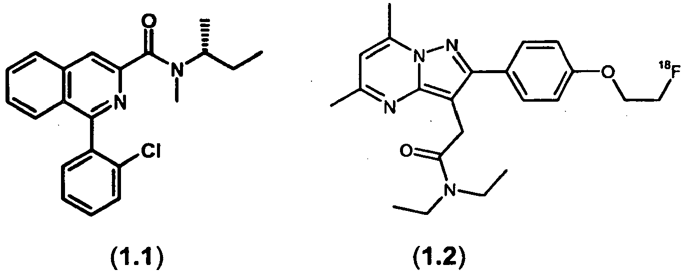

Furthermore, it has been shown that the development of superior positron-emitting ligands, like [¹⁸F]DPA714 **(1.2),** [¹¹C]DAA1106 **(2)** (e.g. Eur J Pharmacol. 1999 Apr 29;371(2-3):197-204 and Life Sci. 1999;64(16):1455-64) and [¹⁸F]fluoroethyl-DAA1106 (3) (e.g. J. Nucl. Med., (2006), 47, 43-50), for visualization of PBRs is possible: The compounds **2** and **3** have a higher binding affinity to PBR and a higher accumulation in the brain than [¹¹C]PK11195 **(1.1a).**

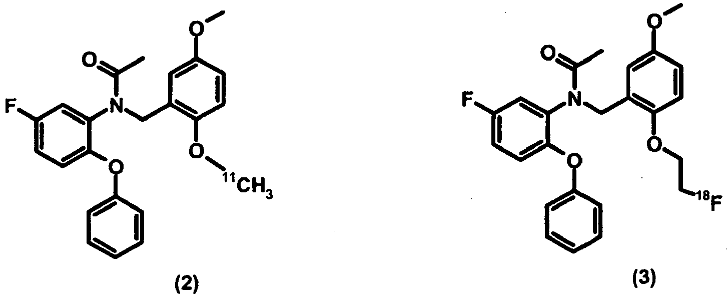

The non-radioactive version of compound **2** is claimed by the patent family related to WO99/006353, whereas the compound **3** is claimed by the patent family related to US 6,870,069.

Recently, new [F-18] and [C-11] labelled PBR ligands has been published, called [¹⁸F]FEPPA, **(4)** and **(5),** respectively (Nuclear Medicine and Biology, 35, (2008), 305-314 and J. Med. Chem. (2008), 51, 17-30, respectively). "[¹⁸F]-FEPPA [compound **4**] showed moderate brain uptake [standard uptake value (SUV) of 0.6 at 5 min] and a slow washout (SUV of 0.35 after 60 min)" (cited from Nuclear Medicine and Biology, 35, (2008)). Thus, compound **4** leads to an image with a relatively low to signal-to-noise ratio.

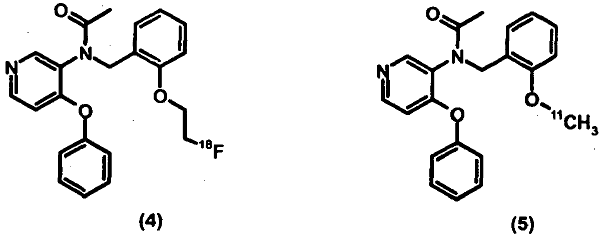

Derivatives of such kind have been also covered by patent application WO2007/060157 and members of the corresponding patent family.

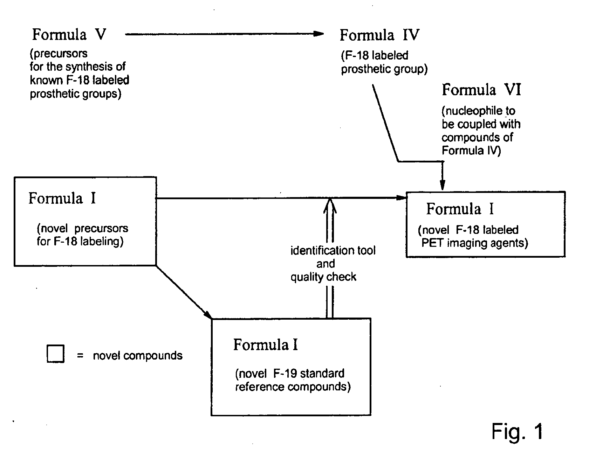

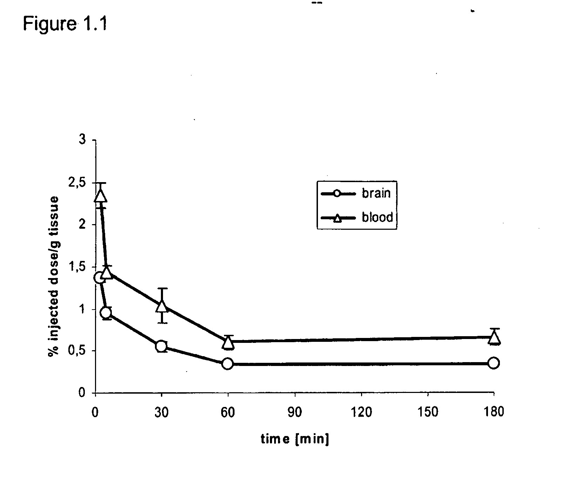

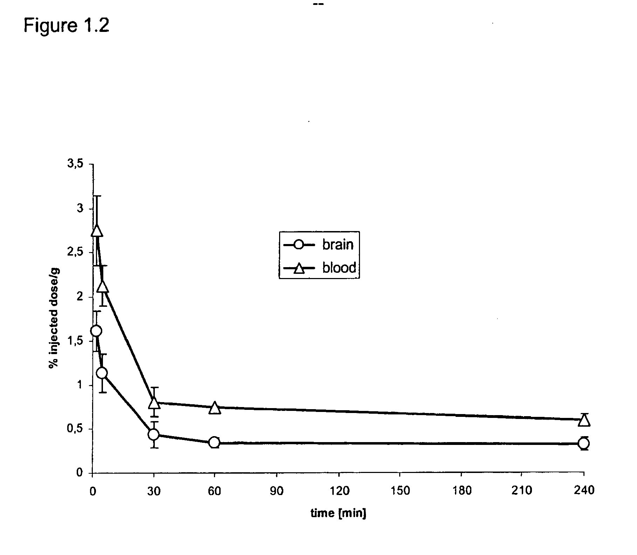

It would be desirable to have new F-18 labeled compounds and methods available to image diseases which go along with increased level of PBR receptor, especially to have imaging agents and methods available which are easy to realize and which are able to image certain levels of PBR receptor with a sufficient signal to background ratio. This task is solved with the following invention (compare fig. 1):
■ The present invention provides novel compounds of Formula I. If these compounds of Formula I are not ¹⁸F-labelled or ¹⁹F-labelled, but instead contain an appropriate leaving group, they are starting materials for the synthesis of ¹⁸ F-labelled or ¹⁹F-labelled compounds of Formula I. ¹⁹F-labelled compounds of Formula I are standard reference compounds (as identification tool and for quality check) of the synthesis towards ¹⁸F-labelled compounds of Formula I. In the following compounds of Formula I which contain an appropriate leaving group and do not contain ¹⁸F or ¹⁹F, are also referred to as "precursor compounds having formula I". Moreover, those compounds of formula I that contain ¹⁹F instead of an appropriate leaving group are also referred to as "¹⁹F standard reference compounds having formula I". Moreover, those compounds of Formula I which contain ¹⁸F and which do not contain an appropriate leaving group are also referred to as "¹⁸F-labelled compounds of formula I".
■ The invention further provides a method of imaging diseases, the method comprising introducing into a patient a detectable quantity of a ¹⁸F-labeled compound of Formula I or a pharmaceutically acceptable salt, ester, amide or prodrug thereof.
■ The invention provides also ¹⁸F-labelled or ¹⁹F-labelled compounds of Formula I for use as medicament.
■ The present invention also provides diagnostic compositions comprising a radiolabeled compounds preferably ¹⁸F-labelled compounds of Formula I, and a pharmaceutically acceptable carrier or diluent.
■ Another aspect of the invention is directed to the use of compounds of Formula I for the manufacture of a medicament, in particular of ¹⁸F- or ¹⁹-F-labelled compounds of formula I.
■ The invention also provides a process to synthesize ¹⁸F-labelled compounds of Formula I from precursor compounds having formula I.
■ The invention also provides a process to synthesize ¹⁹F-labelled compounds of Formula I from precursor compounds having formula I.
■ The present invention provides novel compounds of Formula VI. These compounds serve as precursor compounds towards compounds of formula I by reacting compounds of Formula IV with compounds of Formula VI. Compounds of formula IV can be generated by ¹⁸F- or ¹⁹F-fluorinating a compound of formula V.
■ The invention also provides a process to synthesize ¹⁸F-labelled compounds of formula I by reacting compounds of Formula IV with compounds of Formula VI. Compounds of formulae IV can be generated by ¹⁸F- or ¹⁹F-fluorinating a compound of formula V.
■ The invention also provides a kit for preparing a radiopharmaceutical preparation, said kit comprising a sealed vial containing a predetermined quantity of
   ∘ a precursor compound having formula I, or
   ∘ compounds of Formula V and VI.
■ The present invention also discloses a kit for imaging diseases. More specifically the compounds of this invention are useful for the imaging of CNS diseases including but not limited to inflammatory and autoimmune, allergic, infectious and toxin-triggered and ischemia-triggered diseases, pharmacologically triggered inflammation with pathophysiological relevance, neuroinflammatory, neurodegenerative diseases. In another embodiment the compounds of this invention are useful for the imaging of tissue, in particular tumors. Examples of inflammatory and autoimmune diseases are chronic inflammatory intestinal diseases (inflammatory bowel diseases, Crohn's disease, ulcerative colitis), arthritis, atheroma, atherosclerosis, inflammatory cardiomyopathy, pemphigus, asthma, multiple sclerosis, diabetes, type I insulin-dependent diabetes mellitus, rheumatoid arthritis, lupus diseases and other collagenoses, Graves' disease, Hashimoto's disease, "graft-versus-host disease" and transplant rejections. Examples of allergic, infectious and toxin-triggered and ischemia-triggered diseases are: sarcoidosis, asthma, hypersensitive pneumonitis, sepsis, septic shock, endotoxin shock, toxic shock syndrome, toxic liver failure, ARDS (acute respiratory distress syndrome), eclampsia, cachexia, acute viral infections (e.g., mononucleosis, fulminant hepatitis), and post-reperfusion organ damage. An example of a pharmacologically triggered inflammation with pathophysiological relevance is the "first dose response" after administration of anti-T-cell antibodies such as OKT3. An example of systemic inflammation reactions of an origin that is as yet unclear is eclampsia. Examples of neurodegenerative and neuroinflammatory diseases that are associated with a PBR regulation are dementia, AIDS dementia, amyotrophic lateral sclerosis, encephalitis, neuropathic pain, Creutzfeldt-Jakob disease, Down's syndrome, diffuse Lewy body disease, Huntington's disease, leukoencephalopathy, encephalopathies, septic encephalopathy, hepatic encephalopathy, multiple sclerosis, Parkinson's disease, Pick's disease, Alzheimer's disease, frontotemporal dementia, hippocampal sclerosis, neurocysticercosis, epilepsy, stroke, ischemia, brain tumors, depression, schizophrenia, drug abuse. The invention, therefore, also relates to the use of imaging compounds for diagnosing these diseases as well as for stratification of therapy and therapy monitoring.

In a preferred embodiment compounds of this invention are useful for the imaging of multiple sclerosis, Alzheimer's disease, frontotemporal dementia, dementia with Levy bodies, leukoencephalopathy, epilepsy, neuropathic pain, amyotrophic lateral sclerosis, Parkinson's Disease, encephalopathies, brain tumors, depression, drug abuse, chronic inflammatory intestinal diseases, atheroma, atherosclerosis, arthritis, rheumatoid arthritis, pharmacologically triggered inflammation, systemic inflammation of unclear origin.

In a more preferred embodiment compounds of this invention are useful for the imaging of multiple sclerosis, Alzheimer's disease, amyotrophic lateral sclerosis, Parkinson's Disease, leukoencephalopathy, encephalopathies, epilepsy, brain tumors, drug abuse, chronic inflammatory intestinal diseases, atheroma, rheumatoid arthritis, pharmacologically triggered inflammation and systemic inflammation of unclear origin.

JP 2000-001476 describes similar compounds as disclosed here and their use for treatment of diseases.

### Detailed Description of the Invention:

In a ***first*** aspect the present invention is directed to compounds of formula I 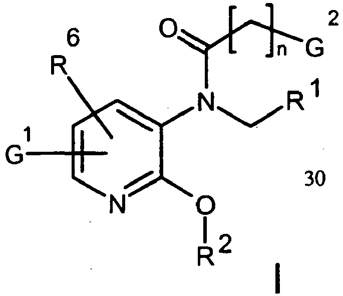 wherein
**R¹** and **R²** are independently and individually, at each occurrence, selected from the group comprising (G³)aryl, substituted (G³)aryl, (G³-(C₁-C₈)alkyl)aryl, (G³-(C₁-C₈)alkoxy)aryl, (G³-(C₂-C₈)alkynyl)aryl, (G³-(C₂-C₈)alkenyl)aryl, substituted (G³-(C₁-C₈)alkyl)aryl, substituted (G³-(C₁-C₈)alkoxy)aryl, substituted (G³-(C₂-C₈)alkynyl)aryl and substituted (G³-(C₂-C₈)alkenyl)aryl;
**G¹, G²** and **G³** are independently and individually, at each occurrence, selected from the group comprising hydrogen and **L,**
   with the proviso that compounds of formula I contain exactly one **L;**
**L** is selected from the group comprising **R³,** [¹⁸F]fluoro and [¹⁹F]fluoro;
**R³** is a leaving group;
**R⁶** is selected from the group comprising hydrogen, halo, trifluoromethyl, (C₁-C₅)alkyl, (C₂-C₅)alkynyl), (C₂-C₅)alkenyl and (C₁-C₅)alkoxy;
wherein **n** is an integer from 0 to 6, preferably 0 to 2, more preferably 0 to 1, even more preferably 1;
including all isomeric forms of said compound, including but not limited to enantiomers and diastereoisomers as well as racemic mixtures,
and any pharmaceutically acceptable salt, ester, amide or complex thereof.

In a preferred embodiment **R¹** and **R²** in formula I are independently and individually, at each occurrence, selected from the group comprising (G³)phenyl, (G³-(C₁-C₅)alkyl)phenyl, (G³-(C₁-C₅)alkoxy)phenyl, (G³-(C₂-C₅)alkynyl)phenyl, (G³-(C₂-C₅)alkenyl)phenyl, substituted (G³)phenyl, substituted (G³-(C₁-C₅)alkyl)phenyl, substituted (G³-(C₁-C₅)alkoxy)phenyl, substituted (G³-(C₂-C₅)alkynyl)phenyl and substituted (G³-(C₂-C₅)alkenyl)phenyl;
in a more preferred embodiment **R¹** and **R²** in formula I are independently and individually, at each occurrence, selected from the group comprising (R⁴)(R⁵)(G³)phenyl, (R⁴)(R⁵)(G³-(C₁-C₄)alkyl)phenyl, (R⁴)(R⁵)(G³ -(C₁-C₄)alkoxy)phenyl, (R⁴)(R⁵)(G³-(C₂-C₄)alkenyl)phenyl and (R⁴)(R⁵)(G³-(C₂-C₄)alkynyl)phenyl,
in an even more preferred embodiment **R¹** and **R²** in formula I are independently and individually, at each occurrence, selected from the group comprising (R⁴)(R⁵)(G³)phenyl, (R⁴)(R⁵)(G³-(C₂-C₃)alkyl)phenyl and (R⁴)(R⁵)(G³-(C₂-C₃)alkoxy)phenyl;
in the most preferred embodiment **R¹** and **R²** in formula I are independently and individually, at each occurrence, selected from the group comprising (R⁴)(R⁵)(G³)phenyl and (R⁴)(R⁵)(G³-(C₂-C₃)alkoxy)phenyl;
wherein **R⁴** and **R⁵** are independently and individually, at each occurrence, selected from the group comprising hydrogen, halo, trifuoromethyl, (C₁-C₅)alkyl, (C₂-C₅)alkynyl), (C₂-C₅)alkenyl and (C₁-C₅)alkoxy;
in a preferred embodiment **R⁴** and **R⁵** are independently and individually, at each occurrence, selected from the group comprising hydrogen, fluoro, chloro, methyl, methoxy and trifluoromethyl;
in an even more preferred embodiment **R⁴** and **R⁵** are independently and individually, at each occurrence, selected from the group comprising hydrogen, fluoro, methyl, and methoxy;
in a preferred embodiment **R⁶** is selected from the group comprising hydrogen, fluoro, chloro, methyl, methoxy and trifluoromethyl;
in a more preferred embodiment **R⁶** is selected from the group comprising hydrogen, fluoro chloro and methyl;
in an even more preferred embodiment **R⁶** is selected from the group comprising hydrogen and chloro;
in the most preferred embodiment **R⁶** is hydrogen;
in one embodiment **L** is [¹⁸F]fluoro;
in one embodiment **L** is [¹⁹F]fluoro;
in one embodiment **L** is R³;
in a preferred embodiment **R³** is selected from the group comprising - I⁺(aryl)(X⁻), -I⁺(heteroaryl)(X⁻), nitro, -N⁺(Me)₃(X⁻), halo, in particular chloro, bromo and iodo, mesyloxy, tosyloxy, trifluormethylsulfonyloxy, nona-fluorobutylsulfonyloxy, (4-bromo-phenyl)sulfonyloxy, (4-nitro-phenyl)sulfonyloxy, (2-nitro-phenyl)sulfonyloxy, (4-isopropylphenyl)sulfonyloxy, (2,4,6-tri-isopropyl-phenyl)sulfonyloxy, (2,4,6-trimethyl-phenyl)sulfonyloxy, (4-tertbutyl-phenyl)sulfonyloxy, and (4-methoxy-phenyl)sulfonyloxy;
in a more preferred embodiment **R³** is selected from the group comprising nitro, -N⁺(Me)₃(X⁻), halo, in particular chloro, bromo and iodo, mesyloxy, tosyloxy, trifluormethylsulfonyloxy, nona-fluorobutylsulfonyloxy, (4-bromo-phenyl)sulfonyloxy, (4-nitro-phenyl)sulfonyloxy, (2-nitro-phenyl)sulfonyloxy, (4-isopropyl-phenyl)sulfonyloxy, (2,4,6-tri-isopropyl-phenyl)sulfonyloxy, (2,4,6-trimethyl-phenyl)sulfonyloxy, (4-*tert*butyl-phenyl)sulfonyloxy, and (4-methoxy-phenyl)sulfonyloxy;
in an even more preferred embodiment **R³** is selected from the group comprising nitro, -N⁺(Me)₃(X⁻), chloro, bromo, mesyloxy and tosyloxy;
wherein **X⁻** is selected from the group comprising
anion of an inorganic acid and anion of an organic acid;
in a preferred embodiment **X⁻** is selected from the group comprising CF₃S(O)₂O⁻, C₄F₉S(O)₂O⁻, CF₃COO⁻, H₃CCOO⁻, iodide anion, bromide anion, chloride anion, perchlorate anion (ClO₄⁻), and phosphate anion;
in an even more preferred embodiment **X⁻** is selected from the group comprising CF₃S(O)₂O⁻, C₄F₉S(O)₂O⁻, iodide anion, bromide anion and CF₃COO⁻.

The term "anion of inorganic or organic acids" as employed herein refers to the corresponding base of mineral acids, including but not limited to: acids such as carbonic, nitric or sulphuric acid, hydrogen chloride, hydrogen bromide, hydrogen iodide, phosphoric acid, perchloric acid or to the corresponding base of appropriate organic acids which includes but not limited to: acids such as aliphatic, cycloaliphatic, aromatic, araliphatic and heterocyclic carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic, 1,1,2,2,3,3,4,4,4-nonafluorobutane-1-sulfonic acid and sulfanilic acid.

The term "corresponding base" as employed herein refers to an acid being dissociated after the proton is donated.

In one embodiment of general formula I, **L** is **R³;** these are the aforementioned "precursor compounds".

Preferred "precursor compounds having formula I" are

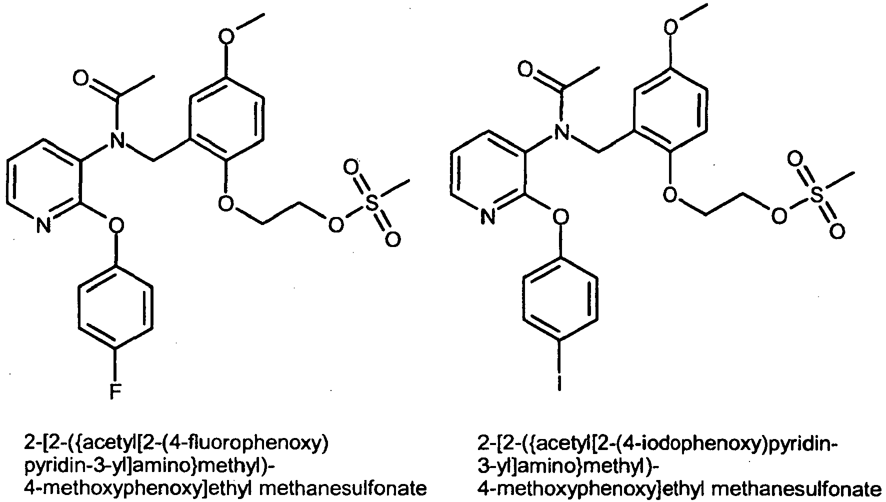

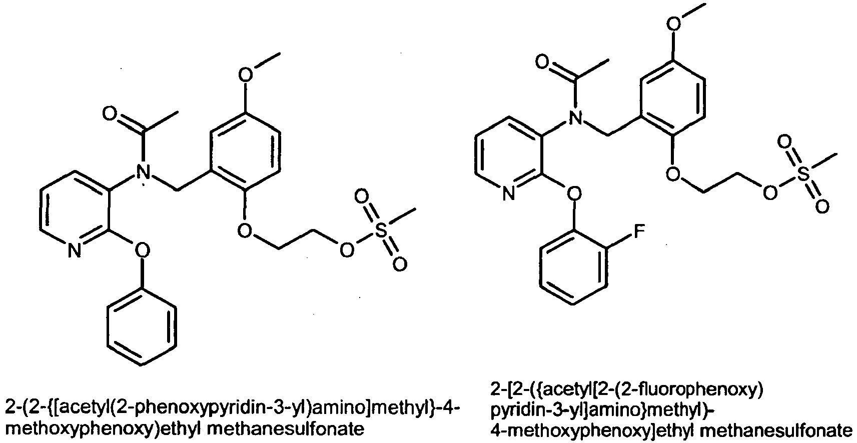

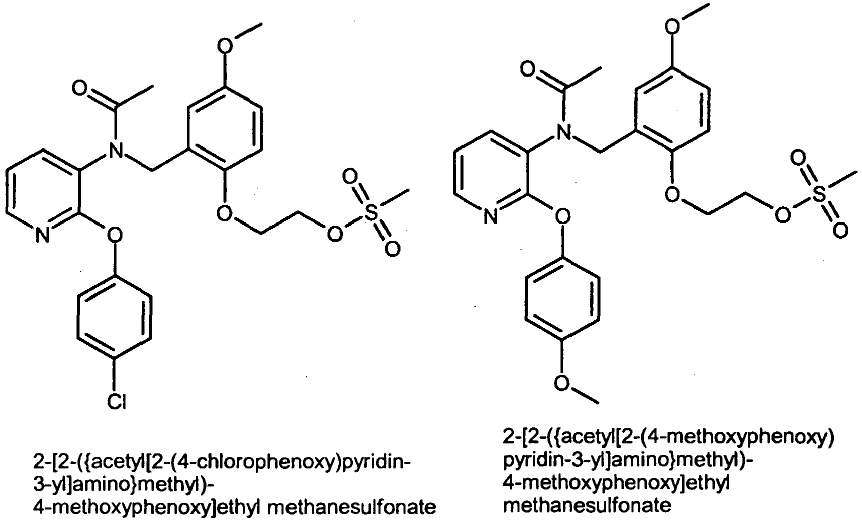

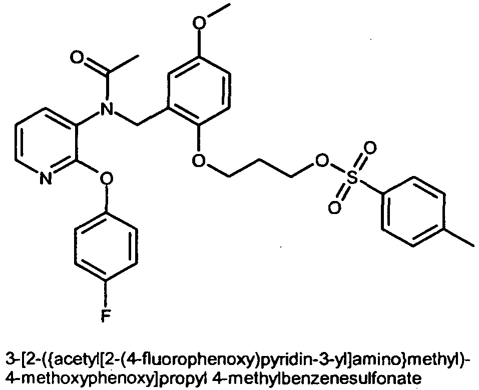

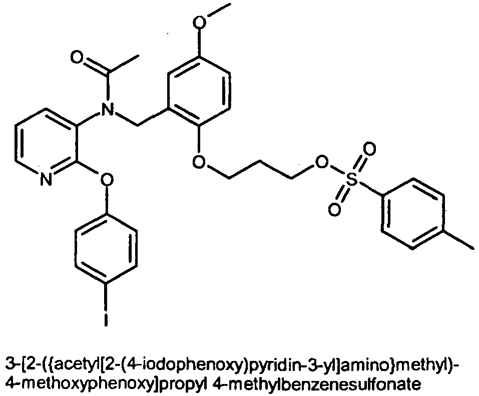

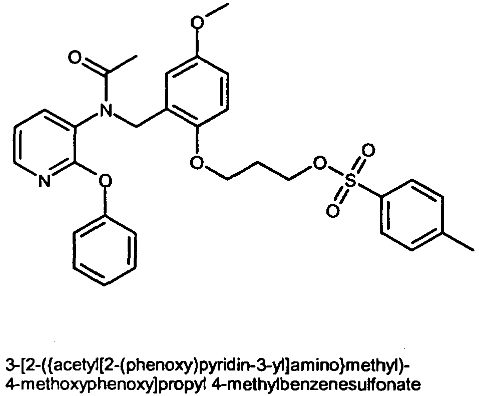

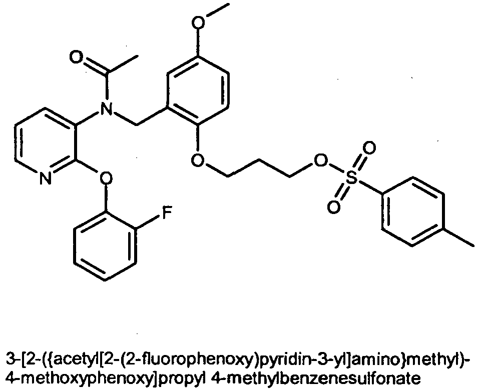

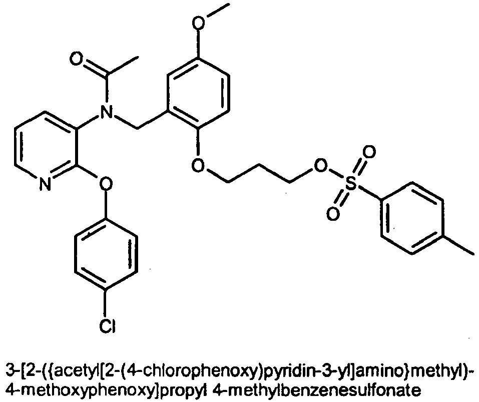

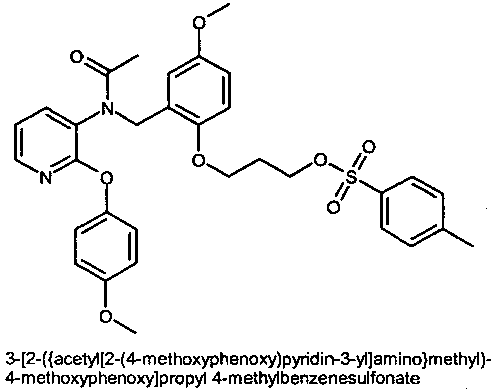

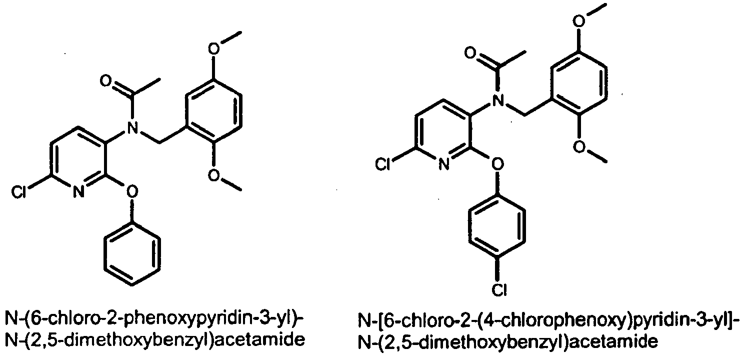

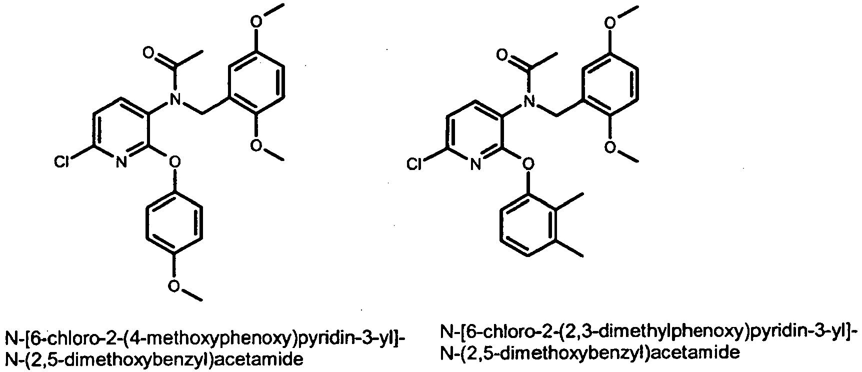

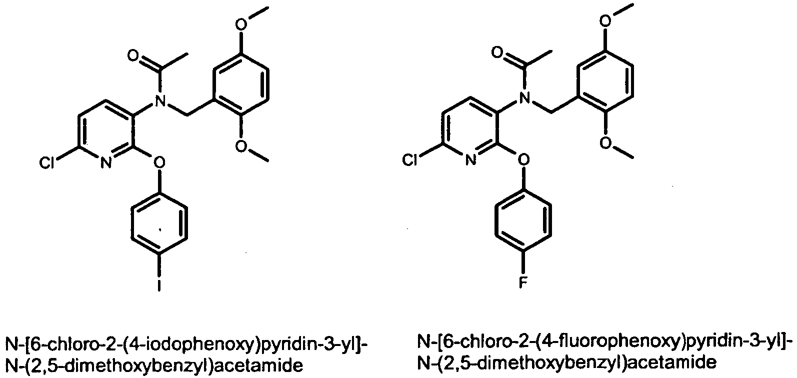

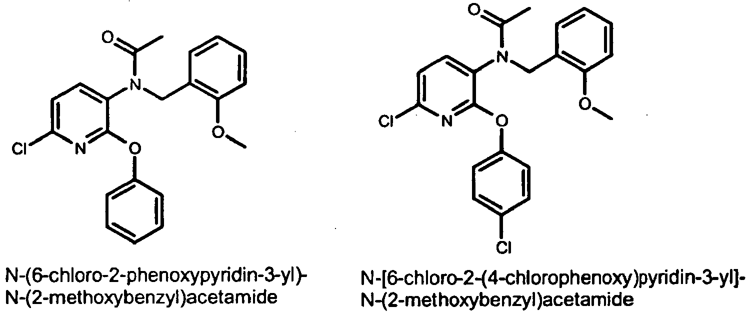

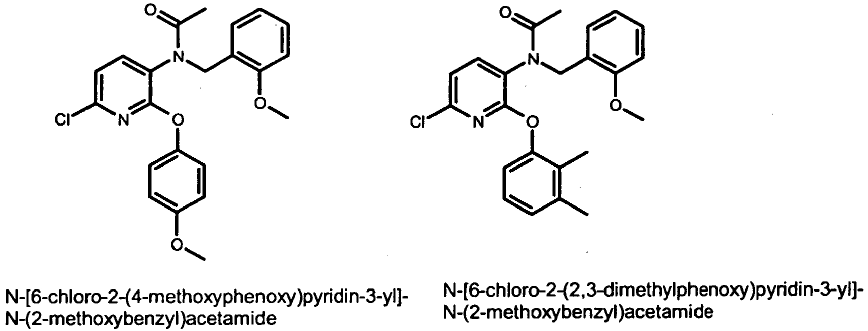

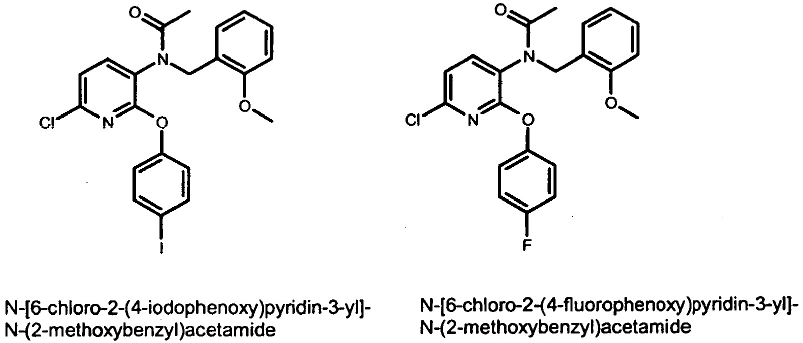

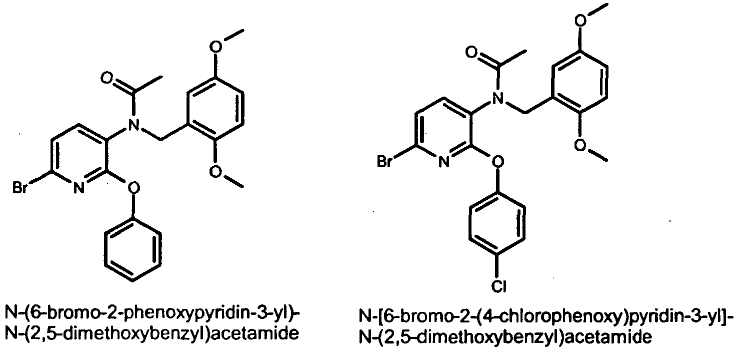

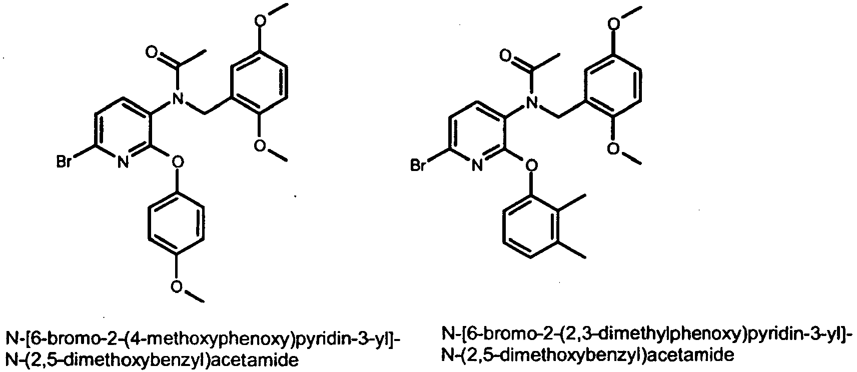

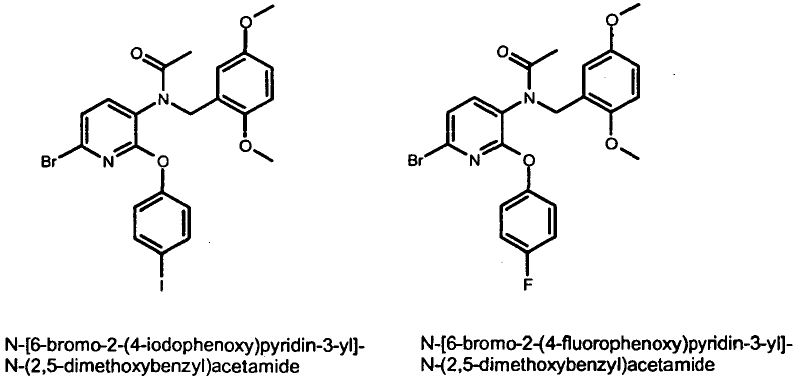

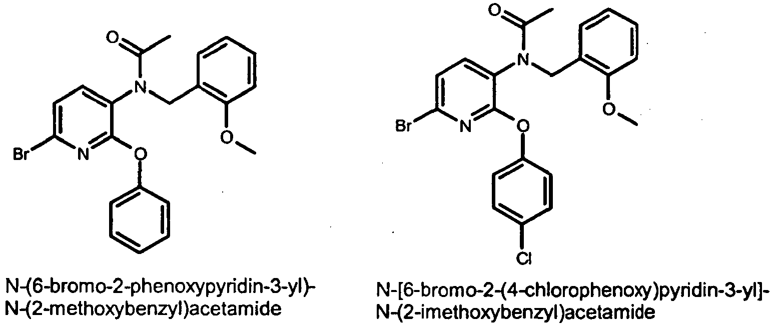

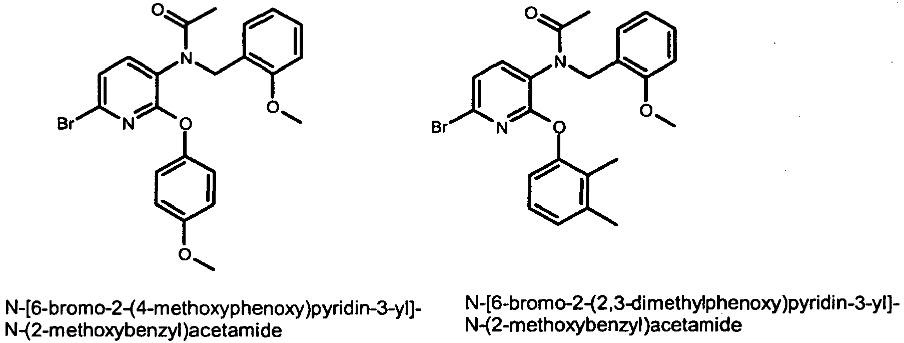

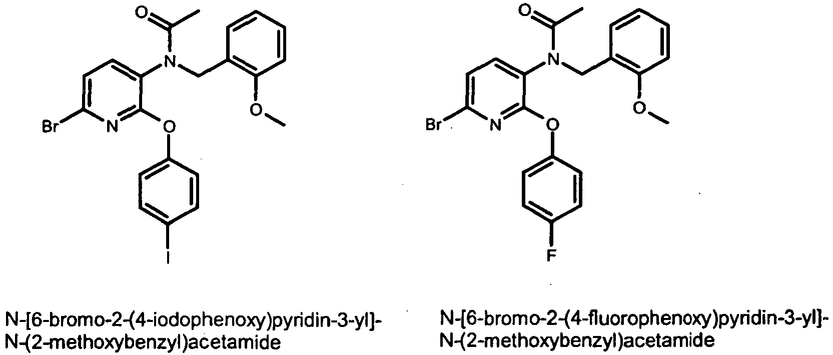

In another embodiment of general formula I, L is [¹⁸F]fluoro, these are the ¹⁸F-labelled compounds having formula I.

Preferred "F-18 labelled compounds having formula I" are

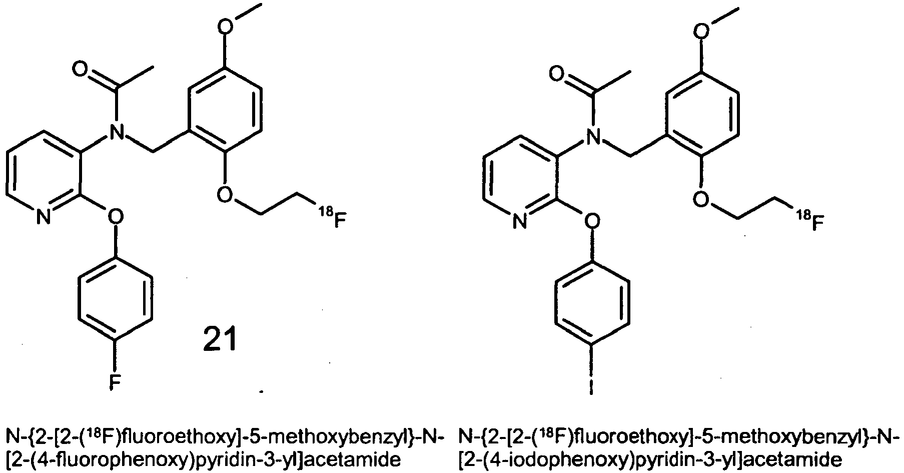

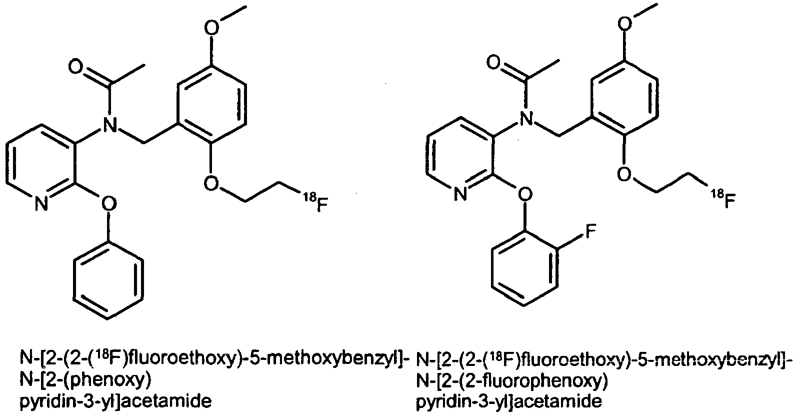

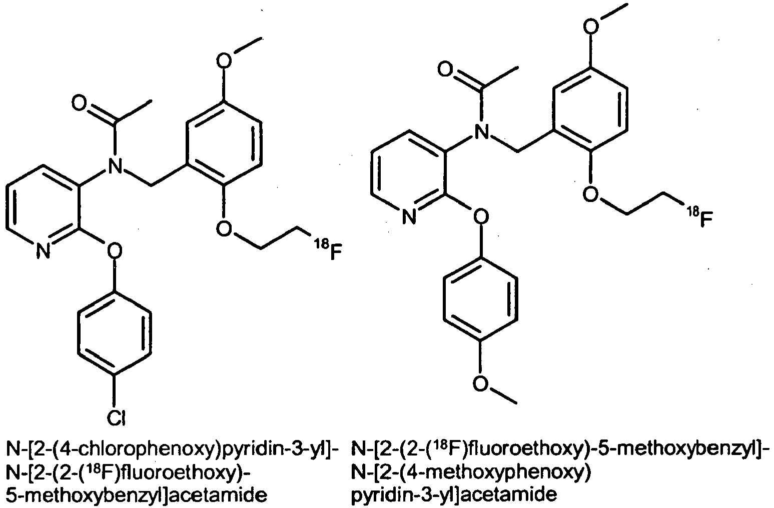

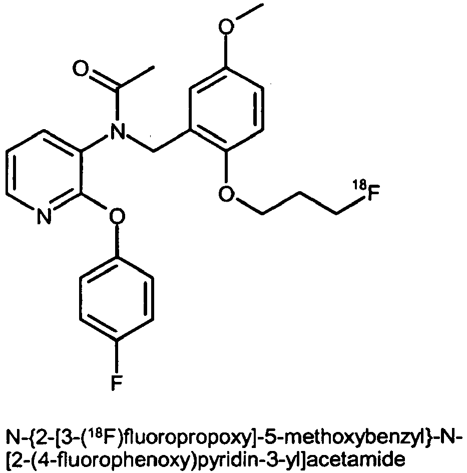

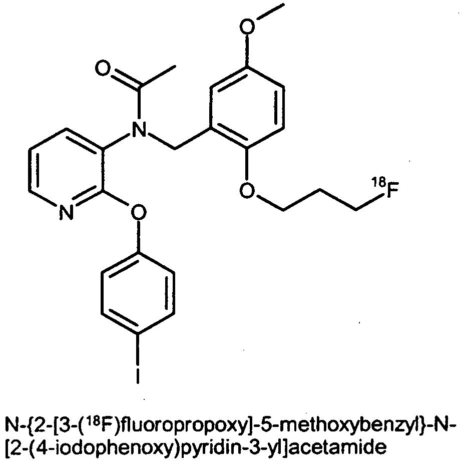

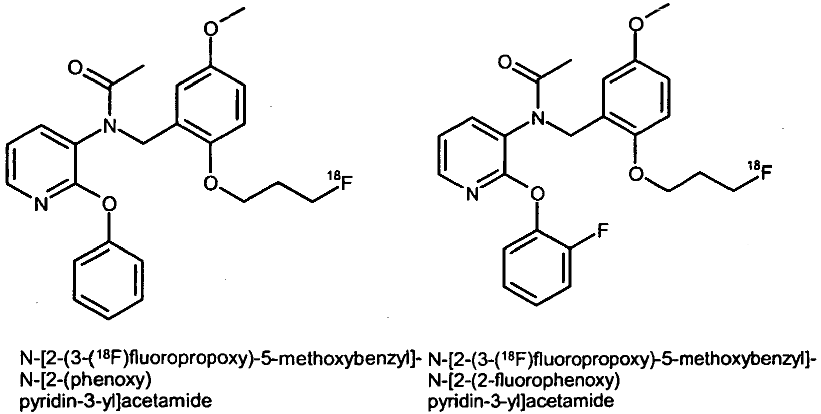

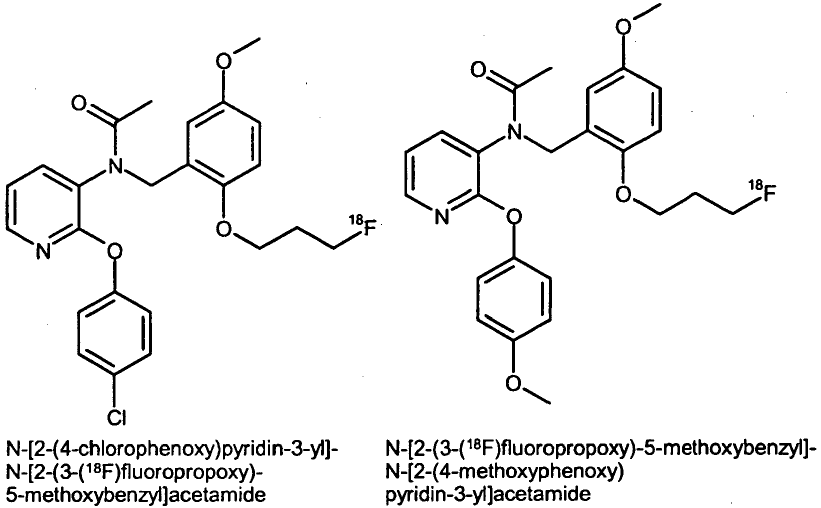

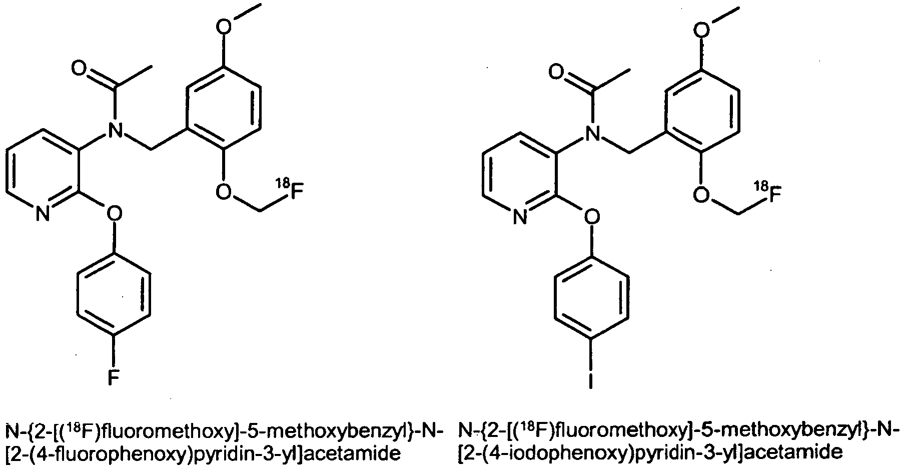

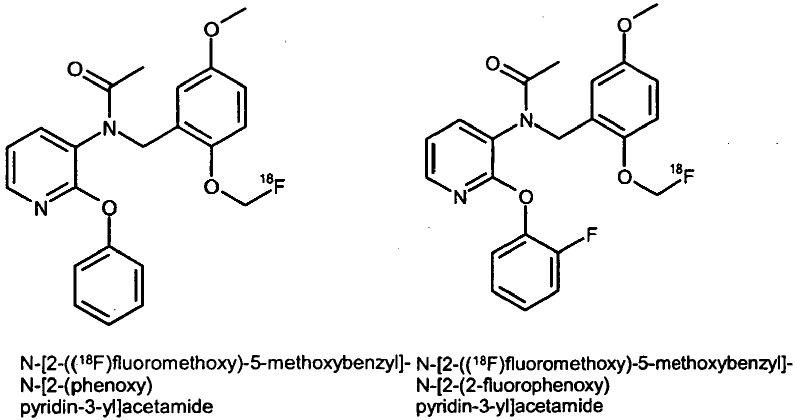

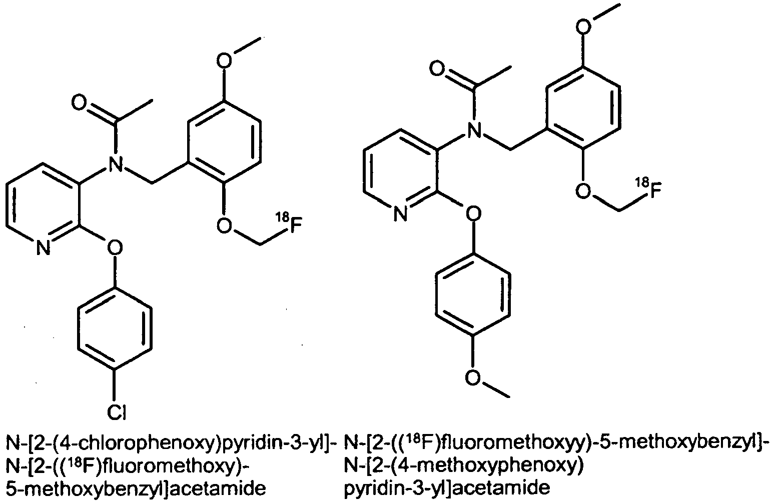

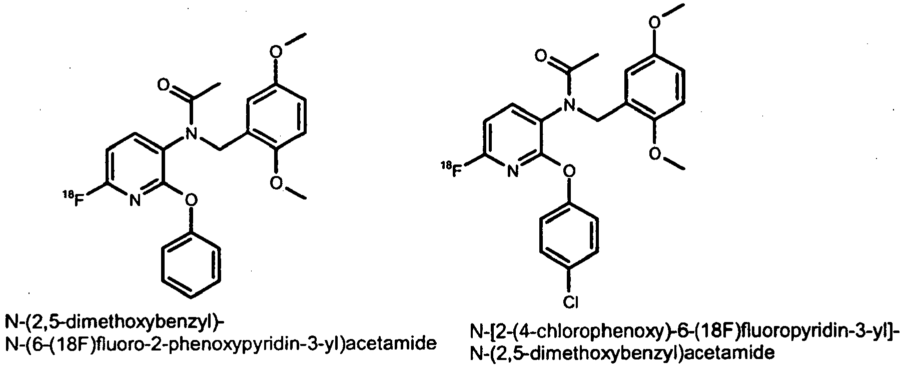

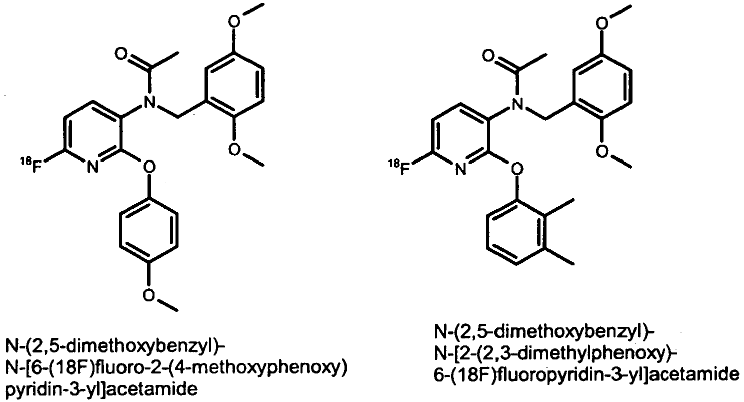

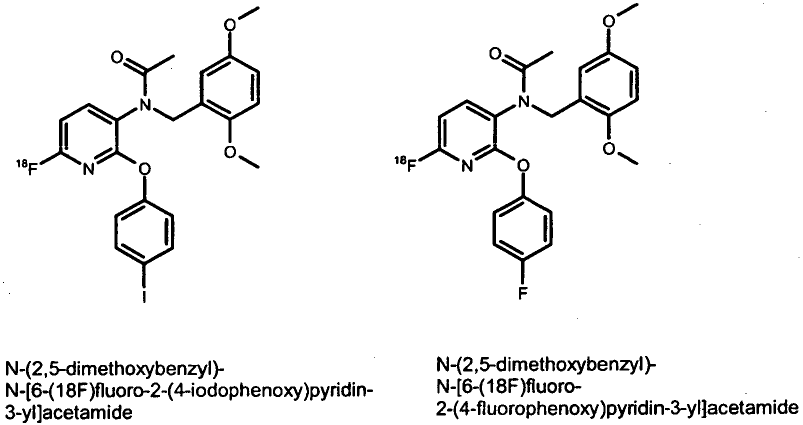

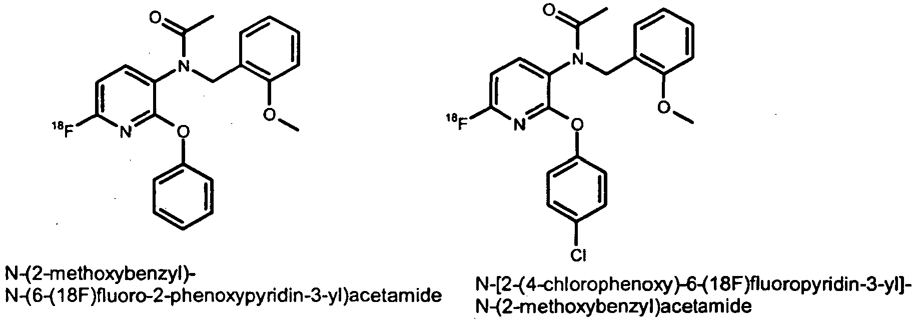

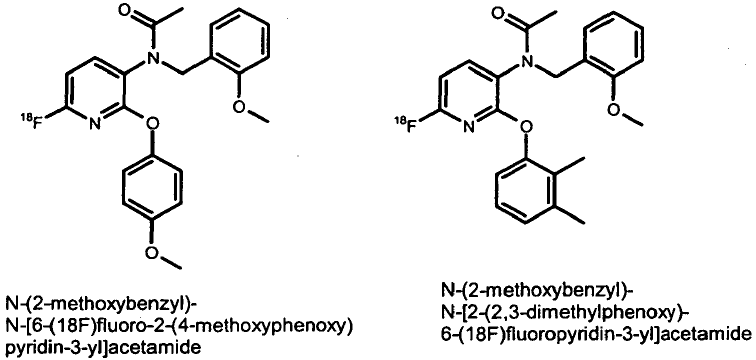

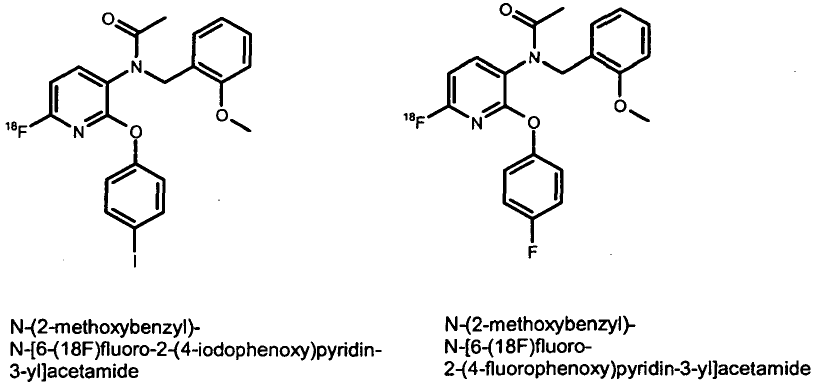

In yet another embodiment of general formula I, L is [¹⁹F]fluoro, these are the aforementioned "standard reference compounds having formula I".

Preferred "standard reference compounds having formula I" are

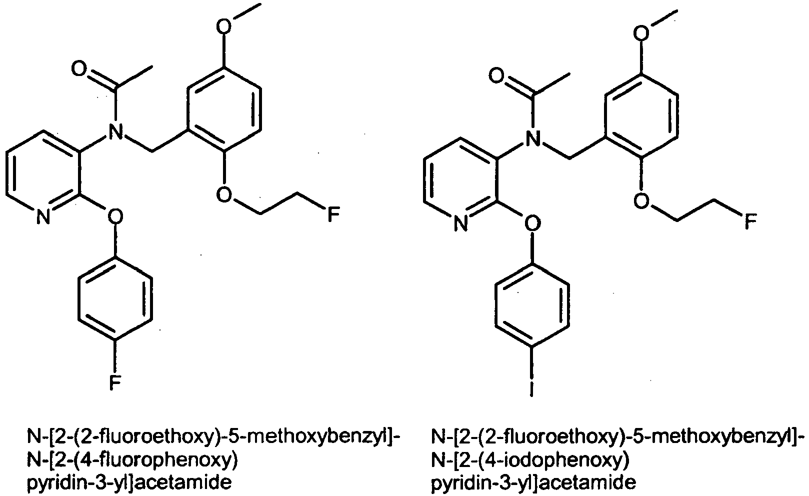

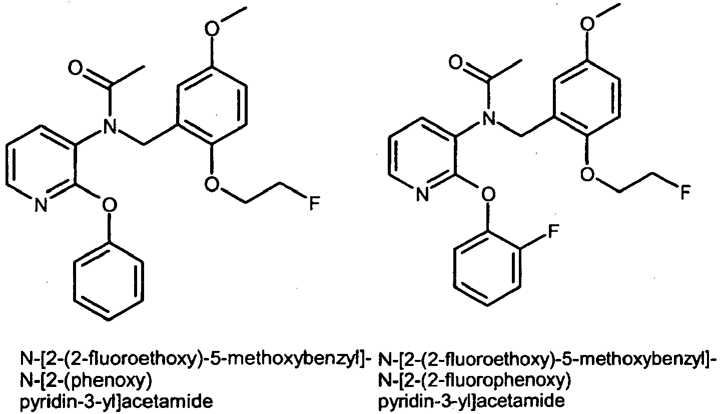

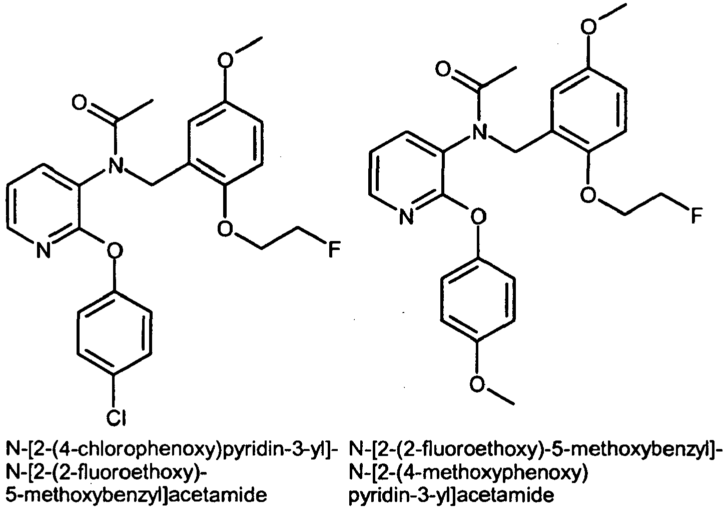

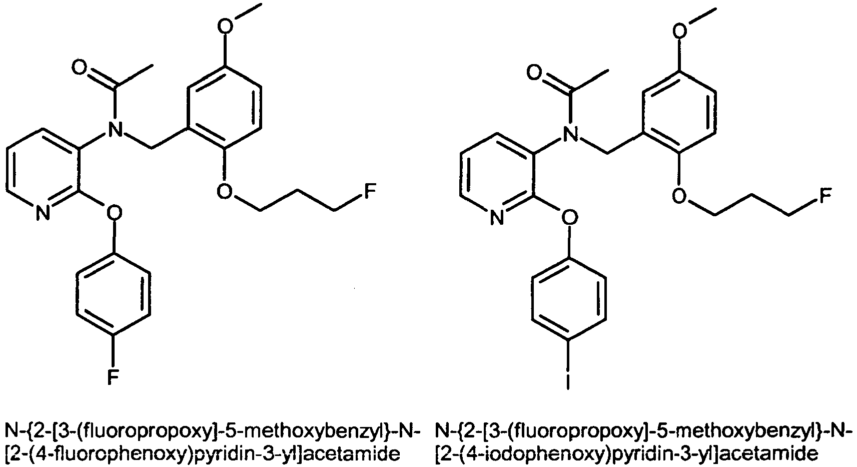

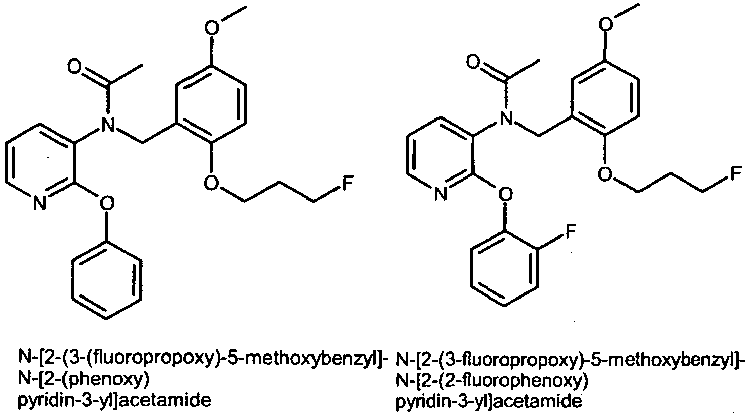

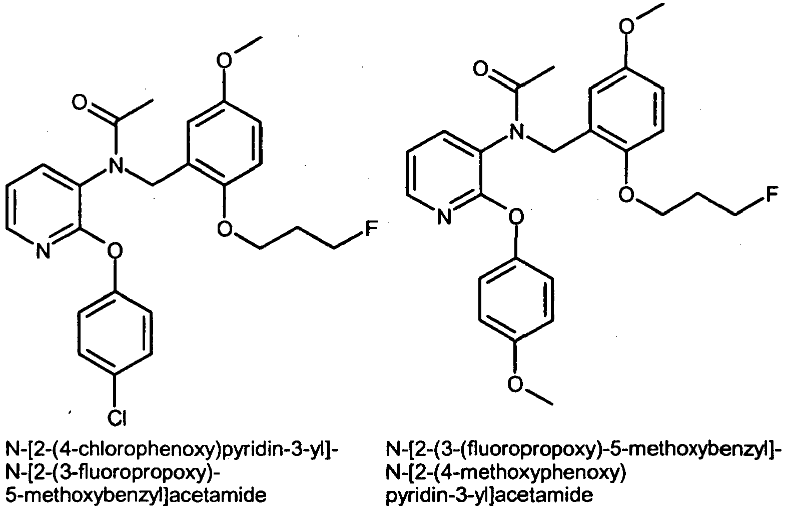

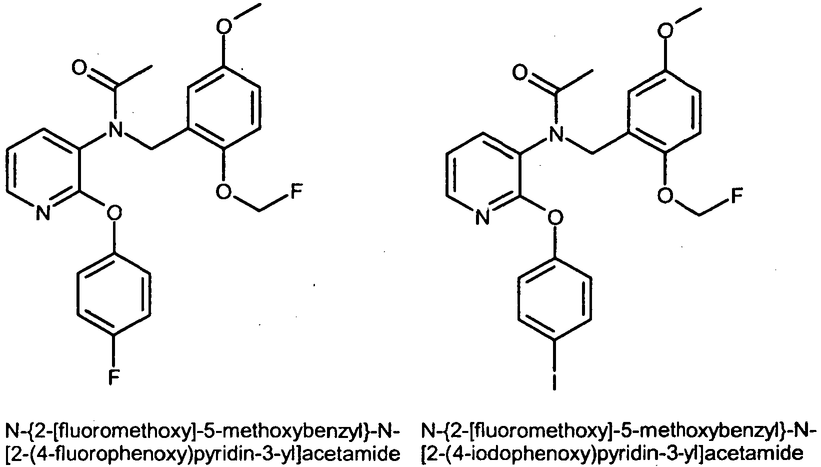

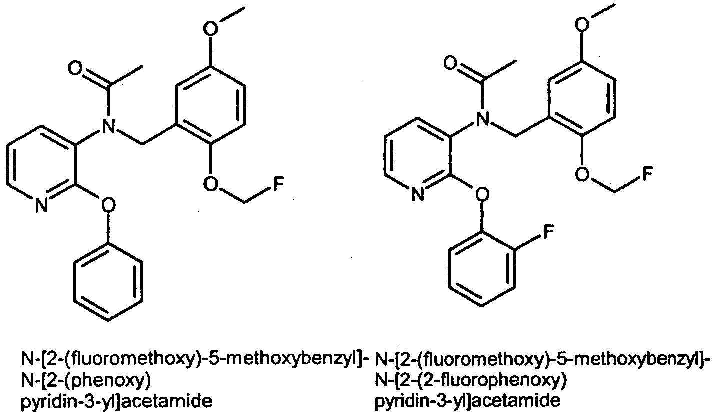

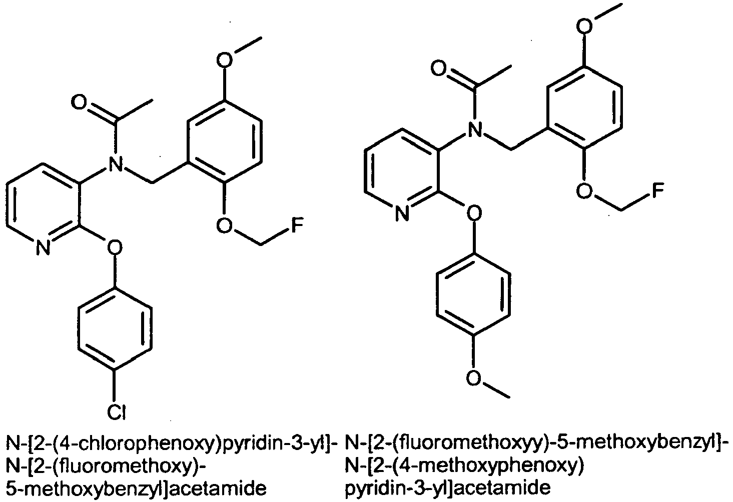

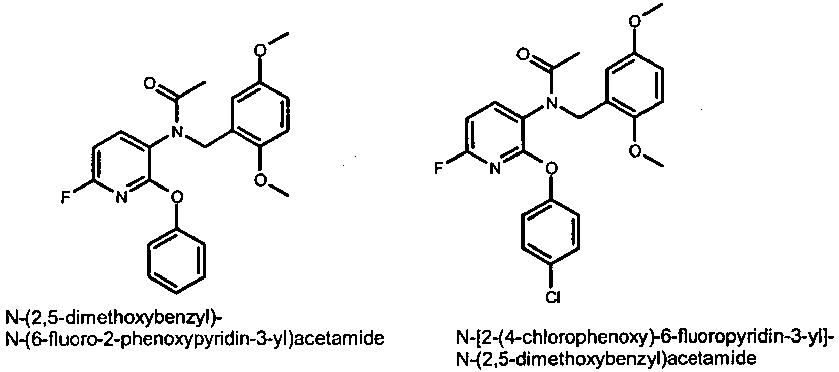

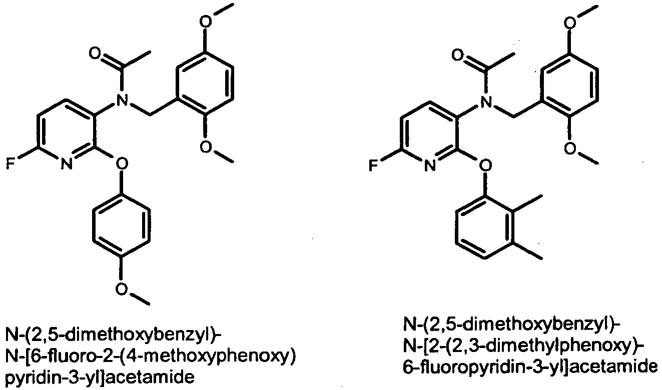

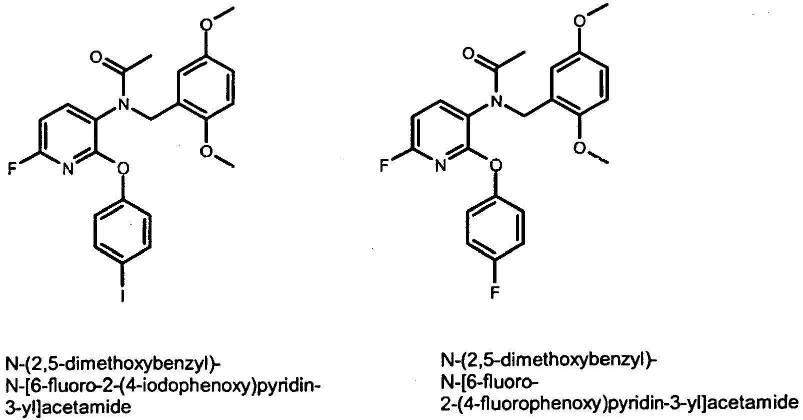

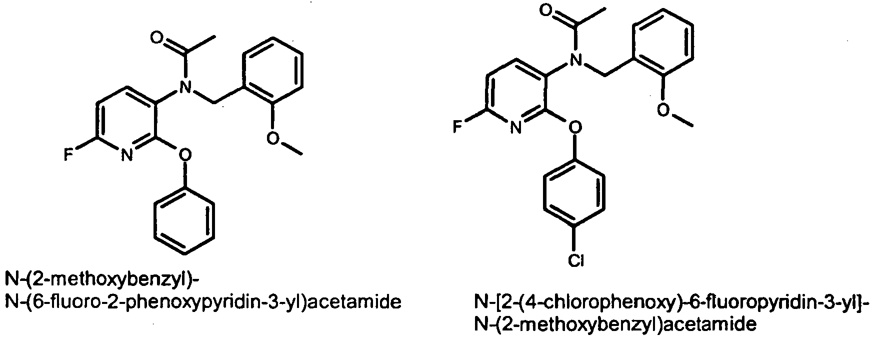

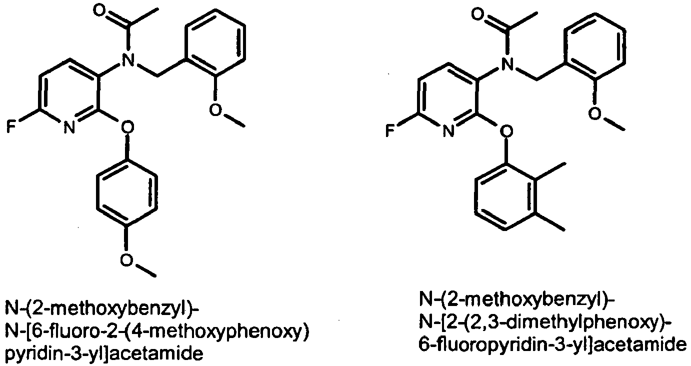

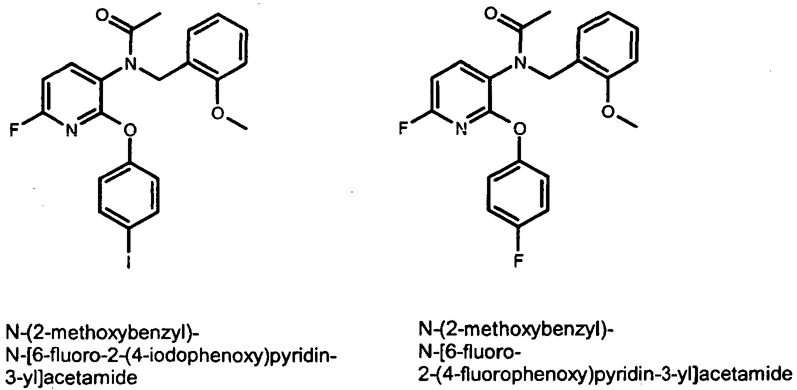

**R³** is a leaving group which is known or obvious to someone skilled in the art and which is taken from but not limited to those described or named in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O- nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Fig 7 pp 33).

It should be clear that wherever in this description the terms "aryl", "heteroaryl" or any other term referring to an aromatic system is used, this also includes the possibility that such aromatic system is substituted by one or more appropriate substituents, such as OH, halo, (C₁-C₆)alkyl, CF₃, CN, (C₁-C₆)alkenyl, (C₁-C₆)alkynyl, (C₁-C₆)alkoxy, NH₂, NO₂, S(O)₂OH, -S(O)₂NH₂ etc.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl, which themselves can be substituted with one, two or three substituents independently and individually selected from the group comprising halo, nitro, ((C₁-C₆)alkyl)carbonyl, cyano, nitrile, hydroxyl, trifluormethyl, ((C₁-C₆)alkyl)-sulfonyl, (C₁-C₆)alkyl, (C₁-C₆)alkoxy and ((C₁-C₆))alkylsulfanyl. As outlined above such "aryl" may additionally be substituted by one or several substituents.

The term "heteroaryl" as employed herein refers to groups having 5 to 14 ring atoms; 6, 10 or 14 Π (pi) electrons shared in a cyclic array; and containing carbon atoms (which can be substituted with halo, nitro, (C₁-C₆)carbonyl, cyano, nitrile, trifluormethyl, (C₁-C₆)sulfonyl, (C₁-C₆)alkyl, (C₁-C₆)alkoxy or (C₁-C₆)sulfanyl) and 1, 2, 3 or 4 oxygen, nitrogen or sulfur heteroatoms (where examples of heteroaryl groups are: thienyl, benzo[b]thienyl, naphtho[2,3-b]thienyl, thianthrenyl, furyl, furanyl, pyranyl, isobenzofuranyl, benzoxazolyl, chromenyl, xanthenyl, phenoxathiinyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, 4aH-carbazolyl, carbazolyl, carbolinyl, phenanthridinyl, acridinyl, perimidinyl, phenanthrolinyl, phenazinyl, isothiazolyl, phenothiazinyl, isoxazolyl, furazanyl and phenoxazinyl groups).

Heteroaryl can be substituted with one, two or three substituents independently and individually selected from the group comprising halo, nitro, ((C₁-C₆)alkyl)carbonyl, cyano, nitrile, hydroxyl, trifluoromethyl, ((C₁-C₆)alkyl)sulfonyl, (C₁-C₆)alkyl, (C₁-C₆)alkenyl, (C₁-C₆)alkynyl, (C₁-C₆)alkoxy and (C₁-C₆)sulfanyl. As outlined above such "heteroaryl" may additionally be substituted by one or several substituents.

As used hereinafter in the description of the invention and in the claims, the term "alkyl", by itself or as part of another group, refers to a straight chain or branched chain alkyl group with 1 to 10 carbon atoms such as, for example methyl, ethyl, propyl, isopropyl, butyl, isobutyl, tert-butyl, pentyl, isopentyl, neopentyl, heptyl, hexyl, decyl. Alkyl groups can also be substituted, such as by halogen atoms, hydroxyl groups, C₁-C₄ alkoxy groups or C₆-C₁₂ aryl groups (which, in turn, can also be substituted, such as by 1 to 3 halogen atoms). More preferably alkyl is C₁-C₁₀ alkyl, C₁-C₆ alkyl or C₁-C₄ alkyl.

As used hereinafter in the description of the invention and in the claims, the term "alkenyl" and "alkynyl" is similarly defined as for alkyl, but contain at least one carbon-carbon double or triple bond, respectively.

As used hereinafter in the description of the invention and in the claims, the term "alkoxy (or alkyloxy)" refer to alkyl groups respectively linked by an oxygen atom, with the alkyl portion being as defined above.

As used herein in the description of the invention and in the claims, the substituent G³ as defined above and being part of the substituents "alkyl", "alkenyl", "alkynyl" and "alkoxy" can be attached at any carbon of the corresponding substituent "alkyl", "alkenyl", "alkynyl" and "alkoxy". Thus, e.g. the term "(G³-(C₁-C₈)alkoxy)aryl" does include different possibilities regarding positional isomerism, e.g. (G³-CH₂-CH₂-CH₂-CH₂-CH₂-CH₂-CH₂-CH₂-O-)aryl, (CH₃-CH₂-CH₂-CH(G³)-CH₂-CH₂-CH₂-CH₂-O-)aryl and (CH(-CH₂-CH₂-G³)(-CH₂-CH₃)-CH2-CH₂-CH₂-O-)aryl, ect..

Whenever the term "substituted" is used, it is meant to indicate that one or more hydrogens on the atom indicated in the expression using "substituted" is replaced with a selection from the indicated group, provided that the indicated atom's normal valency is not exceeded, and that the substitution results in a chemically stable compound, i. e. a compound that is sufficiently robust to survive isolation to a useful degree of purity from a reaction mixture, and formulation into a pharmaceutical composition. The substituent groups may be selected from halogen atoms (fluoro, chloro, bromo, iodo), hydroxyl groups, - SO₃H, nitro, ((C₁-C₆)alkyl)carbonyl, cyano, nitrile, trifluoromethyl, ((C₁-C₆)alkyl)sulfonyl, (C₁-C₆)alkyl, (C₂-C₆)alkenyl, (C₁-C₆)alkynyl, (C₁-C₆)alkoxy and (C₁-C₆)sulfanyl.

*In a **second aspect*** of the invention the ¹⁸F-labelled compounds of formula I, and the ¹⁹F standard reference compounds of formula I are provided as a medicament or pharmaceutical.

The invention relates also to the use of the ¹⁸F-labelled compounds of formula I, and of the ¹⁹F standard reference compounds of formula I for the manufacture of a medicament or a pharmaceutical for treatment.

In a more preferred embodiment the use concerns the treatment of a CNS disease. CNS diseases include but are not limited to: inflammatory and autoimmune, allergic, infectious and toxin-triggered and ischemia-triggered diseases, pharmacologically triggered inflammation with pathophysiological relevance, neuroinflammatory, and neurodegenerative diseases.

More preferably, the CNS disease is selected from multiple sclerosis, Alzheimer's disease, frontotemporal dementia, dementia with Levy bodies, leukoencephalopathy, epilepsy, neuropathic pain, amyotrophic lateral sclerosis, Parkinson's Disease, encephalopathies, brain tumors, depression, drug abuse, chronic inflammatory intestinal diseases, atheroma, atherosclerosis, arthritis, rheumatoid arthritis, pharmacologically triggered inflammation, systemic inflammation of unclear origin.

In one embodiment the disease is rheumatoid arthritis.

The present invention is also directed to a method of treatment of a disease of the central nervous system, as defined above, comprising the step of introducing into a patient a suitable quantity of a compound of formula I, preferably an ¹⁸F-labelled compound of formula I, or of a ¹⁹F standard reference compound of formula I.

*In a **third aspect*** of the invention, ¹⁸F-labelled compounds of formula I are provided as diagnostic imaging agent or imaging agent, preferably as imaging agent for PET applications. It is obvious to persons skilled in the art that compounds of formula I and related derivatives, e.g. compound of formula I wherein L = iodo (e.g. I-123) are suited as imaging agents for SPECT applications.

The invention relates also to the use of ¹⁸F-labelled compounds of formula I for the manufacture of an imaging agent.

In a more preferred embodiment the use concerns the imaging of CNS diseases. CNS diseases include but are not limited to inflammatory and autoimmune, allergic, infectious and toxin-triggered and ischemia-triggered diseases, pharmacologically triggered inflammation with pathophysiological relevance, neuroinflammatory and neurodegenerative diseases.

More preferably, the CNS disease is selected from multiple sclerosis, Alzheimer's disease, frontotemporal dementia, dementia with Levy bodies, leukoencephalopathy, epilepsy, neuropathic pain, amyotrophic lateral sclerosis, Parkinson's Disease, encephalopathies, brain tumors, depression, drug abuse, chronic inflammatory intestinal diseases, atheroma, atherosclerosis, arthritis, rheumatoid arthritis, pharmacologically triggered inflammation, systemic inflammation of unclear origin. The present invention is also directed to a method of imaging comprising the step of introducing into a patient a detectable quantity of an ¹⁸F-labelled compound of formula I and imaging said patient.

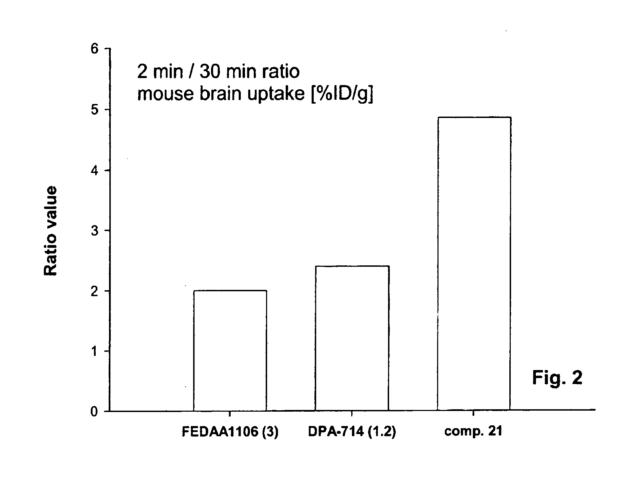

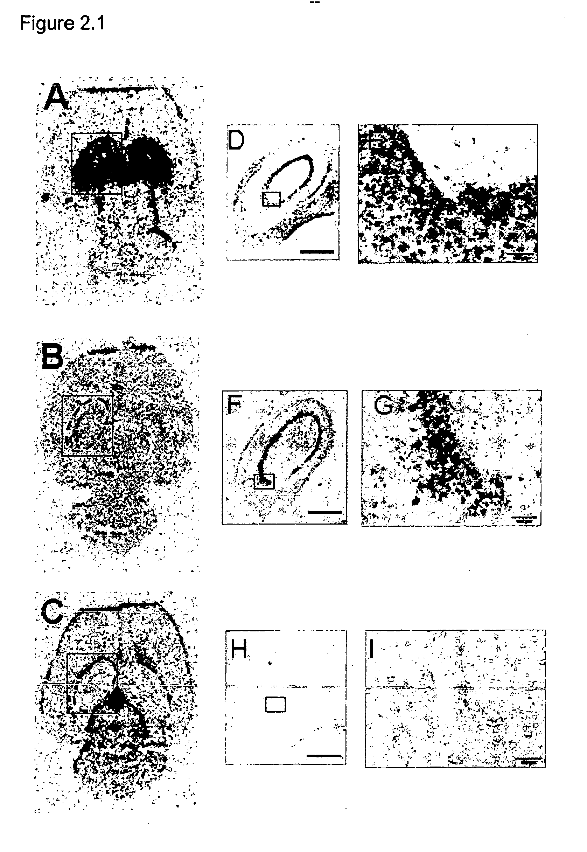

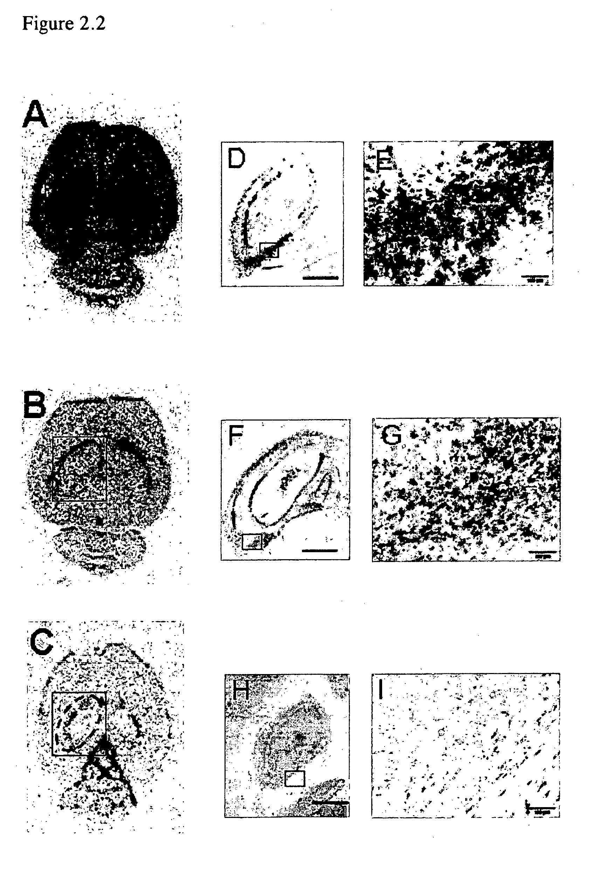

It has been found out that compounds of formula I show a good initial brain uptake and a good elimination at later timepoints. This fact is expressed by the ratio of brain uptake in mice at 2 min to 30 min (uptake percentage of injected dose per one gram tissue (%ID/g)). The higher the ratio value the better the signal to background ratio. Thus, e.g. compound **21** has a superior ratio of 4.85 compared to e.g. FEDAA **(3)** which shows a ratio of 2.00 and DPA-714 **(1.2)** showing a ratio of 2.43 (compare fig. 2).

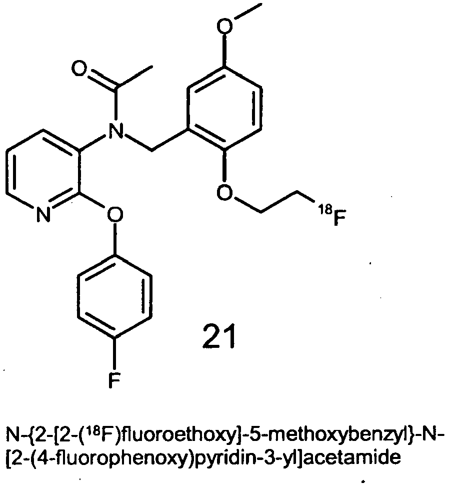

*In a **fourth aspect*** of the invention, pharmaceutical compositions are provided comprising a compound according to formula I, preferably ¹⁸F-labelled compounds of formula I, or ¹⁹F standard reference compounds of formula I or a pharmaceutically acceptable salt of an inorganic or organic acid thereof, a hydrate, a complex, an ester, an amide, a solvate or a prodrug thereof. Preferably the pharmaceutical composition comprises a physiologically acceptable carrier, diluent, adjuvant or excipient.

In a preferred embodiment, pharmaceutical compositions according to the present invention comprise a compound of formula I that is a pharmaceutical acceptable salt, hydrate, complex, ester, amide, solvate or a prodrug thereof.

As used hereinafter in the description of the invention and in the claims, the terms "inorganic acid" and "organic acid", refer to mineral acids, including, but not being limited to: acids such as carbonic, nitric, hydro chloric, hydro bromic, hydro iodic, phosphoric acid, perchloric, perchloric or sulphuric acid or the acidic salts thereof such as potassium hydrogen sulphate, or to appropriate organic acids which include, but are not limited to: acids such as aliphatic, cycloaliphatic, aromatic, araliphatic, heterocyclic, carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic, trifluormethansulfonic, 1,1,2,2,3,3,4,4,4-nonafluorobutane-1-sulfonic acid and sulfanilic acid, respectively.

*In a **fifth aspect*** of the invention, a radiopharmaceutical composition is provided comprising an ¹⁸F-labelled compound of formula I or a pharmaceutically acceptable salt of an inorganic or organic acid thereof, a hydrate, a complex, an ester, an amide, a solvate or a prodrug thereof.

Preferably the pharmaceutical composition comprises a physiologically acceptable carrier, diluent, adjuvant or excipient.

The compounds according to the present invention, preferably the radioactively labeled compounds according to Formula I provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are physiological saline solution and plasma.

Suitable pharmaceutical acceptable carriers are known to someone skilled in the art. In this regard reference can be made to e.g. Remington's Practice of Pharmacy, 13th ed. and in J. of. Pharmaceutical Science & Technology, Vol. 52, No. 5, Sept-Oct., p. 238-311, included herein by reference.

The concentration of the compounds of formula I, preferably of the ¹⁸F-labelled compound according to the present invention and the pharmaceutically acceptable carrier, for example, in an aqueous medium, varies with the particular field of use. A sufficient amount is present in the pharmaceutically acceptable carrier when satisfactory visualization of the imaging target (e.g. PBR (translocator, or an inflammed region or a tumor) is achievable.

The compounds according to the present invention, in particular the ¹⁸F-radioactively labeled compounds according to the present invention, i.e. the ¹⁸F-labelled compounds of formula I, provided by the invention may be administered intravenously in any pharmaceutically acceptable carrier, e.g., conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g., Remington's Practice of Pharmacy, 11th ed. and in J. of. Pharmaceutical Science & Technology, Vol. 52, No. 5, Sept-Oct., p. 238-311.x

In accordance with the invention, the radiolabeled compounds having general chemical Formula I either as a neutral composition or as a salt with a pharmaceutically acceptable counter-ion are administered in a single unit injectable dose. Any of the common carriers known to those with skill in the art, such as sterile saline solution or plasma, can be utilized after radiolabelling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or disease in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

It is obvious for somebody skilled in the art that the radiofluorination reaction of compounds of formula I wherein L is R³, can cause side products and compounds which are not represented by formula I. These products are charaterized by the circumstance that e.g. L is hydroxyl or -N(Me)₂, (goes optionally along with nucleophilc aromatic substation reactions) or that e.g. L is hydroxyl, that e.g the precursor compound dimerizes as ether or that the leaving group is eliminated resulting in a corresponding alkene (goes optionally along with aliphatic nucleophilic substitution reactions). Such side products and similar derivatives are typically separated from the reaction mixture but can be still part in certain amounts in the radiopharmaceutical composition administered to a patient or mamal.

In a ***sixth*** aspect the present invention is directed to compounds of Formula I, wherein L is [¹⁹F]fluoro,
preferred compounds of formula I, with L being [¹⁹F]fluoro are:

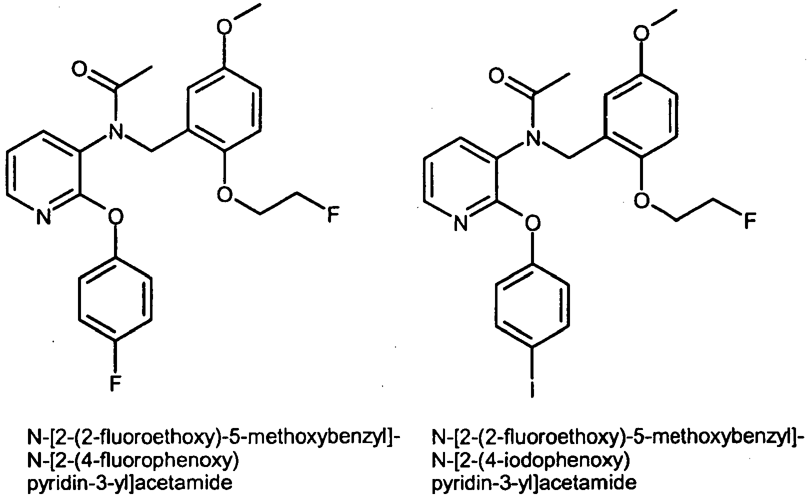

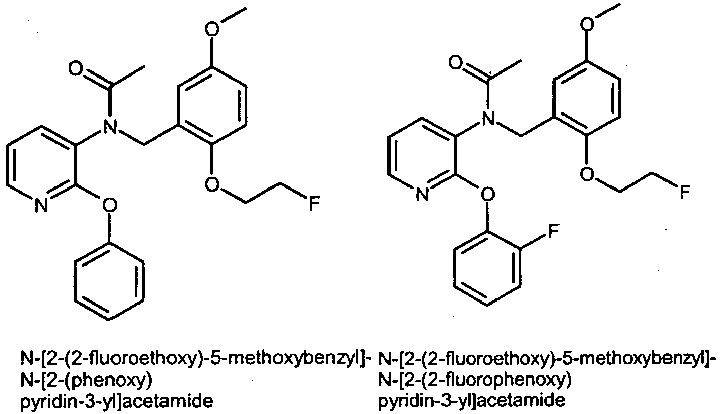

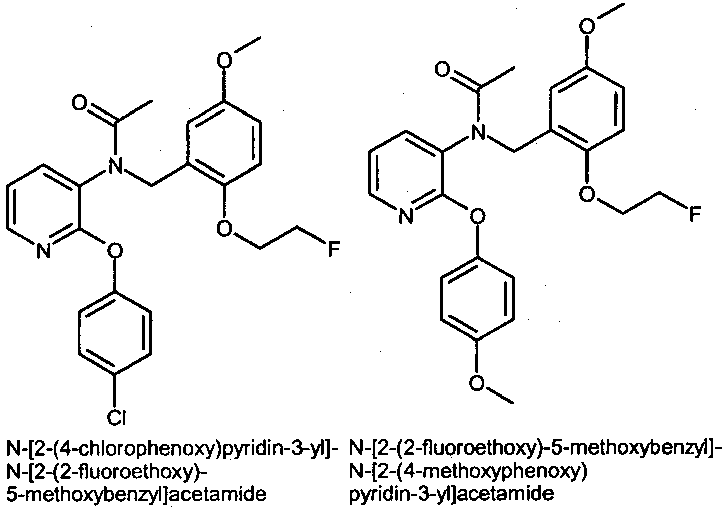

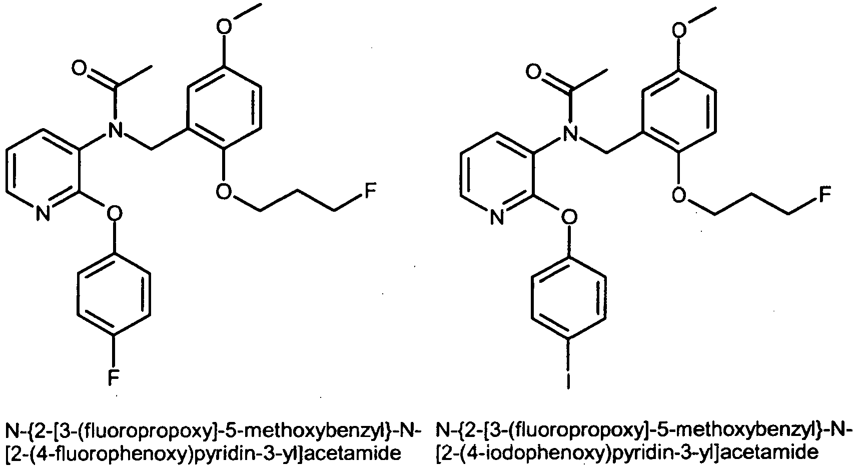

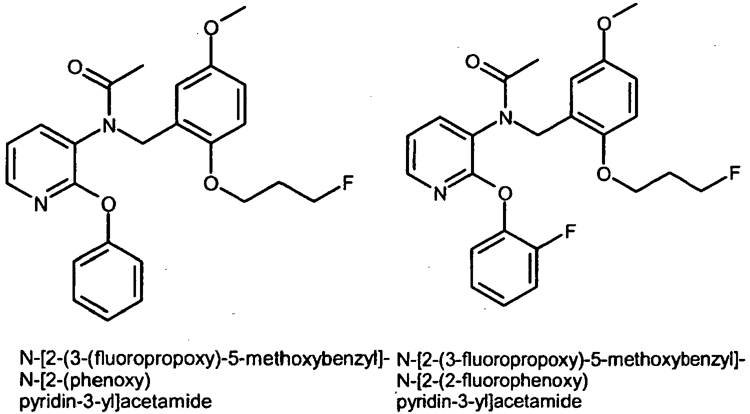

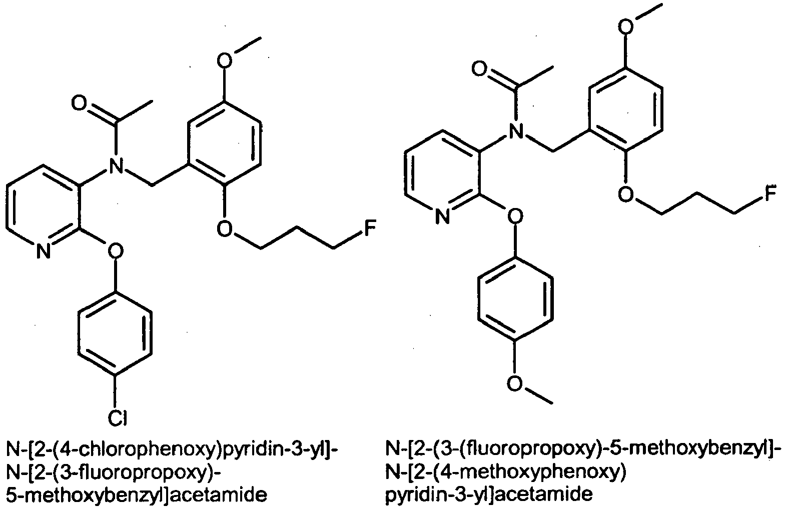

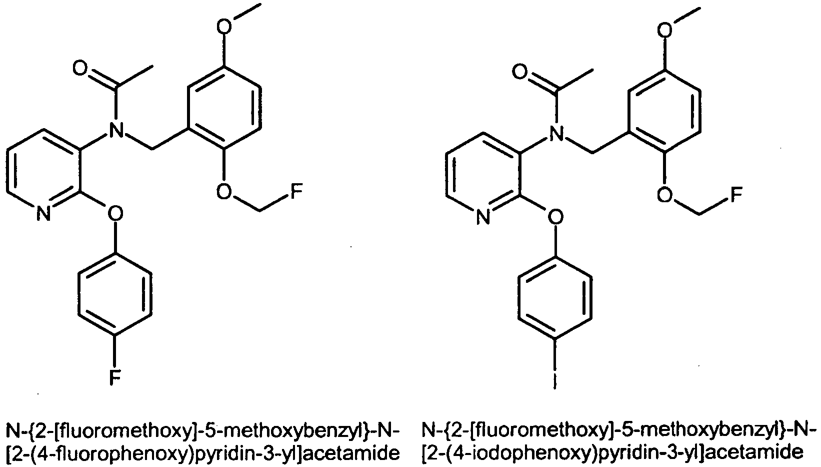

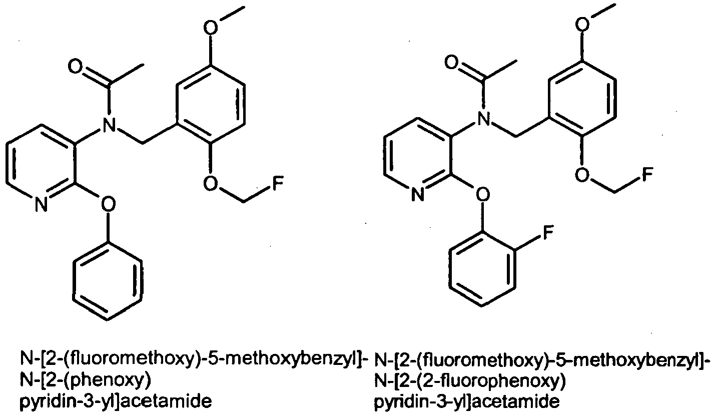

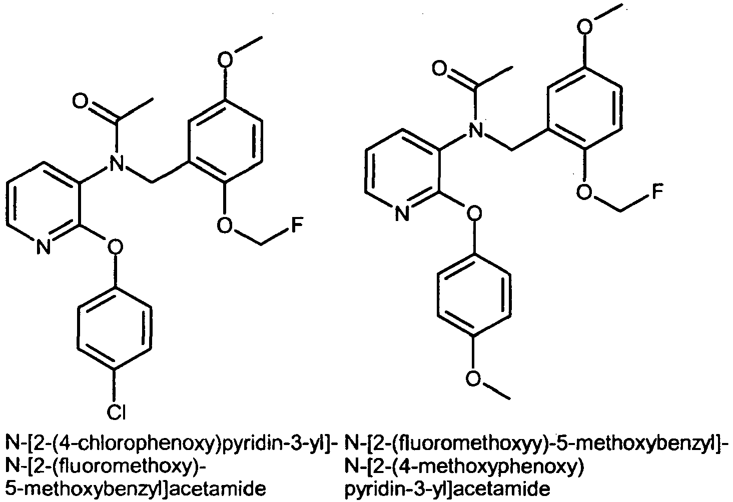

If a chiral center or another form of an isomeric center is present in a compound according to the present invention, all forms of such stereoisomer, including enantiomers and diastereoisomers, are intended to be covered herein. Compounds containing a chiral center may be used as racemic mixture or as an enantiomerically enriched mixture or the racemic mixture may be separated using well-known techniques and an individual enantiomer maybe used alone. In cases in which compounds have unsaturated carbon-carbon bonds double bonds, both the (Z)-isomer and (E)-isomers are within the scope of this invention. In cases wherein compounds may exist in tautomeric forms, such as keto-enol tautomers, each tautomeric form is contemplated as being included within this invention whether existing in equilibrium or predominantly in one form.

Unless otherwise specified, when referring to the compounds of formula the present invention per se as well as to any pharmaceutical composition thereof the present invention includes all of the hydrates, salts, solvates, complexes, and prodrugs of the compounds of the invention. Prodrugs are any covalently bonded compounds, which releases the active parent pharmaceutical according to formula I.

The term "halo" refers to fluorine (F), chlorine (CI), bromine (Br), and iodine (I).

In a ***seventh aspect*** the present invention is directed to compounds of formula IV 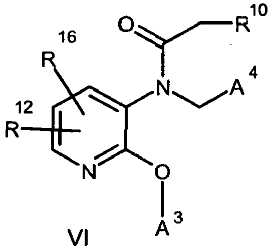 wherein
**R¹⁰** is selected from the group comprising (C₁-C₆)alkyl and hydrogen;
**R¹⁶** is selected from the group comprising hydrogen, halo, trifluoromethyl, (C₁-C₅)alkyl, (C₂-C₅)alkynyl), (C₂-C₅)alkenyl and (C₁-C₅)alkoxy;
**A³** and **A⁴** are the same or different and of the structure (R¹²)(R⁴)(R⁵)phenyl;
**R¹²** is selected from the group comprising **R¹³** and hydrogen;
**R¹³** is hydroxy,
   with the proviso that compounds of formula VI contain exactly one **R¹³;**
**R⁴** and **R⁵** are independently and individually, at each occurrence, selected from the group comprising hydrogen, halo, trifluoromethyl, (C₁-C₅)alkyl, (C₂-C₅)alkynyl), (C₂-C₅)alkenyl and (C₁-C₅)alkoxy;
including all isomeric forms of said compound, including but not limited to enantiomers and diastereoisomers as well as racemic mixtures,
and any pharmaceutically acceptable salt, ester, amide or complex thereof.

In a preferred embodiment **R¹⁶** is selected from the group comprising hydrogen, fluoro, chloro, iodo, methyl, methoxy and trifluoromethyl;

in a more preferred embodiment **R¹⁶** is selected from the group comprising hydrogen, fluoro, chloro, iodo and methyl;

in an even more preferred embodiment **R¹⁶** is selected from the group comprising hydrogen and chloro;

in the most preferred embodiment **R¹⁶** is hydrogen;
in a preferred embodiment **R⁴** and **R⁵** are independently and individually, at each occurrence, selected from the group comprising hydrogen, fluoro, chloro, methyl, methoxy and trifluoromethyl;
in a more preferred embodiment **R⁴** and **R⁵** are independently and individually, at each occurrence, selected from the group comprising hydrogen, fluoro, methyl, and methoxy;
in a preferred embodiment **R¹⁰** is selected from the group comprising methyl and hydrogen;
with the proviso that compounds of formula VI contain exactly one R¹².

In a ***eighth aspect*** of the present invention is directed to a method for obtaining compounds of Formula I, wherein L is [¹⁸F]fluoro or [¹⁹F]fluoro.

Surprisingly two methods have been identified for obtaining such compounds.

In a first embodiment, a precursor compound according to formula I, wherein L is **R³ as** defined above, is reacted with an F-fluorinating agent.

Preferably, said F-fluorinating agent is a compound comprising F-anions, preferably a compound selected from the group comprising 4, 7, 13, 16, 21, 24-hexaoxa-1,10-diazabicyclo[8.8.8]-hexacosane KF, i.e. crownether salt Kryptofix KF, KF, HF, KHF₂, CsF, NaF and tetraalkylammonium salts of F, such as N(butyl)₄F (tetrabutylammonium fluoride), and wherein F = ¹⁸F or ¹⁹F.

More specifically, with respect to ¹⁸F-labelled compounds of formula I, the first embodiment of a radiolabeling method for obtaining an ¹⁸F-labelled compound of formula I comprises the step of
- ¹⁸F-Radiolabelling a compound of formula I having an appropriate leaving group with a fluorination agent for obtaining an ¹⁸F-labelled compound of formula I,

The term "radiolabelling" a molecule, as used herein, usually refers to the introduction of an ¹⁸F-atom into the molecule.

The fluorination agent is defined as above, wherein F = ¹⁸F.

In a second embodiment, a method of synthesis of compounds of Formula I, wherein L is [¹⁸F]fluoro or [¹⁹F]fluoro, comprises the steps:
- F-fluorinating a compound of formula V 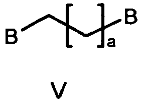
formula V
with an F-fluorinating agent to yield a compound of formula IV, 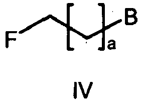 formula IV
- substituting said compound of formula IV with a compound of formula VI 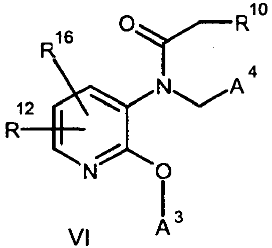
formula VI
wherein **F** in Formula IV is [¹⁸F]fluoro or [¹⁹F]fluoro,
a is an integer from 0 to 5, preferably from 0 to 2, more preferably from 0 to 1, **B** is a leaving group, preferably halo, in particular chloro, bromo, iodo, mesyloxy, tosyloxy, trifluormethylsulfonyloxy, nona-fluorobutylsulfonyloxy, (4-bromo-phenyl)sulfonyloxy, (4-nitro-phenyl)sulfonyloxy, (2-nitro-phenyl)sulfonyloxy, (4-isopropyl-phenyl)sulfonyloxy, (2,4,6-tri-isopropyl-phenyl)sulfonyloxy, (2,4,6-trimethyl-phenyl)sulfonyloxy, (4-*tert*butyl-phenyl)sulfonyloxy, and (4-methoxy-phenyl)sulfonyloxy;
**R¹⁰** is selected from the group comprising (C₁-C₆)alkyl and hydrogen;
**R¹⁶** is selected from the group comprising hydrogen, halo, trifluoromethyl, (C₁-C₅)alkyl, (C₂-C₅)alkynyl), (C₂-C₅)alkenyl and (C₁-C₅)alkoxy;
**A³** and **A⁴** are the same or different and of the structure (R¹²)(R⁴)(R⁵)phenyl;
**R¹²** is selected from the group comprising **R¹³** and hydrogen;
**R¹³** is hydroxy,
   with the proviso that compounds of formula VI contain exactly one **R¹³;**
**R⁴** and **R⁵** are independently and individually, at each occurrence, selected from the group comprising hydrogen, halo, trifluoromethyl, (C₁-C₅)alkyl, (C₂-C₅)alkynyl), (C₂-C₅)alkenyl and (C₁-C₅)alkoxy;
in a preferred embodiment **R⁴** and **R⁵** are independently and individually, at each occurrence, selected from the group comprising hydrogen, fluoro, chloro, methyl, methoxy and trifluoromethyl;
in a more preferred embodiment **R⁴** and **R⁵** are independently and individually, at each occurrence, selected from the group comprising hydrogen, fluoro, methyl, and methoxy;
in a preferred embodiment **R¹⁰** is selected from the group comprising hydrogen and methyl;
in a preferred embodiment **R¹⁶** is selected from the group comprising hydrogen, fluoro, chloro, iodo methyl, methoxy and trifluoromethyl;
in a more preferred embodiment **R¹⁶** is selected from the group comprising hydrogen, fluoro, chloro, iodo and methyl;
in an even more preferred embodiment **R¹⁶** is selected from the group comprising hydrogen and chloro;
in the most preferred embodiment **R¹⁶** is hydrogen;
wherein said F-fluorinating agent is as defined above,
and wherein F = ¹⁸F or ¹⁹F ,
with the proviso that compounds of formula VI contain exactly one R¹².

Preferably, **B** is selected from the group comprising iodo, bromo, chloro, mesyloxy, tosyloxy, trifluormethylsulfonyloxy, and nona-fluorobutylsulfonyloxy.

More specifically the second embodiment of a radiolabeling method for obtaining an ¹⁸F-labelled compound of formula I comprises the steps of
- ¹⁸F radiolabeling a compound of formula V with a fluorination agent to yield a compound of formula IV, and
- substituing a compound of formula IV with a compound of Formula VI.

The ¹⁸F-labelled compound of Formula IV is 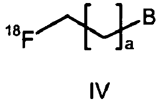 or pharmaceutically acceptable salts of an inorganic or organic acid thereof, hydrates, complexes, esters, amides or solvates thereof,
wherein
**B** is a leaving group;
the leaving group **B** is known or obvious to someone skilled in the art and which is taken from but not limited to those described or named in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O-nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15;
in a more preferred embodiment **B** is selected from the group comprising:
a) iodo,
b) bromo,
c) chloro,
d) mesyloxy,
e) tosyloxy,
f) trifluormethylsulfonyloxy and
g) nonafluorobutylsulfonyloxy;
a is an integer from 0 to 4, preferably a is an integer of from 0 to 2 and more preferably a is an integer of from 0 to 1;

The compound of Formula V is 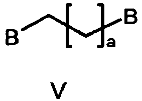 or pharmaceutically acceptable salts of an inorganic or organic acid thereof, hydrates, complexes, esters, amides or solvates,
wherein
**B** is defined as above for compounds of Formula IV, and
**a** is defined as above for compounds of Formula IV,

The fluorination agent is defined as above.

In a preferred embodiment, the fluorination agent is a fluorine radioactive isotope derivative.

More preferably the fluorine radioactive isotope derivative is a ¹⁸F derivative. More preferably, the ¹⁸F derivative is 4,7,13,16,21,24-Hexaoxa-1,10-diazabicyclo[8.8.8]-hexacosane K¹⁸F (crownether salt Kryptofix K¹⁸F), K¹⁸F, H¹⁸F, KH¹⁸F₂, Cs¹⁸F, Na¹⁸F or tetraalkylammonium salt of ¹⁸F (e.g.[F-18] tetrabutylammonium fluoride). More preferably, the fluorination agent is K¹⁸F, H¹⁸F, or KH¹⁸F₂, most preferably K¹⁸F (¹⁸F fluoride anion).

The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxide and acetonitrile as solvent and tetraalkyl ammonium and tertraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. These and other conditions for such radiofluorinations are known to persons skilled in the art (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (review: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

Furthermore, the invention provides for a composition comprising a compound according to the present invention and a pharmaceutically acceptable carrier or diluent.

In one embodiment said compound is an ¹⁸F-labelled compound.

In another embodiment said compound is a ¹⁹F-labelled compound.

In yet another embodiment said compound is a precursor compound.

The invention also provides for a compound according to the present invention, preferably an ¹⁸F- or ¹⁹F-labelled compound according the present invention, or a composition according to the present invention for use as a pharmaceutical or diagnostic agent or imaging agent.

The invention also provides for the use of a compound according to the present invention, preferably an ¹⁸F- or ¹⁹F-labelled compound according to the present invention, or a composition according to the present invention for the manufacture of a medicament for the treatment and/or diagnosis and/or imaging of diseases of the central nervous system (CNS).

The invention also provides for an ¹⁸F-labelled compound of formula I or a composition containing such compound for use as a diagnostic agent or imaging agent, in particular for diseases of the central nervous system.

The invention also provides for a method for detecting the presence of PBR receptor (translocator protein) in a patient's body, preferably for imaging a disease of the central nervous system in a patient, comprising:
introducing into a patient's body a detectable amount of an ¹⁸F-labelled compound according to the present invention or a composition comprising such compound,
and detecting said compound or said composition by positron emission tomography (PET).

The invention also provides for compounds for use in a method of treatment of a disease of the central nervous system comprising the step of introducing into a patient a suitable quantity of a compound according to the present invention, preferably of an ¹⁸F-or ¹⁹F-labelled compound according to the present invention.

The general synthesis scheme of the tricyclic scaffold comprising aromatic ring systems A, B and C is shown in scheme 1: Thus, compounds of type E1 are alkylated with phenol anions towards compounds of type E2 whereas "(N)" represents a nitrogen containing substituent, preferably nitro, and "(X)" represents a leaving group, e.g. halo. The substituent "(N)" is converted to an aniline derivative E3 by methods which are known to persons skilled in the art (e.g. if "(N)" is nitro then a hydrogenation reaction leads to the desired compound E3). The reductive amination reaction with aldehydes of type E8 leads anilines of type E4.

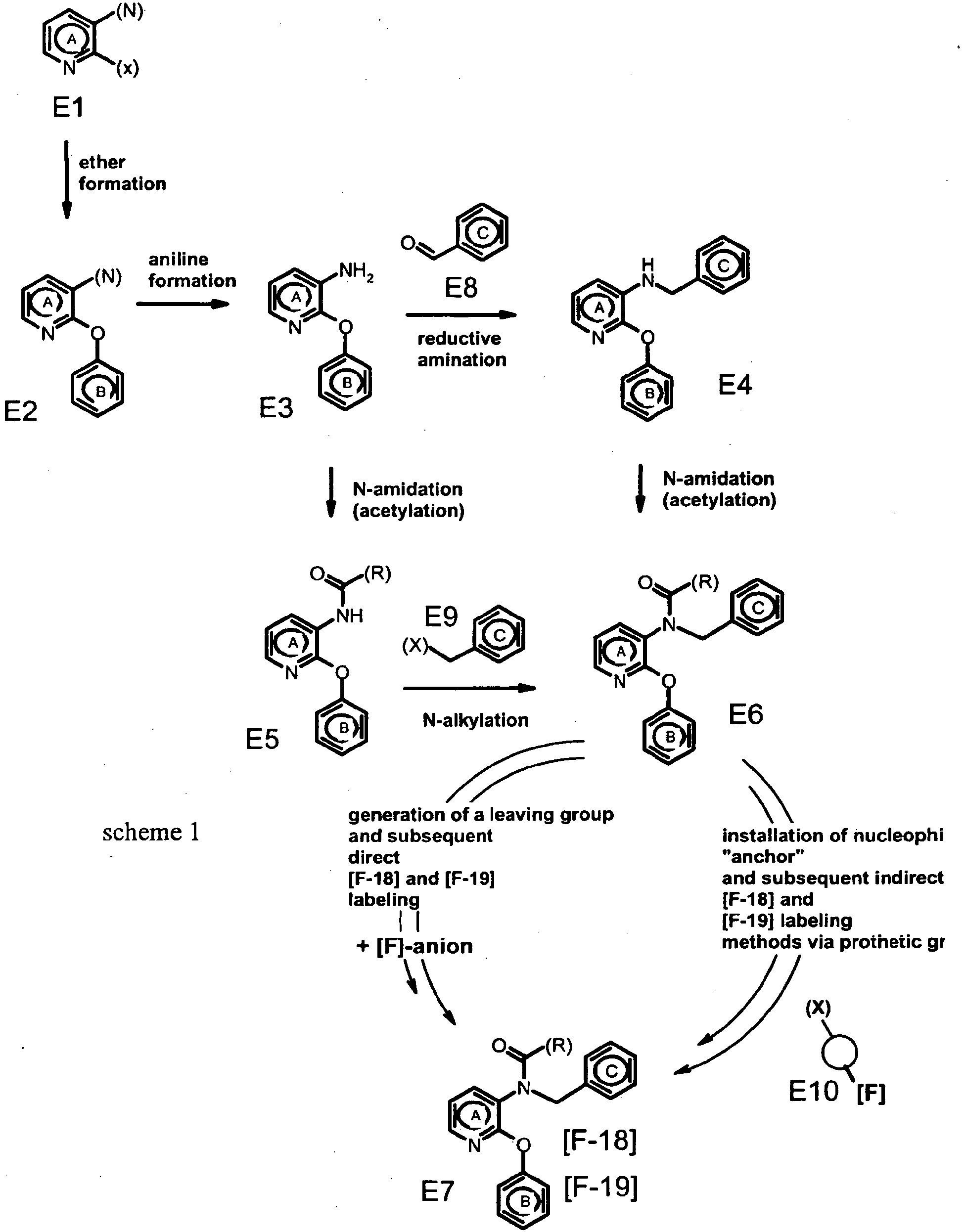

Compounds of type E3 can also be converted to compounds of E5 by amidation or N-acetylation reaction which are known to people skilled in the art. Compounds of type E4 can be converted to amides of type 6. But also alkylation reactions of compounds of type E5 with alkylating agents of type E9 representing the "C-ring" lead to compounds of type E6. There are two approaches to introduce the F-18 label (and optionally also the corresponding F-19 label). For example the installation of a suited leaving group can be achieved by mesylation of the corresponding alcohol (compare scheme 2: (9) → (10)). But also the alkylation reaction of small F-18 labelled building blocks (prosthetic groups, E10) can be used to link them to a nucleophilc functional group being introduced to compounds of type E6 (compare scheme 3, (15) → (19)).

Scheme 2 describes a particular example of methods to synthesize compounds of formula I:

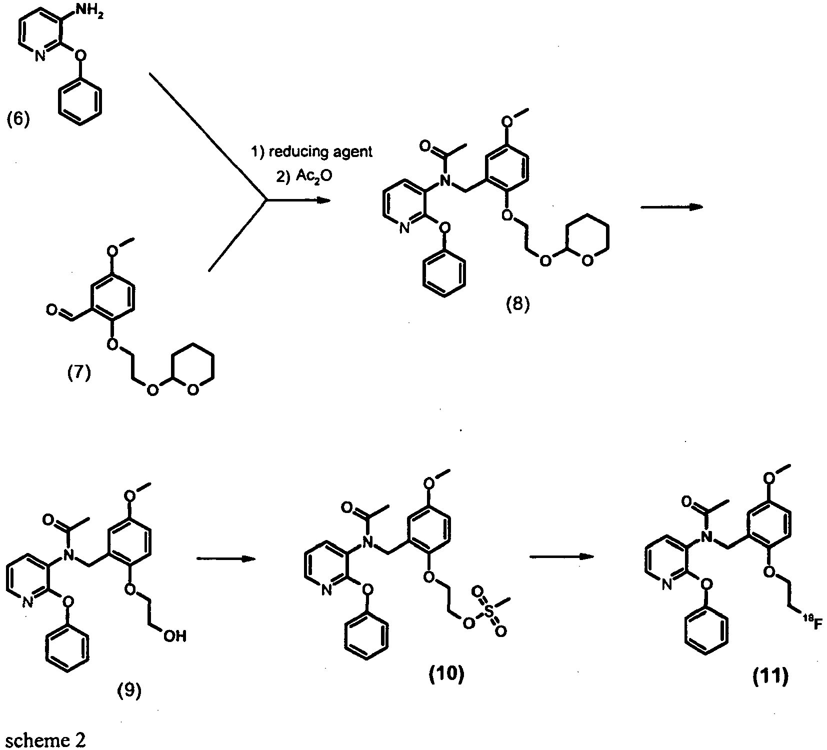

Aniline **6** (J. Med. Chem. (2002), 45, 23, 5182-5185) and aldehyde **7** (EP1894915A1) are converted in a reductive amination reaction using sodium tris(acetoxy)borohydride. The subsequent acetylation reaction of the crude secondary aniline leads to the desired product **8.** Tetrahydropyranyl ether **8** is cleaved under acidic conditions using PPTS in methanol. The desired alcohol **9** is converted to the corresponding mesylate **10** using mesyl chloride and triethyl and hünig's base in dichloromethane. The subsequent fluorination with KF and kryptofix leads to the desired [F-18] labelled compound **11.** The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxid and acetonitril as solvent and tetraalkyl ammonium and tertraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (review: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

Another approach is shown as example in scheme 3: Aniline **12** (ABCR) and aldehyde **13** (Bioorg. Med. Chem. Lett. (2007), 2614-2617) are reacted with eachother by reductive amination reaction using sodium tris(acetoxy) borohydride. Subsequent acetylation of the crude product leads to the desired product **14.** Benzyl ether **14** is cleaved by methods which are known to persons skilled in the art. Typically heterogeneous catalytic hydrogenation with hydrogen and palladium on charcoal is used to obtain phenol **15.** Phenol **15** can be either alkylated with [¹⁸F]-fluoro ethyl bromide (which is generated from 2-bromo ethyl triflate (Bioorg. Med. Chem.; 11; 12; 2003; 2519 - 2528)) to obtain compound **19.** The alkylation of compound **15** with 2-benzyloxy-ethyl bromide using sodium carbonate as base in acetonitril leads to compound **16.** Compound **15** can also be alkylated with 1-fluoro-2-iodoethane or with 1-bromo-2-fluoroethane. The product of this conversion is the corresponding F-19 reference standard **20** for radiofluorination experiments using mesylate **18.** Mesylate **18** can be prepared from alcohol **17** by use of mesylchloride and triethylamine in dichloromethane. The hydrogenation of the benzyl ether **16** with hydrogen on palladium/charcoal in iso-propanol leads to the aforementioned alcohol **17.**

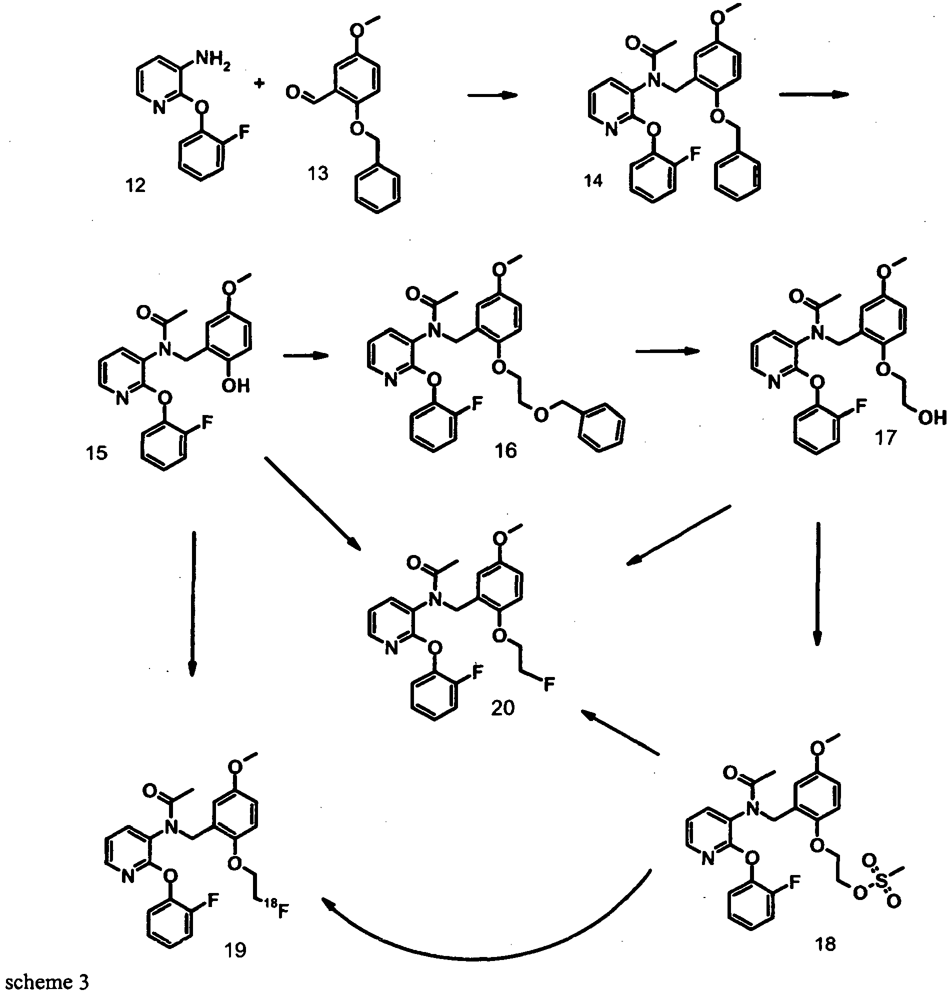

Similar compounds which can be generated by described methods are:

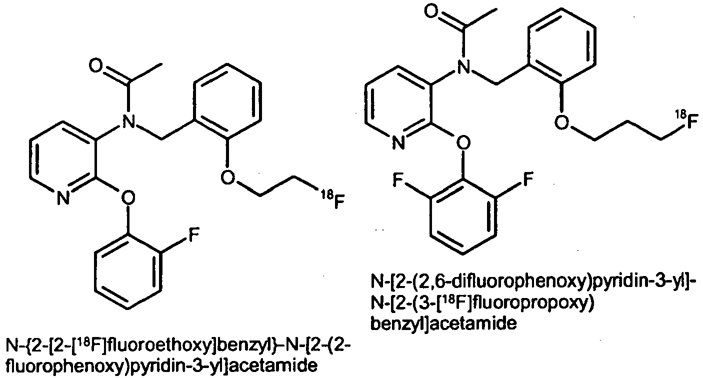

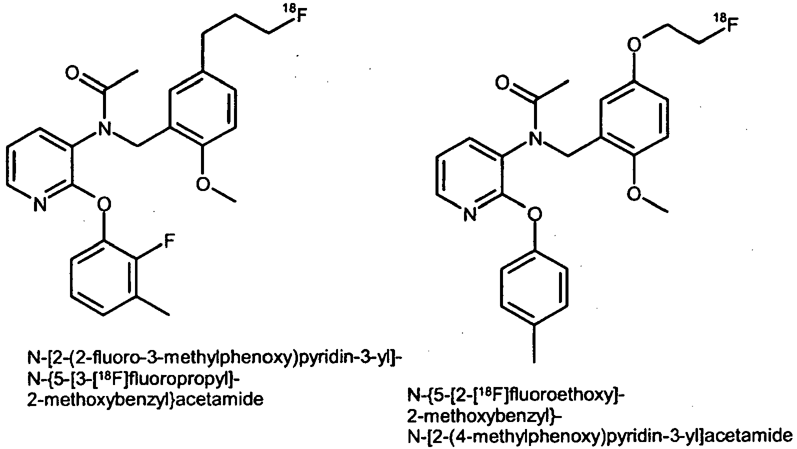

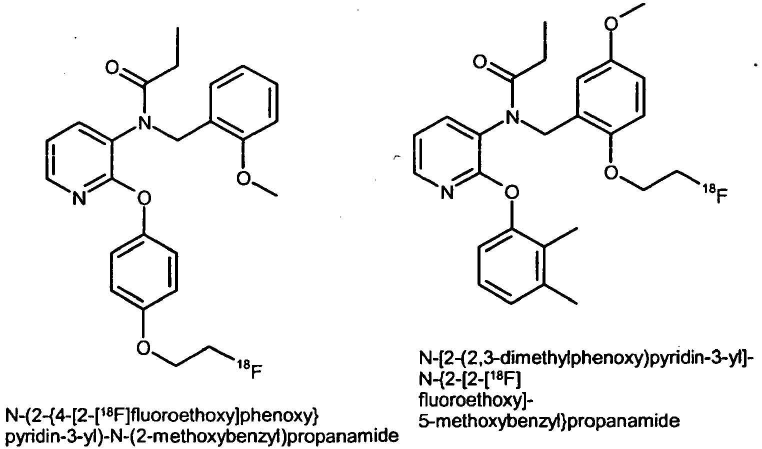

Scheme 4 shows another approach to synthesize compounds of formula I:

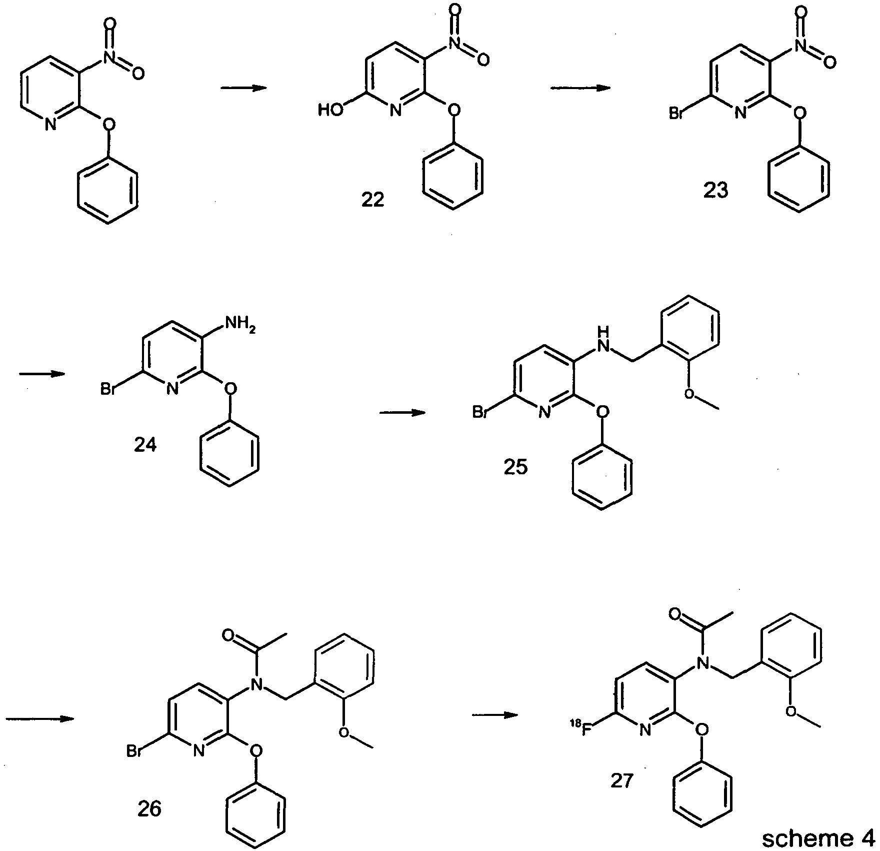

Compound **22** (scheme 4) is generated from 3-nitro-2-phenoxypyridine by oxidation with tert-butyl hydroperoxide (e.g. Journal of Medicinal Chemistry; English; 50; 1; 2007; 2 - 5). Bromination of alcohol **22** is carried out with phosphoric tribromide (e.g. Tetrahedron Letters; English; 32; 34; 1991; 4263 - 4266) towards compound **23.** Reduction of the nitro group of compound 23 is performed using iron powder in acid (e.g. Recueil des Travaux Chimiques des Pays-Bas; 64; 1945; 102,104)

Reductive amination (e.g. Journal of Organic Chemistry (2004), 69, 35) of 2-methoxy benzaldehyde with aniline **24** leads to amine **25** which can be acetylated towards amide **26.** Radiolabelling of bromo pyridine **26** with F-18 pottasium fluoride leads to the desired compound F-18 labelled **27.** Radiolabeling procedures of fluoro pyridines are well known to skilled persons in the field (Dolle et al., (2006), in: Schubiger P.A., Friebe M., Lehmann L., (eds), PET-Chemistry - The Driving Force in Molecular Imaging. Springer, Berlin Heidelberg).

Non-radioactive Fluorination of compound 26 with potassium fluoride or tetrabutylammonium reagent leads to corresponding F-19 reference compound. The compounds of the invention can be used in methods for imaging, diagnosing and treating central nervous system disorders and neurodegenerative disorders. A preferred method of imaging is PET. Central nervous system or neurodegenerative disorders can be but are not limited to Alzheimer's disease, dementia, multiple sclerosis, or amyotrophic lateral sclerosis.

### Brief description of the figures

**Figure 1****:** Scheme showing ways to syntheze compounds of the invention.
**Figure 1.1****:** Uptake and elimination of [¹⁸F]-radioactivity in blood and brain over time after i.v. injection of [¹⁸F]-**2d** into normal mice (n=3 per time point).
**Figure 1****.****2****:** Uptake an elimination of [¹⁸F]-radioactivity in blood and brain over time after i.v. injection of [¹⁸F]-**5d** into normal mice (n=3 per time point).
**Figure 2****:** Ratio of brain uptake in mice at 2 min to 30 min (uptake percentage of injected dose per one gram tissue (%ID/g)) of compound **21** compared to FEDAA **(3) and** DPA-714. The higher the ratio value the better the signal to background ratio.
**Figure 2****.****1****:** *Ex vivo* autoradiography of transversal rat brain sections of kainic acid treated rats model and respective sham controls 30 min after injection of [¹⁸F]-**2d** (A-C). Activated microglia was visualized on the same sections by subsequent immunohistochemistry using the Ox-42 antibody (D-I). Kainic acid treated rats (A) were compared to sham treated rats (C). Note the intense signal in the hippocampal region, known to be affected in this model. In kainic acid treated rats co-injection of [¹⁹F]-**2e** blocked these signals (B).
   Bars represent 1000 µm (D, F, H) and 100 µm (E,G,I). Slices are located at -3.1 mm bregma. Regions of interest for quantification and calculation of the respective hippocampus / cerebellum ratio are marked by dotted circles.
**Figure 2.2****:** *Ex vivo* autoradiography of transversal rat brain sections of the kainic acid treated rats and respective sham controls 30 min after injection of [¹⁸F]-**5d** (A-C). Activated microglia was visualized on the same sections by subsequent immunohistochemistry using the Ox-42 antibody (D-I). Kainic acid treated rats (A) were compared to sham treated rats (C). Note the intense signal in the hippocampal region, known to be affected in this model. In kainic acid treated rats co-injection of [¹⁹F]-**5e** blocked these signals (B).
   Bars represent 1000 µm (D, F, H) and 100 µm (E,G,I). Slices are located at -3.1 mm bregma. Regions of interest for quantification and calculation of the respective hippocampus / cerebellum ratio are marked by dotted circles.
**Figure 3****:**
   Signal-to-background ratio expressed as hippocampus / cerebellum ratio calculated from quantified *ex vivo* autoradiography signals from brain slices of kainic acid treated rats .

### Experimental section

### General procedures:

### A: Fluorination with non-radioactive [F-19] fluoride

To a solution of 1 eq. starting material in acetonitrile (2ml/eq.) 1.1 eq. potassium fluoride and kryptofix (1.1 eq.) are added. The reaction mixture is heated by microwave (130° C, 15 min) and cooled to room temperature again. The reaction mixture is diluted with 10 ml diethyl ether and 10 ml water. The organic phase is separated. The aqueous phase is extracted three times with 10 ml diethyl ether. The combined organic phases are washed with brine and dried with magnesium sulfate. The solvent is evaporated and the residue is purified by column chromatography with ethyl acetate-hexane gradient.

### B: Fluorination with radioactive [F-18] fluoride

Aqueous [¹⁸F]Fluoride (0.1-5GBq) was trapped on a QMA cartridge and eluted with 5mg K2.2.2 in 0,95ml MeCN +1mg K2CO3 in 50µl water into a Wheaton vial (5ml). The solvent is removed by heating at 120°C for 10 mins under a stream of nitrogen. Anhydrous MeCN (1 ml) is added and evaporated as before. This step is repeated three times. A solution of starting material (1 mg) in 300 µl anhydrous DMF is added. After heating at 120°C for 10 min the crude reaction mixture is analyzed using analytical HPLC: ACE3-C18 50 mm x 4,6 mm; solvent gradient: start 5%acetonitril - 95%acetonitril in water in 7 min., flow: 2ml/min. The desired F-18 labeled product is confirmed by co-injection with the corresponding non-radioactive F-19 fluoro-standard on the analytical HPLC. The crude product is pre-purified via a C18 SPE cartridge and (50-2500 MBq) of that pre-purified product are purified by preparative HPLC: ACE 5-C18-HL 250mmx10mm; 62% isocratic acetonitril in water 25 min., flow: 3ml/min The desired product is obtained (30-2000 MBq) as reconfirmed by co-injection with the non-radioactive F-19 fluoro standard on the analytical HPLC. The sample was diluted with 60ml water and immobilized on a Chromafix C18 (S) cartridge, which was washed with 5ml water and eluted with 1ml ethanol to deliver 20-1800 MBq product in 1000µl EtOH.

### H: Alkylation of phenols

To a stirred solution of 1 eq. starting material (phenol derivative) and 1.5 eq. potassium carbonate in dimethyl formamide 3ml/1eq. is added 2.5 mmol alkylating agent. The reaction mixture is heated at 70°C for 6 hours or by microwave to 110°C for 15 min. The solvent of the reaction mixture is evaporated. Water and methyl tert-butyl ether are added. The organic phase is separated. The aqueous phase is extracted three times with methyl tert-butyl ether diethyl ether. The combined organic phases are washed with water, brine and dried with magnesium sulfate. The solvent is evaporated and the residue is purified by column chromatography with ethyl acetate-hexane gradient.

### I: Conversion of alcohol to corresponding O- sulfonate

To a solution of 1 eq. starting material and 1.5 eq. diisopropyl ethyl amine in 3 ml/mmol dichloromethane was added 1.3 eq. mesyl chloride in some dichloromethane dropwisely at -10°C. The stirred reaction mixture was warmed over a period of 4,5 h to room temperature and diluted with dichloromethane. The organic phase was washed with saturated sodium hydrogen carbonate solution, water and brine. The organic phase was dried with magnesium sulfate. The crude product was purified by silica column chromatography (ethyl acetate-hexane gradient).

### K: Conversion of alcohol to corresponding O- sulfonate (version 2)

To a solution of 1 eq. starting material in dichloromethane (1.4 ml/eq.) and pyridine (1.4 ml/eq.) pyridine was added (1.1 eq.) aryl sulfonyl chloride in dichloromethane (1ml/eq.) dropwisely at -10°C. The stirred reaction mixture was warmed over a period of 4,5 h to room temperature and diluted with dichloromethane. The organic phase was washed with 0.25 N sulfuric acid (three times), saturated sodium hydrogen carbonate solution, water and brine. The organic phase was dried with magnesium sulfate. The crude product was purified by silica column chromatography (ethyl acetate-hexane gradient).

### L(1): Heterogenous hydrogenation

To a stirred solution of ca. 20-50 mg palladium on coal (10%)) isopropanol (8 ml per 1 mmol starting material) benzyl ether (educt) were added in some iso-propanol. The reaction mixture is stirred at hydrogen atmosphere for 16-20 hours. The reaction mixture is filtered; and the solvent is evaporated. The residue is purified by column chromatography with ethyl acetate-hexane gradient.

### L(2): Hydrogenation with iron

To a stirred solution of 1 eq. starting material (nitro derivative) and 5eq. iron powder in ethanol (∼86 eq) 1 ml/eq. HCl (37% aqueous solution) is added. The solution is refluxed for 1 hour. The solution is cooled to 0°C. 1 N NaOH

(40ml/mmol starting material) is added dropwisely. Dichloromethane and brine are added. The organic phase is separated. The aqueous solution is extracted trice with dichloromethane. The combined organic phases are washed with brine and dried with magnesium sulfate. The solvent is evaporated. The residue is purified by column chromatography with ethyl acetate-hexane gradient.

### W: Reductive amination and subsequent acetylation:

A stirred solution of aldehyde (1 eq.) and amine (1 eq.) in 60 ml dichloroethane (pH = 5) was adjusted with glacial acetic acid to pH=5. To this solution was added 70 mmol sodium tris-acetoxy hydro borane. The reaction mixture was stirred over night and diluted with 5 ml water. The pH value was adjusted with aqueous sodium hydroxide solution to pH = 8-9. The mixture was extracted three times with dichloromethane. The combined organic phases were washed with water and brine and were dried with magnesium sulfate. The desired crude product was obtained after evaporation. The crude product was diluted in dry pyridine (1.3 ml/mmol starting material) and was cooled to 0°C. To this stirred solution was added 1.25 eq. acetic acid anhydride drop by drop. The reaction mixture was stirred over night and reduced to a third of its volume and diluted with dichloromethane (2ml/mmol) and water (2ml/mmol). The aqueous phase is extracted three times with dichloromethane. The combined organic phases are washed with brine and dried with magnesium sulfate. The solvent is evaporated and the residue is purified by column chromatography with ethyl acetate-hexane gradient.

### Z: Deprotection of THP ether:

0.15 eq. PPTS is added to a solution of 1 eq. tetrahydropyranyl ether in 7 ml/mmol methanol. The reaction mixture is stirred over night and poured onto a stirred solution of ice-water and tert-butyl methyl ether. The organic phase is separated. The aqueous phase is extracted three times with tert-butyl methyl ether. The combined organic phases are washed with diluted sodium hydrogen carbonate, brine and dried with magnesium sulfate. The solvent is evaporated and the residue is purified by column chromatography with ethyl acetate-hexane gradient.

### Example 1

### a) Synthesis of N-{2-[2-(benzyloxy)ethoxy]-5-methoxybenzyl}-N-[2-(4-fluorophenoxy)pyridin-3-yl]acetamide (1a)

4.1 g (20 mmol) 2-(4-fluorophenoxy)pyridin-3-amine (Helv. Chim. Acta; 48; 1965; 336-347) and 5.7 g (20 mmol) 2-[2-(benzyloxy)ethoxy]-5-methoxybenzaldehyde (EP1894915A1) were converted according to general procedure W. The desired product was obtained in quantitative yield.
MS-ESI: 517 (M⁺ +1, 100).
Elementary analysis:

| | | | |
|---|---|---|---|
| Calculated: | C 69.75% | H 5.66% | N 5.42% |
| Determined: | C 69.72 % | H 5.67 % | N 5.40% |

### b) Synthesis of N-[2-(4-fluorophenoxy)pyridin-3-yl]-N-[2-(2-hydroxyethoxy)-5-methoxybenzyl]acetamide (1b)

The desired procuct **1b** (1.15 g) was obtained from **1a** (1.67 g, 3,23 mmol) according to the general procedure "L" in 83 % yield.
MS-ESI: 427 (M⁺ +1, 100).
Elementary analysis:

| | | | |
|---|---|---|---|
| Calculated: | C 64.78% | H 5.44% | N 6.57% |
| Determined: | C 64.75% | H 5.45% | N 6.56% |

### c) Synthesis of 2-[2-({acetyl[2-(4-fluorophenoxy)pyridin-3-yl]amino}methyl)-4-methoxyphenoxy]ethyl methanesulfonate (1c)

The desired product **1c** was obtained in 97% yield (350 mg) from **1b** (300 mg, 0.7 mmol) according to the general procedure "I".
MS-ESI: 505 (M⁺ +1, 100).
Elementary analysis:

| | | | |
|---|---|---|---|
| Calculated: | C 57.13% | H 4.99% | N 5.55% |
| Determined: | C 57.14% | H 5.00% | N 5.56% |

### d) Synthesis N-[2-(2-[¹⁸F]fluoroethoxy)-5-methoxybenzyl]-N-[2-(4-fluorophenoxy)pyridin-3-yl]acetamide (1d)

The desired product (1 d) was obtained from (1 c) according to general procedure "B".

### e) Synthesis of 2-(2-fluoroethoxy)-5-methoxybenzaldehyde (1e)

The desired product **1e** was obtained according general procedure "H" in 82% yield (82 mmol) from 100 mmol 2-hydroxy-5-methoxybenzaldehyde (Aldrich) and 250 mmol 1-bromo-2-fluoroethane (Aldrich).
MS-ESI: 199 (M⁺ +1, 100). (Aldrich).
Elementary analysis:

| | | |
|---|---|---|
| Calculated: | C 60.60% | H 5.59% |
| Determined: | C 60.61% | H 5.59% |

### f) Synthesis of N-[2-(2-fluoroethoxy)-5-methoxybenzyl]-N-[2-(4-fluorophenoxy)pyridin-3-yl]acetamide (1f) (reference standard)

The desired product **1f** was obtained from 1.51 mmol (309 mg) 2-(4-fluorophenoxy)pyridin-3-amine (Helv. Chim. Acta; 48; 1965; 336-347) and 1.51 mmol (300 mg) **1e** in 88.2 % yield (572mg) acoording to the general procedure "W".
MS-ESI: 429 (M⁺ +1, 100). (Aldrich).
Elementary analysis:

| | | | |
|---|---|---|---|
| Calculated: | C 64.48% | H 5.18% | N 6.54% |
| Determined: | C 64.46% | H 5.19% | N 6.54% |

### Example 2

### a) Synthesis of N-[2-(4-methoxyphenoxy)pyridin-3-yl]-N-{5-methoxy-2-[2-(tetrahydro-2H-pyran-2-yloxy)ethoxy]benzyl}acetamide (2a)

The desired product **2a** was obtained from 463mg 5-methoxy-2-[2-(tetrahydro-2H-pyran-2-yloxy)ethoxy]benzaldehyde (EP1894915A1) and 340mg 2-(4-methoxyphenoxy)pyridin-3-amine (J. Org. Chem.; 60; 16; 1995; 4991-4994) in 55% yield according to the general procedure "W".
MS-ESI: 523 (M⁺ +1, 100).
Elementary analysis:

| | | | |
|---|---|---|---|
| Calculated: | C 66.65% | H 6.56% | N 5.36% |
| Determined: | C 66.63% | H 6.57% | N 5.35% |

### b) Synthesis of N-[2-(2-hydroxyethoxy)-5-methoxybenzyl]-N-[2-(4-methoxyphenoxy)pyridin-3-yl]acetamide (2b)

The desired product **2b** was obtained in 73% yield (265mg) from **2a** (432mg, 0.83 mmol) according to the general procedure "Z".
MS-ESI: 439 (M⁺+1, 100).
Elementary analysis:

| | | | |
|---|---|---|---|
| Calculated: | C 65.74% | H 5.98% | N 6.39% |
| Determined: | C 65.73% | H 5.97% | N 6.39% |

### c) Synthesis of 2-[2-({acetyl[2-(4-methoxyphenoxy)pyridin-3-yl]amino}methyl)-4-methoxyphenoxy]ethyl methanesulfonate (2c)

The desired product **2c** was obtained in 96% yield (289mg) from **2b** (256 mg, 0.58 mmol) according to the general procedure "I".
MS-ESI: 517 (M⁺+1, 100).
Elementary analysis:

| | | | |
|---|---|---|---|
| Calculated: | C 58.13% | H 5.46% | N 5.42% |
| Determined: | C 58.16% | H 5.47% | N 5.41% |

### d) Synthesis of N-[2-(2-[¹⁸F]fluoroethoxy)-5-methoxybenzyl]-N-[2-(4-methoxyphenoxy)pyridin-3-yl]acetamide (2d)

The desired product (2d) was obtained from (2c) according to general procedure "B".

### e) Synthesis of N-[2-(2-fluoroethoxy)-5-methoxybenzyl]-N-[2-(4-methoxyphenoxy)pyridin-3-yl]acetamide (2e)

The desired product **2e** was obtained from 0.3 mmol (64.3mg) 2-(4-methoxyphenoxy)pyridin-3-amine (J. Org. Chem.; 60; 16; 1995; 4991-4994) and 58.9 mg (0.3mmol) **1e** in 76 % yield (100 mg) acoording to the general procedure "W".
MS-ESI: 441 (M⁺+1, 100) (Aldrich)
Elementary analysis:

| | | | |
|---|---|---|---|
| Calculated: | C 65.44% | H 5.72% | N 6.36% |
| Determined: | C 65.42% | H 5.71% | N 6.37% |

### Example 3

### a) Synthesis of N-[2-(4-iodophenoxy)pyridin-3-yl]-N-{5-methoxy-2-[2-(tetrahydro-2H-pyran-2-yloxy)ethoxy]benzyl}acetamide (3a)

The desired product **3a** was obtained from 449mg 5-methoxy-2-[2-(tetrahydro-2H-pyran-2-yloxy)ethoxy]benzaldehyde (EP1894915A1) and 500 mg 2-(4-iodophenoxy)pyridin-3-amine J. Chem. Soc. (1931), 529,533 in 75% yield (747 mg) according to the general procedure "W".
MS-ESI: 619 (M⁺ +1, 100).
Elementary analysis:

| | | | |
|---|---|---|---|
| Calculated: | C 54.38% | H 5.05% | N 4.53% |
| Determined: | C 54.38% | H 5.05% | N 4.53% |

### b) N-[2-(2-hydroxyethoxy)-5-methoxybenzyl]-N-[2-(4-iodophenoxy)pyridin-3-yl]acetamide (3b)

The desired product **3b** was obtained in 75% yield (459mg) from **3a** (707mg, 1.14 mmol) according to the general procedure "Z".
MS-ESI: 535 (M⁺ +1, 100).
Elementary analysis:

| | | | |
|---|---|---|---|
| Calculated: | C 51.70% | H 4.34% | N 5.24% |
| Determined: | C 51.72% | H 4.35% | N 5.23% |

### c) Synthesis of 2-[2-({acetyl[2-(4-iodophenoxy)pyridin-3-yl]amino}methyl)-4-methoxyphenoxy]ethyl methanesulfonate

The desired product **3c** was obtained in 96% yield (494mg) from **3b** (431 mg, 0.81 mmol) according to the general procedure "I".
MS-ESI: 613 (M⁺ +1, 100).
Elementary analysis:

| | | | |
|---|---|---|---|
| Calculated: | C 47.07% | H 4.11% | N 4.57% |
| Determined: | C 47.10% | H 4.12% | N 4.56% |

### d) Synthesis of N-[2-([¹⁸F]2-fluoroethoxy)-5-methoxybenzyl]-N-[2-(4-iodophenoxy)pyridin-3-yl]acetamide (3d)

The desired product **(3d)** was obtained from **(3c)** according to general procedure "B".

### e) Synthesis of N-[2-(2-fluoroethoxy)-5-methoxybenzyl]-N-[2-(4-iodophenoxy)pyridin-3-yl]acetamide (3e)

The desired product **3e** was obtained from 0.23 mmol (71 mg) 2-(4-iodophenoxy)pyridin-3-amine J. Chem. Soc. (1931), 529, 533 and 45.1mg (0.23mmol) **1e** in 37 % yield (37,2 mg) acoording to the general procedure "W".
MS-ESI: 537 (M⁺ +1, 100).
Elementary analysis:

| | | | |
|---|---|---|---|
| Calculated: | C 51.51% | H 4.13% | N 5.22% |
| Determined: | C 51.53% | H 4.14% | N 5.21% |

### Example 4

### a) Synthesis of N-[2-(2-fluorophenoxy)pyridin-3-yl]-N-{5-methoxy-2-[2-(tetrahydro-2H-pyran-2-yloxy)ethoxy]benzyl}acetamide (4a)

The desired product **4a** was obtained from 0.25g (1.22 mmol) 2-(4-fluorophenoxy)pyridin-3-amine (ABCR).and 342 mg (1.17 mmol) 5-methoxy-2-[2-(tetrahydro-2H-pyran-2-yloxy)ethoxy]benzaldehyde (EP1894915A1) in 66% yield (412mg) according to general procedure W.
MS-ESI: 511 (M⁺+1, 100).
Elementary analysis:

| | | | |
|---|---|---|---|
| Calculated: | C 65.87% | H 6.12% | N 5.49% |
| Determined: | C 65.85% | H 6.11% | N 5.49% |

### b) Synthesis of N-[2-(2-fluorophenoxy)pyridin-3-yl]-N-[2-(2-hydroxyethoxy)-5-methoxybenzyl]acetamide (4b)

The desired product **4b** was obtained in 84% yield (140mg) from **4a** (200mg, 0.39 mmol) according to the general procedure "Z".
MS-ESI: 427 (M⁺ +1, 100).
Elementary analysis:

| | | | |
|---|---|---|---|
| Calculated: | C 65.87% | H 6.12% | N 5.49% |
| Determined: | C 65.85% | H 6.11% | N 5.49% |

### c) Synthesis of 2-[2-({acetyl[2-(2-fluorophenoxy)pyridin-3-yl]amino}methyl)-4-methoxyphenoxy]ethyl methanesulfonate (4c)

The desired product **4c** was obtained in 71% yield (102mg) from 4b (122mg, 0.29 mmol) according to the general procedure "I".
MS-ESI: 505 (M⁺ +1, 100).
Elementary analysis:

| | | | |
|---|---|---|---|
| Calculated: | C 57.13% | H 4.99% | N 5.55% |
| Determined: | C 57.15% | H 5.00% | N 5.56% |

### d) Synthesis of N-[2-(2-[¹⁸F]fluoroethoxy)-5-methoxybenzyl]-N-[2-(2-fluorophenoxy)pyridin-3-yl]acetamide (4d)

The desired product (4d) was obtained from 4c according to general procedure "B".

### e) Synthesis of N-[2-(2-fluoroethoxy)-5-methoxybenzyl]-N-[2-(2-fluorophenoxy)pyridin-3-yl]acetamide (4e)

The desired product **4e** was obtained from 103 mg (0.5 mmol) 2-(4-fluorophenoxy)pyridin-3-amine (ABCR).and 100 mg (0.5 mmol) **1e** in 74 % yield (159 mg) acoording to the general procedure "W".
MS-ESI: 429 (M⁺ +1, 100).
Elementary analysis:

| | | | |
|---|---|---|---|
| Calculated: | C 64.48% | H 5.18% | N 6.54% |
| Determined: | C 64.47% | H 5.19% | N 6.53% |

### Example 5

### a) Synthesis of N-[2-(2,3-dimethylphenoxy)pyridin-3-yl]-N-{5-methoxy-2-[2-(tetrahydro-2H-pyran-2-yloxy)ethoxy]benzyl}acetamide (5a)

250 mg (1,17 mg) 2-(2,3-dimethylphenoxy)pyridin-3-amine (ABCR) and 327 mg (1,17 mg) 5-methoxy-2-[2-(tetrahydro-2H-pyran-2-yloxy)ethoxy]benzaldehyde (EP1894915A1) were converted according to general procedure W. The desired product **5a** (442 mg) was obtained in 73% yield.
MS-ESI: 621 (M⁺+1, 100).
Elementary analysis:

| | | | |
|---|---|---|---|
| Calculated: | C 69.21% | H 6.97% | N 5.38% |
| Determined: | C 69.20% | H 6.98% | N 5.37% |

### b) Synthesis of N-[2-(2,3-dimethylphenoxy)pyridin-3-yl]-N-[2-(2-hydroxyethoxy)-5-methoxybenzyl]acetamide (5b)

The desired product **5b** was obtained in 73% yield (122mg) from **5a** (200mg, 0.38 mmol) according to the general procedure "Z".
MS-ESI: 437 (M⁺ +1, 100).
Elementary analysis:

| | | | |
|---|---|---|---|
| Calculated: | C 68.79% | H 6.47% | N 6.42% |
| Determined: | C 68.77% | H 6.46% | N 6.43% |

### c) Synthesis of 2-[2-((acetyl[2-(2,3-dimethylphenoxy)pyridin-3-yl]amino)methyl)-4-methoxyphenoxy]ethyl methanesulfonate (5c)

The desired product **5c** was obtained in 60% yield (74mg) from 4b (105mg, 0.24 mmol) according to the general procedure "I".
MS-ESI: 515 (M⁺ +1, 100).
Elementary analysis:

| | | | |
|---|---|---|---|
| Calculated: | C 60.69% | H 5.88% | N 5.44% |
| Determined: | C 60.68% | H 5.89% | N 5.43% |

### d) Synthesis of N-[2-(2,3-dimethylphenoxy)pyridin-3-yl]-N-[2-(2-[18F]fluoroethoxy)-5-methoxybenzyi]acetamide (5d)

The desired product **(5d)** was obtained from 5c according to general procedure "B".

### e) Synthesis of N-[2-(2,3-dimethylphenoxy)pyridin-3-yl]-N-[2-(2-fluoroethoxy)-5-methoxybenzyl]acetamide (5e)

The desired product **5e** was obtained from 108 mg (0.5mg) 2-(2,3-dimethylphenoxy)pyridin-3-amine (ABCR) and 100mg (0.5 mmol) **1e** in 17 % yield (38 mg) acoording to the general procedure "W".
MS-ESI: 439 (M⁺ +1, 100) (Aldrich)
Elementary analysis:

| | | | |
|---|---|---|---|
| Calculated: | C 68.48% | H 6.21% | N 6.39% |
| Determined: | C 68.46% | H 6.22% | N 6.38% |

### Example 6

### a) Synthesis of N-{5-methoxy-2-[2-(tetrahydro-2H-pyran-2-yloxy)ethoxy]benzyl}-N-(2-phenoxypyridin-3-yl)acetamide (6a)

244 mg (1.31mmol) 2-(phenoxy)pyridin-3-amine (J. Med. Chem. (2002), 45, 23, 5182) and 367 mg (1.31 mmol) 5-methoxy-2-[2-(tetrahydro-2H-pyran-2-yloxy)ethoxy]benzaldehyde (EP1894915A1) were converted according to general procedure W. The desired **product 6a** was obtained in 50% yield (322mg; 648 micromol).
MS-ESI: 492 (M⁺+1, 100).
Elementary analysis:

| | | | |
|---|---|---|---|
| Calculated: | C 68.28% | H 6.55% | N 5.69% |
| Determined: | C 68.27% | H 6.56% | N 5.68% |

### b) Synthesis of N-[2-(2-hydroxyethoxy)-5-methoxybenzyl]-N-(2-phenoxypyridin-3-yl)acetamide (6b)

The desired product **6b** (438 micromol; 179mg) was obtained in 73% yield from **6a** (295mg, 0.6 mmol) according to the general procedure "Z".
MS-ESI: 409 (M⁺ +1, 100).
Elementary analysis:

| | | | |
|---|---|---|---|
| Calculated: | C 67.63% | H 5.92% | N 6.86% |
| Determined: | C 67.62% | H 5.93% | N 6.85% |

### c) Synthesis of 2-(2-([acetyl(2-phenoxypyridin-3-yl)amino]methyl)-4-methoxyphenoxy)ethyl methanesulfonate (6c)

The desired product **6c** was obtained in 94% yield (146 mg, 0.3 mmol) from 6b (130mg, 0.32 mmol) according to the general procedure "I".
MS-ESI: 487 (M⁺ +1, 100).
Elementary analysis:

| | | | |
|---|---|---|---|
| Calculated: | C 59.25% | H 5.39% | N 5.76% |
| Determined: | C 59.27% | H 5.40% | N 5.77% |

### d) Synthesis of N-[2-([¹⁸F]2-fluoroethoxy)-5-methoxybenzyl]-N-(2-phenoxypyridin-3-yl)acetamide (6d)

The desired product **(6d)** was obtained from 6c according to general procedure "B".

### e) Synthesis of N-[2-(2-fluoroethoxy)-5-methoxybenzyl]-N-(2-phenoxypyridin-3-yl)acetamide (6e)

The desired product **6e** was obtained from 244 mg (1.31 mmol) 2-(phenoxy)pyridin-3-amine (J. Med. Chem. (2002), 45, 23, 5182) and 260mg (1.31 mmol) **1e** in 82 % yield (442mg) acoording to the general procedure "W".
MS-ESI: 411 (M⁺+1, 100) (Aldrich)
Elementary analysis:

| | | | |
|---|---|---|---|
| Calculated: | C 67.31% | H 5.65% | N 6.83% |
| Determined: | C 67.30% | H 5.66% | N 6.82% |

### Example 7

### a) Synthesis of 2-[4-(2-tetrahydropyranyloxy-ethoxy)-phenoxy]-3-nitro-pyridine (7a)

The desired product **7a** was synthesized according to a modified procedure by Alsaidi et al. (Synthesis; 11; 1980; 921 - 924) using 2-chloro-3-nitro-pyridine (Aldrich) and 4-(2-tetrahydropyranyloxy-ethoxy)-phenol (J. Med. Chem. (1998), 41, 9, 1540-1554). The desired product **7a** was obtained in 76% yield.
MS-ESI: 361 (M+⁺1, 100).
Elementary analysis:

| | | | |
|---|---|---|---|
| Calculated: | C 59.99% | H 5.59% | N 7.77% |
| Determined: | C 60.00% | H 5.58% | N 7.75% |

### b) 2-[4-(2-tetrahydropyranyloxy-ethoxy)-phenoxy]-pyridin-3-ylamine (7b)

The desired product **7b** (526 mg; 1.6 mmol) was obtained from **7a** (722 mg; 2.0 mmol) according to the general procedure"L2".
MS-ESI: 330 (M⁺ +1, 100).
Elementary analysis:

| | | | |
|---|---|---|---|
| Calculated: | C 65.44% | H 6.71% | N 8.48% |
| Determined: | C 65.42% | H 6.70% | N 8.47% |

### c) Synthesis of N-(2,5-dimethoxybenzyl)-N-(2-{4-[2-(tetrahydro-2H-pyran-2-yloxy)ethoxy]phenoxy}pyridin-3-yl)propanamide (7c)

MS-ESI: 537 (M⁺ +1, 100).
Elementary analysis:

| | | | |
|---|---|---|---|
| Calculated: | C 67.15% | H 6.76% | N 5.22% |
| Determined: | C 67.12% | H 6.75% | N 5.21% |

The desired product 7c (436 mg) was obtained from 7b (1,21 mmol; 400 mg) and 2,5-dimethoxy-benzaldehyde (Aldrich) according to general procedure W with the exception that not acetic acid anhydride but propionyl chloride was used. The desired product was obtained in 67% yield (0.81 mmol).

### d) Synthesis of N-(2,5-dimethoxybenzyl)-N-(2-{4-[2-(tetrahydro-2H-pyran-2-yloxy)ethoxy]phenoxy}pyridin-3-yl)propanamide (7d)

The desired product **7d** was obtained in 75% yield (0.49 mmol; 221 mg) from **7c** (350mg, 0.65 mmol) according to the general procedure "Z".
MS-ESI: 453 (M⁺ +1, 100).
Elementary analysis:

| | | | |
|---|---|---|---|
| Calculated: | C 66.36% | H 6.24% | N 6.19% |
| Determined: | C 66.36% | H 6.24% | N 6.19% |

### e) Synthesis of 2-[4-({3-[(2,5-dimethoxybenzyl)(propanoyl)amino]pyridin-2-yl}oxy)phenoxy]ethyl 4-methylbenzenesulfonate (7e)

MS-ESI: 607 (M⁺+1, 100).
Elementary analysis:

| | | | |
|---|---|---|---|
| Calculated: | C 63.35% | H 5.65% | N 4.62% |
| Determined: | C 63.33% | H 5.65% | N 4.63% |

The desired product **7e** (0.26 mmol; 158 mg) was obtained from **7d** (0.33 mmol, 150 mg) according to the general procedure K in 75% yield.

### f) Synthesis of N-(2,5-dimethoxybenzyl)-N-{2-[4-(2-[¹⁸F]fluoroethoxy)phenoxy]pyridin-3-yl}propanamide (7f)

The desired product **(7f)** was obtained from **(7e)** according to general procedure "B".

### g) Synthesis of N-(2,5-dimethoxybenzyl)-N-{2-[4-(2-fluoroethoxy)phenoxy]pyridin-3-yl}propanamide (7g)

The desired product **7g** (48mg; 0.106 mmol) was synthesized from **7f** (0.156 mmol; 94 mg) according to the general procedure "A" in 68% yield
MS-ES1:455(M⁺+1, 100).
Elementary analysis:

| | | | |
|---|---|---|---|
| Calculated: | C 66.07% | H 5.99% | N 6.16% |
| Determined: | C 66.07% | H 5.99% | N 6.16% |

### Biology

The aim of the present invention was to find an improved F-18 labelled compound in comparison to the current state of the art that can be used to detect activated microglia by means of PET Imaging targeting the peripheral benzodiazepine receptor (PBR) also known as 18 kDa translocator protein (TSPO). As the data of the present invention demonstrate, the compounds [¹⁸F]-**2d** and [¹⁸F]-**5d** surprisingly showed an improved signal-to-background ratio in kainic acid induced brain lesions in rats compared to the known tracers [¹⁸F]-FEDAA1106 (3) and [¹⁸F]-DPA-714 **(1.2).**

The biodistribution of [¹⁸F]-**2d** and [¹⁸F]-**5d** was investigated in healthy male NMRI mice (28.3 -35.6 g body weight, n=3 animals per time point) at 2, 5, 30, 60 and 180 / 240 min after intravenous injection of 0.264 MBq [¹⁸F]-**2d** and 0.268 MBq [¹⁸F]-**5d** per animal, respectively. Until the indicated time points urine and faeces were quantitatively collected. At the respective time points the mice were sacrificed and the tissues were removed. [¹⁸F]-radioactivity was analyzed in a gamma counter (Tab. 1.1, 2.1 and 1.2, 2.2).

[¹⁸F]-**2d** showed a high initial brain uptake of [¹⁸F]-radioactivity (1.37 ± 0.04% injected dose/g at 2 min p.i.) and a high initial elimination of ca. 72% of the radioactivity from the brain 30 min p.i. (0.37 ± 0.04 % injected dose/g; Fig. 1.1) with a 2 min / 30 min ratio of 3.7 (Tab.3). Overall, the amount of radioactivity in blood and brain decreased over the investigated time period. No relevant bone uptake of radioactivity was detected (2.3 % injected dose/g at 180 min). Substantial radioactivity accumulated in organs with a known constitutive PBR expression (e.g. lung, heart, adrenals).

The excretion of radioactivity during the observed time period was mainly via urine (urine 13.09 ± 1.33 % injected dose, faeces 0.59 ± 0.61 % injected dose at 180 min p.i.) (Tab. 1.1).

[¹⁸F]-**5d** showed a high initial brain uptake of [¹⁸F]-radioactivity (1.61 ± 0.23% injected dose/g at 2 min p.i.) and a high initial elimination of ca. 73% of the radioactivity from the brain 30 min p.i. (0.44 ± 0.15% injected dose/g; Fig. 1.2) with a 2 min / 30 min ratio of 3.7 (Tab.3). Overall, the amount of radioactivity in blood and brain decreased over the investigated time period. No relevant bone uptake of radioactivity was detected (2.6 % injected dose/g at 240 min). Substantial radioactivity accumulated in organs with a known constitutive PBR expression (e.g. lung, heart, adrenals).

The excretion of radioactivity during the observed time period was mainly via urine (urine 15.24 ± 1.25 % injected dose, faeces 0.74 ± 1.01 % injected dose at 240 min p.i.) (Tab.1.2).

Using a rat kainic acid induced epilepsy model and respective sham controls the accumulation of [¹⁸F]-**2d** and [¹⁸F]-**5d** was visualized *ex vivo.* In brief, epilepsy was induced in rats by injecting kainic acid i.p.. At day eight after the start of the kainic acid treatment [¹⁸F]-**2d** and [¹⁸F]-**5d** were injected into the tail vein of the rats and their sham treated controls (PBS instead of kainic acid) at a dose of 25.8-35.8 MBq (approximately 1.0 µg) per rat. Thirty minutes after intravenous injection the rats were sacrificed, the brains were taken out, snap frozen and sliced transversally in a cryostat. The slices were exposed to Phospholmager plates over night. The resulting autoradiographic signals were analyzed qualitatively and quantitatively (Fig. 2.1 A and C, 2.2 A and C). After exposure, the sections were immunohistochemically stained with an Ox-42 antibody (anti CD11b/c) to confirm the kainic acid induced microglia activation (Fig. 2.1 D and H, 2.2 D and H,). The signals, located mainly in the hippocampal region as seen by autoradiography from kainic acid treated rats, matched the immunohistochemical signals (Fig. 2.1 D-E and Fig. 2.2 D-E). To determine the specificity of the [¹⁸F]-**2d** and [¹⁸F]-**5d** binding, kainic acid treated rats were coinjected with [¹⁹F]-**2e** and [¹⁹F]-**5e**, respectively. A significantly reduced autoradiographic signal was obtained from these rats in the respective brain regions (Fig. 2.1 B and 2.2 B), while microglia activation could be confirmed in these brain sections by Ox-42 staining (Fig. 2.1 F-G and 2.2 F-G). Sham treated controls received [¹⁸F]-**2d** or [¹⁸F]-**5d** injection only but did not show significant autoradiographic signals in the hippocampus or any other brain region despite those regions with constitutive PBR expression (Fig. 2.1 C and 2.2 C). Immunohistochemistry confirmed the absence of activated microglia (Fig. 2.1 H-I and 2.2 H-I). The autoradiographic signals in the ventricles are due to the known constitutive expression of the PBR in ependymal and choroid plexus cells.

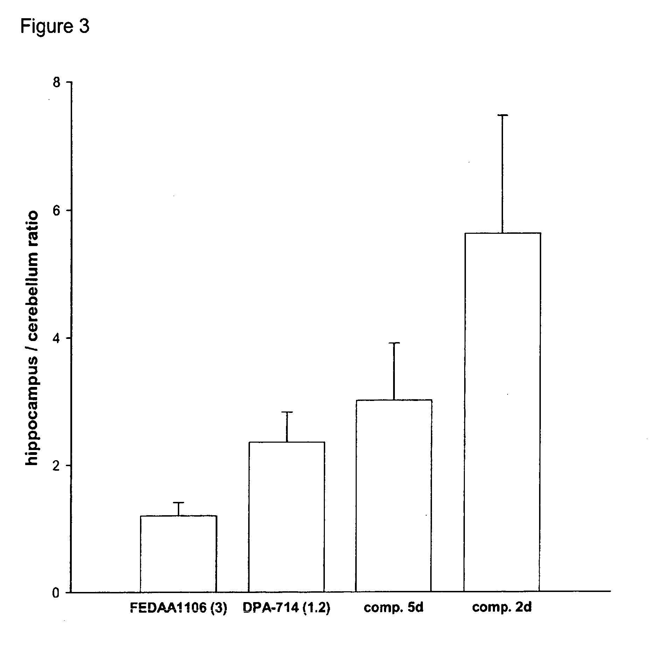

The autoradiographic signals were quantified. The signal intensity in a hippocampal region of interest (ROI) was measured and compared to a ROI in the cerebellum, that was used as reference region. The signal-to-background ratio was expressed as hippocampus / cerebellum ratio (Tab. 3, Fig. 3) and was higher for 5d (3.0 ± 0.9) and 5e (5.6 ± 1.8) compared to [¹⁸F]-FEDAA1106 (1.2 ± 0.2) and [¹⁸F]-DPA-714 (2.4 ± 0.5).

Surprisingly, the signal-to-background ratio induced by [¹⁸F]-**2d** and [¹⁸F]-**5d** as well as their elimination from the brain was superior to that of known substances as [¹⁸F]-FEDAA1106 **(3)** and [¹⁸F]-DPA-714 **(1.2)** (Tab. 3) while all four compounds are high affinity PBR ligands, that do not bind to the CBR (central diazepine receptor) (Tab.4).

**Table 1.1:** Excretion of [¹⁸F]-radioactivity via urine and faeces after [¹⁸F]-**2d** injection in normal mice detected via a gamma-counter, given in % injected dose.

**Table 1.1: % injected dose (decay corrected)**

| Time / Organ: | 2 min | 5 min | 30 min | 60min | 180 min |
|---|---|---|---|---|---|
| **Urine** | 0.02 ± 0.01 | 0.02 ± 0.01 | 1.72 ± 0.08 | 4.21 ± 0.39 | 13.09 ± 1.33 |
| **Faeces** | 0.00 ± 0.00 | 0.02 ± 0.03 | 0.01 ± 0.00 | 0.03 ± 0.03 | 0.59 ± 0.61 |

**Table 1.2:** Excretion of [¹⁸F]-radioactivity via urine and faeces after [¹⁸F]-**5d** injection in normal mice detected via a gamma-counter, given in % injected dose.

**Table 1.2: % injected dose (decay corrected)**

| Time / Organ: | 2 min | 5 min | 30 min | 60min | 240 min |
|---|---|---|---|---|---|
| **Urine** | 0.02 ± 0.02 | 0.04 ± 0.00 | 2.33 ± 1.20 | 4.21 ± 0.97 | 15.24 ± 1.25 |
| **Faeces** | 0.00 ± 0.00 | 0.00 ± 0.00 | 0.03 ± 0.02 | 0.04 ± 0.02 | 0.74 ± 1.01 |

**Table 2.1:** Biodistribution of [¹⁸F]-radioactivity at different time points after [¹⁸F]-**2d** injection in normal mice detected via a gamma-counter in the respective organs, given in % injected dose/g (,n=3 per time point).. The uptake of the tracer in the different organs is consistent with the known local constitutive expression of the PBR.

**Table 2.1: % injected dose/g (decay corrected)**

| Time / Organ | 2 min | 5 min | 30 min | 60min | 180 min |
|---|---|---|---|---|---|
| **Spleen** | 1.50±0.20 | 4.78±0.73 | 6.98±0.82 | 6.09±0.50 | 4.07±0.51 |
| **Liver** | 1.20±0.28 | 1.41±0.26 | 1.61±0.43 | 1.56±0.22 | 1.29±0.20 |
| **Kidney** | 5.92±1.40 | 7.45±1.00 | 10.99±1.08 | 11.04±0.48 | 10.63±1.41 |
| **Lung** | 61.50±10.42 | 34.75±6.56 | 10.66±0.71 | 6.31±0.34 | 4.57±1.44 |
| **Bone** | 0.87±0.11 | 1.30±0.02 | 1.67±0.32 | 1.75±0.09 | 2.25±0.20 |
| **Heart** | 16.53±0.48 | 17.09±0.64 | 7.94±1.52 | 5.22±0.61 | 2.78±0.18 |
| **Brain** | 1.37±0.04 | 0.95±0.07 | 0.37±0.04 | 0.34±0.02 | 0.35±0.02 |
| **Fat** | 0.41±0.49 | 0.25±0.06 | 0.46±0.10 | 0.49±0.06 | 2.02±1.06 |
| **Thyroid** | 2.15±1.49 | 2.65±0.58 | 3.95±0.81 | 3.88±0.10 | 3.91±0.75 |
| **Muscle** | 1.23±0.47 | 1.58±0.52 | 1.80±0.17 | 2.34±0.23 | 1.84±0.29 |
| **Skin** | 0.51±0.14 | 0.60±0.11 | 0.98±0.06 | 1.14±0.06 | 1.60±0.19 |
| **Blood** | 2.35±0.15 | 1.44±0.07 | 0.70±0.14 | 0.60±0.09 | 0.66±0.09 |
| **Stomach** | 2.15±1.25 | 1.89±1.77 | 3.49±0.45 | 4.37±0.51 | 4.37±0.68 |
| **Testes** | 0.48±0.10 | 0.50±0.15 | 0.79±0.11 | 0.84±0.09 | 1.14±0.11 |
| **Adrenals** | 7.37±2.53 | 10.39±3.44 | 15.51±0.48 | 23.01±8.20 | 13.74±7.36 |
| **Intestine** | 0.82±0.04 | 1.36±0.33 | 2.15±0.62 | 2.33±0.34 | 4.28±0.94 |
| **Pancreas** | 1.91±0.33 | 2.23±0.60 | 2.85±0.03 | 2.56±0.26 | 1.76±0.40 |

**Table 2.2:** Biodistribution of [¹⁸F]-radioactivity at different time points after [¹⁸F]-**5d** injection in normal mice detected via a gamma-counter in the respective organs, given in % injected dose/g (n=3 per time point). The uptake of the tracer in the different organs is consistent with the known local constitutive expression of the PBR.

**Table 2.2: % injected dose/g (decay corrected)**

| Time / Origan : | 2 min | 5 min | 30 min | 60min | 240 min |
|---|---|---|---|---|---|
| **Spleen** | 1.40±0.06 | 3.25±0.15 | 5.68±0.59 | 5.79±0.10 | 3.02±0.25 |
| **Liver** | 1.51±0.38 | 2.19±.31 | 3.52±1.35 | 2.35±0.35 | 1.76±0.23 |
| **Kidney** | 6.15±1.95 | 8.53±1.50 | 9.53±0.82 | 9.57±0.76 | 6.84±0.69 |
| **Lung** | 65.87±15.87 | 45.38±5.54 | 9.83±1.66 | 6.46±1.07 | 3.42±0.73 |
| **Bone** | 1.02±0.31 | 1.41±0.19 | 2.05±0.08 | 1.69±0.26 | 2.59±0.56 |
| **Heart** | 17.21±1.43 | 18.45±1.69 | 7.53±2.41 | 5.76±0.87 | 2.21±0.24 |
| **Brain** | 1.61±0.23 | 1.13±0.22 | 0.44±0.51 | 0.34±0.06 | 0.32±0.08 |
| **Fat** | 0.24±0.08 | 0.24±0.03 | 0.61±0.26 | 3.27±0.30 | 0.86±0.10 |
| **Thyroid** | 3.97±1.12 | 4.25±0.79 | 3.81±0.42 | 3.21±0.75 | 2.50±0.15 |
| **Muscle** | 1.23±0.27 | 1.89±0.32 | 2.14±0.31 | 2.13±0.21 | 1.32±0.11 |
| **Skin** | 0.52±0.02 | 0.79±0.07 | 1.23±0.21 | 1.39±0.10 | 1.47±0.06 |
| **Blood** | 2.75±0.40 | 2.13±0.23 | 0.80±0.17 | 0.75±0.01 | 0.60±0.07 |
| **Stomach** | 2.03±1.05 | 3.04±0.49 | 3.01±0.70 | 4.21±0.25 | 3.43±0.54 |
| **Testes** | 0.50±0.01 | 0.71±0.10 | 0.75±0.19 | 0.90±0.10 | 0.94±0.12 |
| **Adrenals** | 9.90±1.17 | 28.94±11.26 | 12.31±0.43 | 16.66±2.86 | 13.93±6.07 |
| **Intestine** | 0.77±0.24 | 1.42±0.02 | 2.48±0.78 | 3.31±0.16 | 3.96±0.16 |
| **Pancreas** | 1.54±0.34 | 2.37±0.56 | 2.46±0.19 | 2.34±0.48 | 1.41±0.22 |

**Table 3:** Comparison of different parameters of [¹⁸F]-**2d**, [¹⁸F]-**5d**_{,} [¹⁸F]-FEDAA1106 (3) and [¹⁸F]-DPA-714 (1.2).

**Table 3:**

| | **[¹⁸F]-FEDAA1106** | **[¹⁸F]-DPA-714** | **[¹⁸F] -2d** | **[¹⁸F] -5d** |
|---|---|---|---|---|
| **Hipp/ Cereb ratio** rat kainic acid model: 30 min ex *vivo* autoradiography | 1.2 ± 0.2 | 2.4 ± 0.5 | 5.6 ± 1.8 | 3.0 ± 0.9 |
| **Elimination from mouse brain** 2min / 30 min ratio | 2.0 | 2.4 | 3.7 | 3.7 |

**Table 4:** Comparison of different parameters of [¹⁹F]-**2e**, [¹⁹F]-**5e**, [¹⁹F]-FEDAA1106 and [¹⁹F]-DPA-714

**Table 4:**

| | **[¹⁹F]-FEDAA1106** | **[¹⁹F]-DPA-714** | **[¹⁹F]-2e** | **[¹⁹F]-5e** |
|---|---|---|---|---|
| **PBR Kᵢ (nM)** [³H]-PK11195 | 1.5 ± 0.15 | 1.46 ± 0.12 | 3.6 ± 1.3 | 2.57 ± 0.06 |
| **CBR Kᵢ (nM)** [³H]-Flumazenil | >20000 | >20000 | >20000 | >20000 |

In particular, the invention relates to
1. A compound of formula I 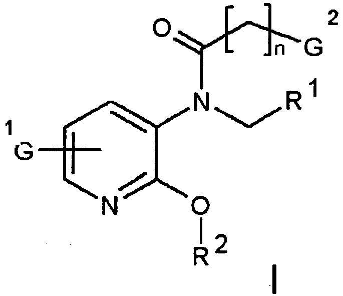 wherein
   **R¹** and **R²** are independently and individually, at each occurrence, selected from the group consisting of (G³)aryl, substituted (G³)aryl, (G³-(C₁-C₈)alkyl)aryl, (G³-(C₁-C₈)alkoxy)aryl, (G³-(C₂-C₈)alkynyl)aryl, (G³-(C₂-C₈)alkenyl)aryl, substituted (G³-(C₁-C₈)alkyl)aryl, substituted (G³-(C₁-C₈)alkoxy)aryl, substituted (G³-(C₂-C₈)alkynyl)aryl and substituted (G³-(C₂-C₈)alkenyl)aryl;
   **G¹, G²** and **G³** are independently and individually, at each occurrence, selected from the group consisting of hydrogen and **L,**
      with the proviso that compounds of formula I contain exactly one **L;**
   **L** is selected from the group consisting of **R³,** [¹⁸F]fluoro and and [¹⁹F]fluoro;
   **R³** is a leaving group;
   wherein **n** is an integer from 0 to 6;
   including all isomeric forms of said compound, including but not limited to enantiomers and diastereoisomers as well as racemic mixtures,
   and any pharmaceutically acceptable salt, ester, amide, complex or prodrug thereof.
2. The compound according to count 1, wherein **R³** is selected from the group consisting of -I⁺(aryl)(X⁻), -I⁺(heteroaryl)(X⁻), nitro, -N⁺(Me)₃(X⁻), halo, in particular chloro, bromo and iodo, mesyloxy, tosyloxy, trifluormethylsulfonyloxy, nona-fluorobutylsulfonyloxy, (4-bromo-phenyl)sulfonyloxy, (4-nitro-phenyl)sulfonyloxy, (2-nitro-phenyl)sulfonyloxy, (4-isopropyl-phenyl)sulfonyloxy, (2,4,6-tri-isopropylphenyl)sulfonyloxy, (2,4,6-trimethyl-phenyl)sulfonyloxy, (4-tertbutyl-phenyl)sulfonyloxy, and (4-methoxy-phenyl)sulfonyloxy.
3. The compound according to count 2, wherein X⁻ is selected from the group consisting of anion of an inorganic acid and anion of an organic acid.
4. The compound according to any of counts 3, wherein X⁻ is selected from the group consisting of CF₃S(O)₂O⁻, C₄F₉S(O)₂O⁻, CF₃COO⁻, H₃CCOO⁻, iodide anion, bromide anion, chloride anion, perchlorate anion (ClO₄⁻), and phosphate anion.
5. A compound according to any of the foregoing counts which is selected from the group of compounds consisting of 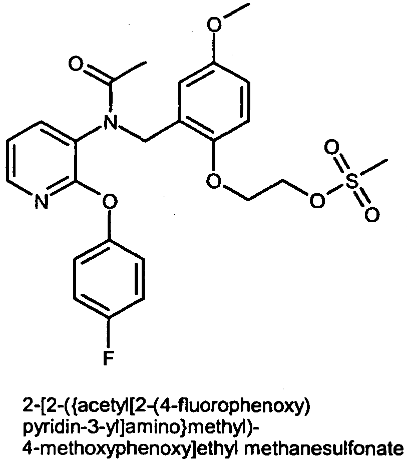 or 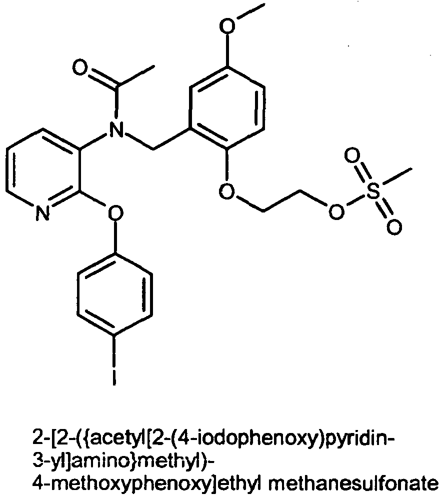 or 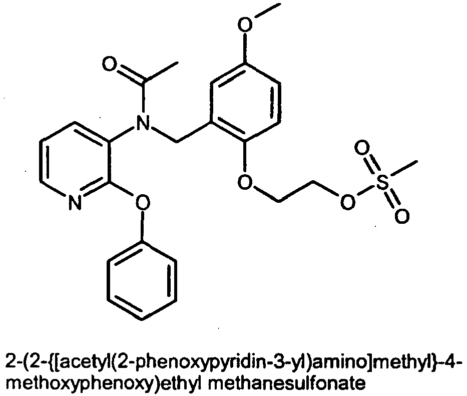 or 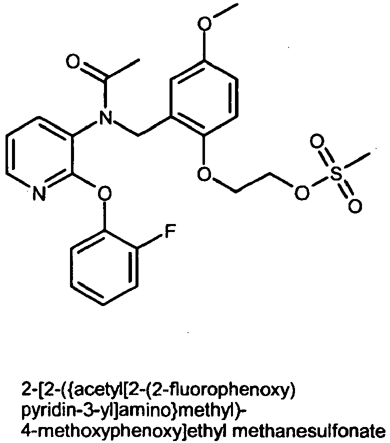 or 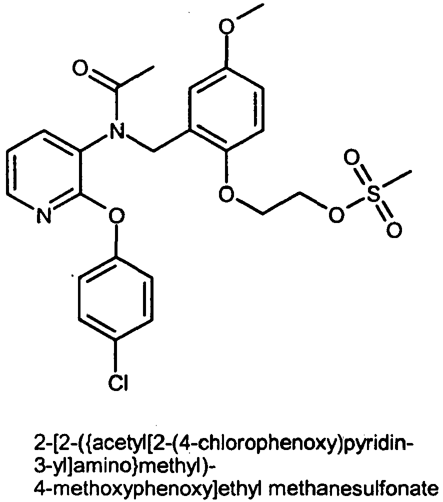 or 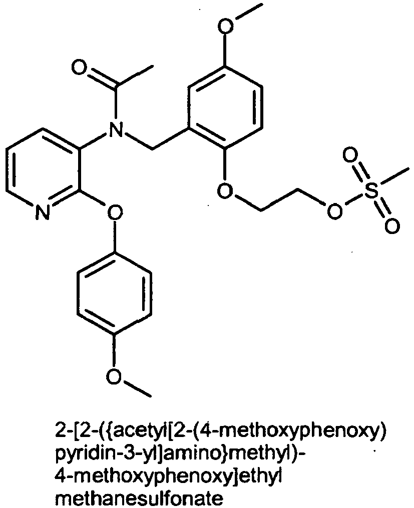 or 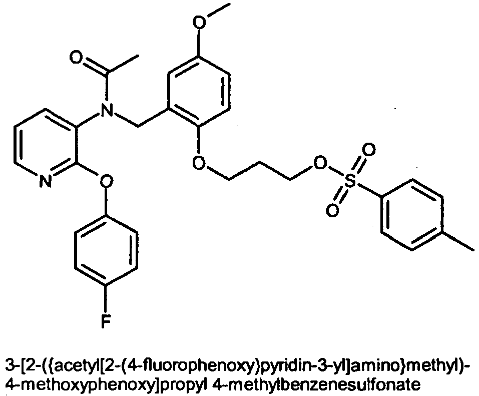 or 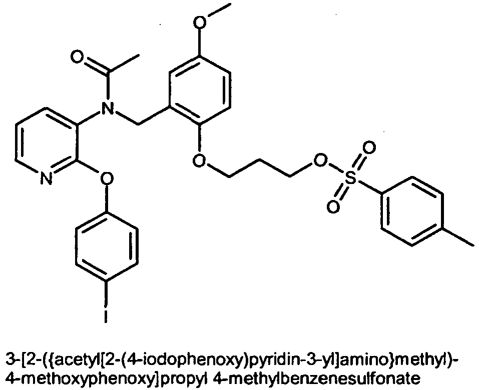 or 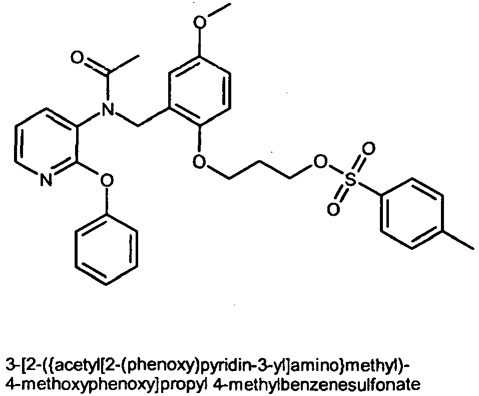 or 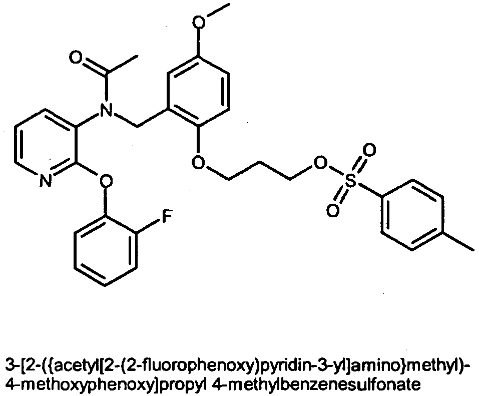 or 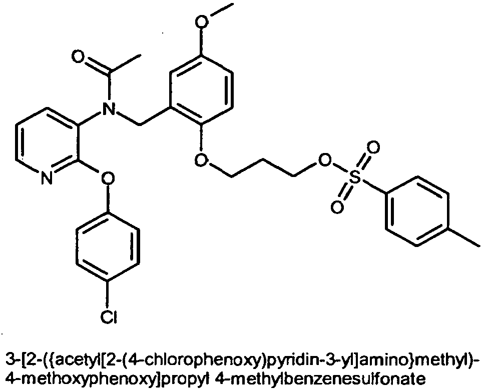 or 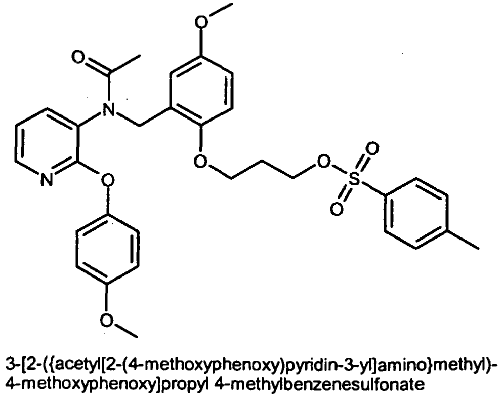
6. A compound of the formula 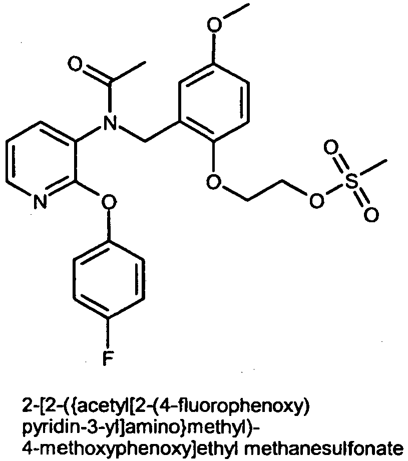
7. A compound of the formula 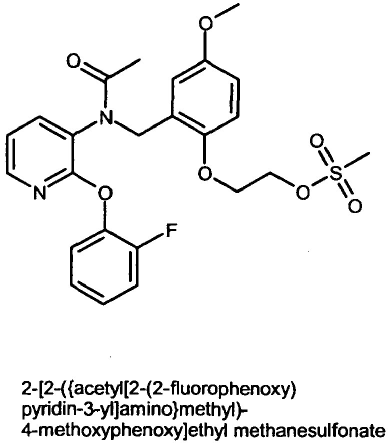
8. The compound according to any of counts 1-4 wherein L is not fluoro, in particular not [¹⁸F]fluoro and not [¹⁹F]fluoro.
9. The compound according to any of counts 1-4, wherein L is [¹⁸F]fluoro or a compound of count 5, 6 or 7 wherein the mesyloxy-group and the tosyloxy-group is replaced by [¹⁸F]fluoro.
10. The compound according to any of counts 1-4, wherein L is [¹⁹F]fluoro or a compound of count 5, 6 or 7, wherein the mesyloxy-group and the tosyloxy-group is replaced by [¹⁹F]fluoro.
11. A method of synthesis of a compound as defined in count 9 or 10, in which a compound according to count 1-8 is reacted with an F-fluorinating agent, wherein F= ¹⁸F or ¹⁹F.
12. The method according to count 11, wherein said F-fluorinating agent is a compound consisting of F-anions, preferably a compound selected from the group consisting of 4, 7, 13, 16, 21, 24-hexaoxa-1,10-diazabicyclo[8.8.8]-hexacosane K F, i.e. crownether salt Kryptofix KF, KF, HF, KH F₂, CsF, NaF and tetraalkylammonium salts of F, such as [¹⁸F]tetrabutylammonium fluoride, and wherein F = ¹⁸F or ¹⁹F.
13. A compound of formula VI 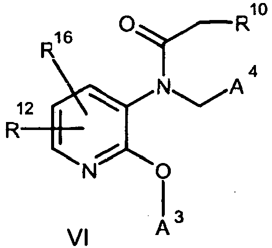 wherein
   **R¹⁰** is selected from the group consisting of (C₁-C₆)alkyl and hydrogen;
   **R¹⁶** is selected from the group consisting of hydrogen, halo, trifluoromethyl, (C₁-C₅)alkyl, (C₂-C₅)alkynyl), (C₂-C₅)alkenyl and (C₁-C₅)alkoxy;
   **A³** and **A⁴** are the same or different and of the structure (R¹²)(R⁴)(R⁵)phenyl;
   **R¹²** is selected from the group consisting of **R¹³** and hydrogen;
   **R¹³** is hydroxy;
      with the proviso that compounds of formula VI contain exactly one **R¹³ .**
   **R⁴** and **R⁵** are independently and individually, at each occurrence, selected from the group consisting of hydrogen, halo, trifluoromethyl, (C₁-C₅)alkyl, (C₂-C₅)alkynyl, (C₂-C₅)alkenyl and (C₁-C₅)alkoxy;
   including all isomeric forms of said compound, including but not limited to enantiomers and diastereoisomers as well as racemic mixtures,
   and any pharmaceutically acceptable salt, ester, amide, complex or prodrug thereof.
14. A method of synthesis of a compound as defined in count 9 or count 10, consisting of the steps:
   - F-fluorinating a compound of formula V 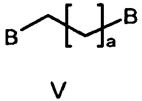
   formula V
   with an F-fluorinating agent to yield a compound of formula IV, 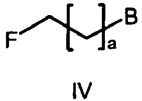 formula IV
   - substituting said compound of formula IV with a compound of formula VI

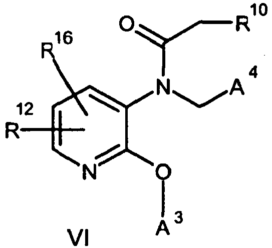

   wherein F in Formula IV is [¹⁸F]fluoro or [¹⁹F]fluoro,
   a is an integer from 0 to 5,
   **B** is a leaving group,
   **R¹⁰** is selected from the group consisting of (C₁-C₆)alkyl and hydrogen;
   **R¹⁶** is selected from the group consisting of hydrogen, halo, trifluoromethyl, (C₁-C₅)alkyl, (C₂-C₅)alkynyl), (C₂-C₅)alkenyl and (C₁-C₅)alkoxy;
   **A³** and **A⁴** are the same or different and of the structure (R¹²)(R⁴)(R⁵)phenyl;
   **R¹²** is selected from the group consisting of R¹³ and hydrogen;
   **R¹³** is hydroxy,
      with the proviso that compounds of formula VI contain exactly one **R¹³;**
   **R⁴** and **R⁵** are independently and individually, at each occurrence, selected from the group consisting of hydrogen, halo, trifluoromethyl, (C₁-C₅)alkyl, (C₂-C₅)alkynyl), (C₂-C₅)alkenyl and (C₁-C₅)alkoxy;
   including all isomeric forms of said compound, including but not limited to enantiomers and diastereoisomers as well as racemic mixtures,
   and any pharmaceutically acceptable salt, ester, amide, complex or prodrug thereof
   and
   wherein said F-fluorinating agent is as defined in count 10,
   and wherein F = ¹⁸F or ¹⁹F,
   with the proviso that compounds of formula VI contain exactly one R¹³.
15. The method according to count 14, wherein B is selected from the group consisting of iodo, bromo, chloro, mesyloxy, tosyloxy, trifluormethylsulfonyloxy, and nona-fluorobutylsulfonyloxy.
16. A composition consisting of a compound according to any of counts 1-10 and 13 and a pharmaceutically acceptable carrier or diluent.
17. The composition according to count 16, wherein said compound is a compound according to count 9.
18. The composition according to count 16, wherein said compound is a compound according to count 10.
19. The composition according to count 16, wherein said compound is a compound according to count 8.
20. The composition according to count 16, wherein said compound is a compound according to count 13.
21. A compound according to any of counts 1-10, preferably a compound according to count 8 or 9, 31 or 32 or a composition according to any of counts 16, 17, 18, 19, 20 or 36 as a pharmaceutical or diagnostic agent or imaging agent.
22. Use of a compound according to any of counts 1-10, preferably a compound according to count 9, 10, 31 or 32 or a composition according to any of counts 16, 17, 18, 19 or 20 for the manufacture of a medicament for the treatment and/or diagnosis and/or imaging of diseases of the central nervous system (CNS).
23. A compound according to count 9, 31 or 32 or a composition according to count 17 or 36 for use as a diagnostic agent or imaging agent, in particular for diseases of the central nervous system.
24. A compound according to count 9 having the structure 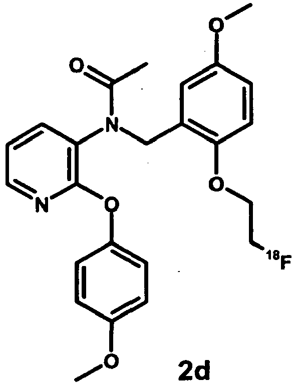
25. A compound according to count 9 having the structure 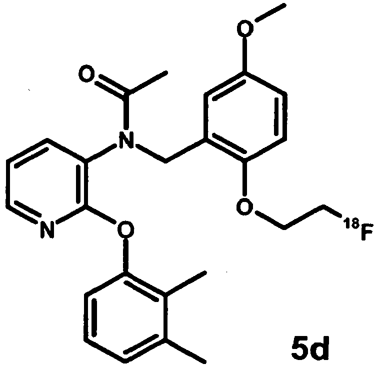
26. A compound according to counts 32 and 33 as a diagnostic compound.
27. A compound according to counts 32 and 33 as a diagnostic compound useful for PET imaging of Alzheimer's disease.
28. A pharmaceutical or diagnostic composition comprising a compound according to counts 32 and 33.
29. A diagnostic composition according to count 36 for PET imaging of Alzheimer's Disease.
30. A kit comprising a sealed vial comprising a compound according to counts 24 or 25.
31. A pharmaceutical or diagnostic composition comprising a compound according to count 9.
32. A diagnostic composition comprising a compound according to claim 9 for PET imaging.
33. A diagnostic composition according to count 32 for imaging of a neural or CNS disease.
34. A diagnostic composition according to count 33, wherein the disease is Alzheimer's Disease.
35. A method of synthezising a compound according to count 24 or 25 comprising the steps of reacting a suitable precursor molecule with a F-18 fluorinating agent.
36. A method of synthezising a compound according to count 25, comprising fluorinating a compound having the formula 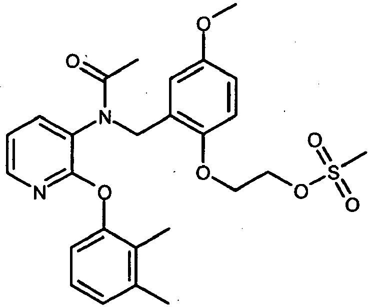 with a suitable F-18 fluorinating agent.

Furthermore the compounds according to count 9, 10, 24 or 25 or the compositions according to count 17 or 28 are useful in the diagnosis of rheumatoid arthritis. In a preferred embodiment, the method of diagnosing rheumatoid arthritis is PET imaging.

## Claims

1. A compound of formula I 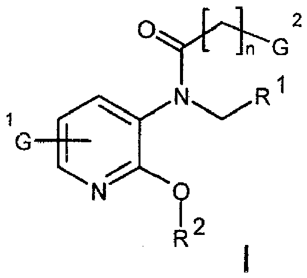 wherein
R¹ and R² are independently and individually, at each occurrence, selected from the group consisting of (G³)aryl, substituted (G³)aryl, (G³-(C₁-C₈)alkyl)aryl, (G³-(C₁-C₈)alkoxy)aryl, (G³-(C₂-C₈)alkynyl)aryl, (G³-(C₂-C₈)alkenyl)aryl, substituted (G³-(C₁-C₈)alkyl)aryl, substituted (G³-(C₁-C₈)alkoxy)aryl, substituted (G³-(C₂-C₈)alkynyl)aryl and substituted (G³-(C₂-C₈)alkenyl)aryl;
G¹, G² and G³ are independently and individually, at each occurrence, selected from the group consisting of hydrogen and L,
with the proviso that compounds of formula I contain exactly one **L;**
**L** is selected from the group consisting of **R³,** [¹⁸F]fluoro and and [¹⁹F]fluoro;
**R³** is a leaving group selected from the group consisting of -I⁺(aryl)(X-),-I⁺(heteroaryl)(X-), nitro, -N⁺(Me)₃(X⁻), halo, in particular chloro, bromo and iodo, mesyloxy, tosyloxy, trifluormethylsulfonyloxy, nona-fluorobutylsulfonyloxy, (4-bromo-phenyl)sulfonyloxy, (4-nitro-phenyl)sulfonyloxy, (2-nifiro-phenyl)sulfonyloxy, (4-isopropyl-phenyl)sulfonyloxy, (2,4,6-tri-isopropyl-phenyl)sulfonyloxy, (2,4,6-trimethyl-phenyl)sulfonyloxy, (4-tertbutyl-phenyl)sulfonyloxy, and (4-methoxy-phenyl)sulfonyloxy;
wherein **X-** is selected from the group consisting of anion of an inorganic acid and anion of an organic acid;
wherein n is an integer from 0 to 6;
and tautomeric, enantiomeric, diastereoisomeric forms as well as racemic mixtures thereof,
and any pharmaceutically acceptable salt thereof.

2. The compound according to claim 1, wherein **X⁻** is selected from the group consisting of CF₃S(O)₂O⁻, C₄F₉S(O)₂O⁻, CF₃COO⁻, H₃CCOO⁻, iodide anion, bromide anion, chloride anion, perchlorate anion (ClO₄⁻), and phosphate anion.

3. A compound according to any of the foregoing claims which is selected from the group of compounds consisting of 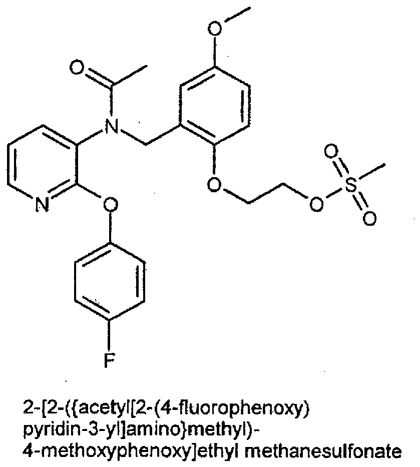 or 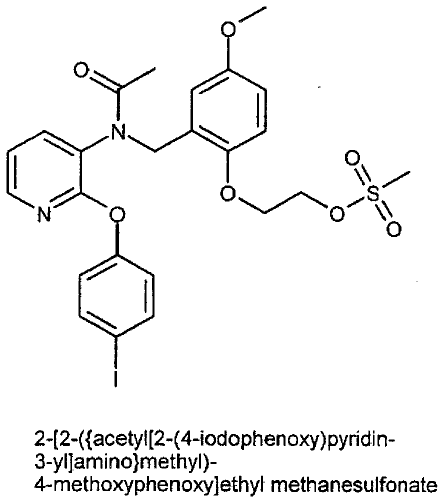 or 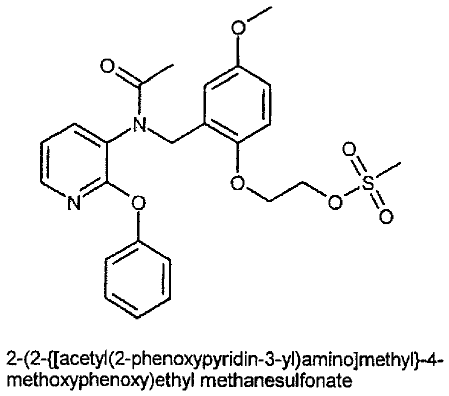 or 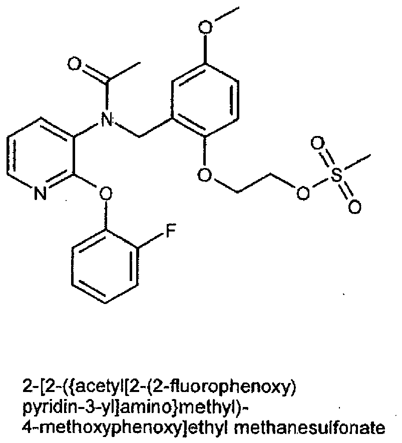 or 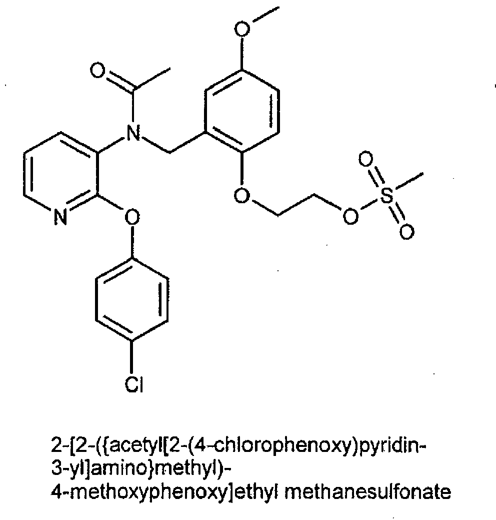 or 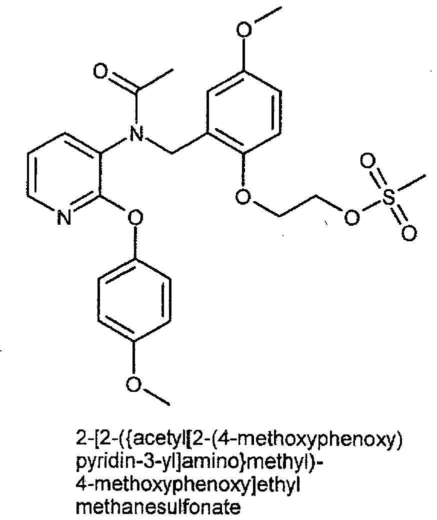 or 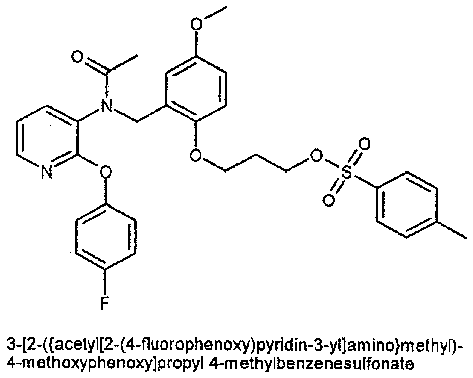 or 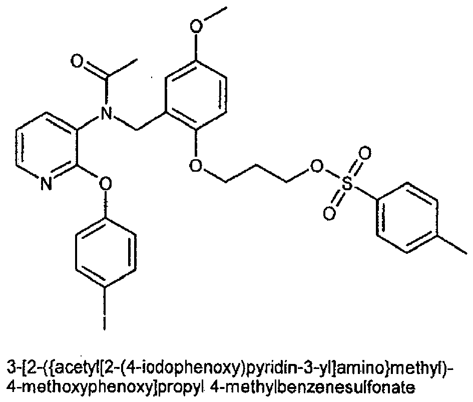 or 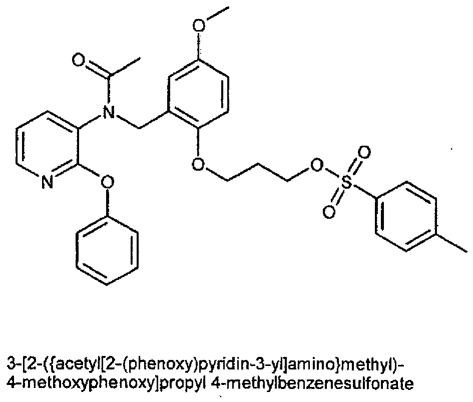 or 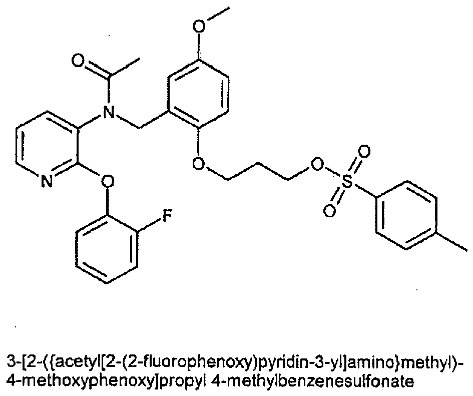 or 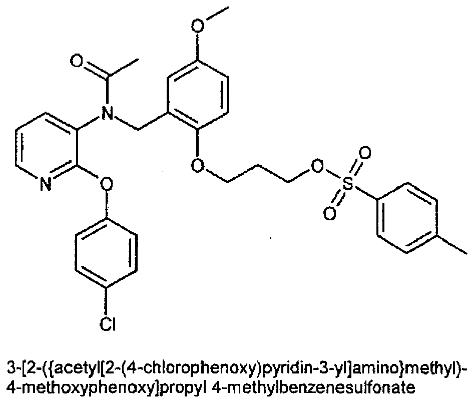 or

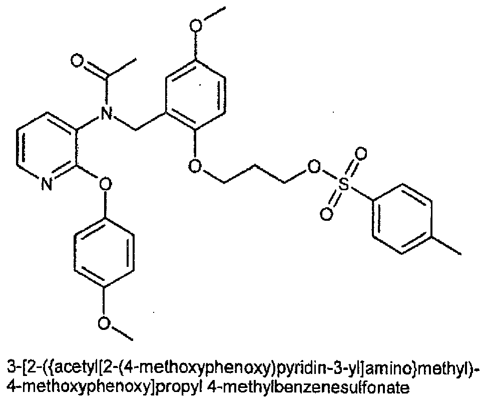

4. A compound according to any of the foregoing claims having the formula

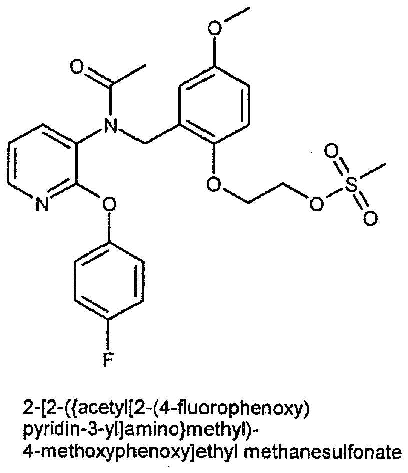

5. A compound according to any of the foregoing claims having the formula

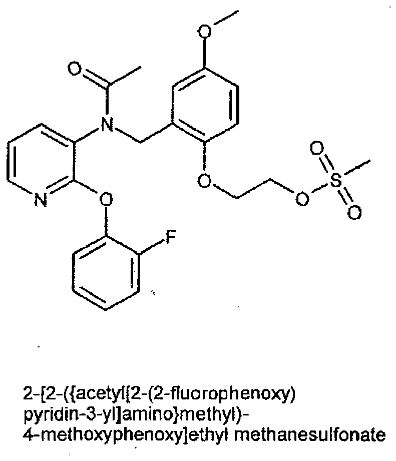

6. The compound according to any of claims 1-2, wherein L is [¹⁸F]fluoro or a compound of claim 3, 4 or 5 wherein the mesyloxy-group and the tosyloxy-group is replaced by [¹⁸F]fluoro.

7. The compound according to any of claims 1-2, wherein L is [¹⁹F]fluoro or a compound of claim 3, 4 or 5, wherein the mesyloxy-group and the tosyloxy-group is replaced by [¹⁹F]fluoro.

8. A method of synthesis of a compound as defined in claim 6 or 7, in which a compound according to claim 1-7 is reacted with an F-fluorinating agent, wherein F = ¹⁸F or ¹⁹F, and wherein L is R³.

9. The method according to claim 8, wherein said F-fluorinating agent is a compound consisting of F-anions, preferably a compound selected from the group consisting of 4, 7, 13, 16, 21, 24-hexaoxa-1,10-diazabicyclo[8.8.8]-hexacosane K F, i.e. crownether salt Kryptofix KF, KF, HF, KH F₂, CsF, NaF and tetraalkylammonium salts of F, such as N(butyl)₄F (tetrabutylammonium fluoride), and wherein F = ¹⁸F or ¹⁹F.

10. A compound of formula VI 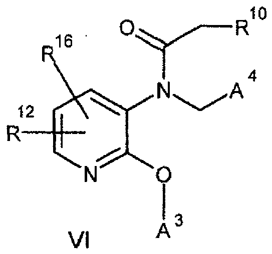 wherein
**R¹⁰** is selected from the group consisting of (C₁-C₆)alkyl and hydrogen;
**R¹⁶** is selected from the group consisting of hydrogen, halo, trifluoromethyl, (C₁-C₅)alkyl, (C₂-C₅)alkynyl), (C₂-C₅)alkenyl and (C₁-C₅)alkoxy;
**A³** and **A⁴** are the same or different and of the structure (R¹²)(R⁴)(R⁵)phenyl;
**R¹²** is selected from the group consisting of **R¹³** and hydrogen;
**R¹³** is hydroxy,
with the proviso that compounds of formula VI contain exactly one **R¹³;**
**R⁴** and **R⁵** are independently and individually, at each occurrence, selected from the group consisting of hydrogen, halo, trifluoromethyl, (C₁-C₆)alky), (C₂-C₅)alkynyl), (C₂-C₅)alkenyl and (C₁-C₅)alkoxy;
and tautomeric, enantiomeric, diastereoisomeric forms as well as racemic mixtures thereof,
and any pharmaceutically acceptable salt thereof.

11. A composition consisting of a compound according to any of claims 1-7 and a pharmaceutically acceptable carrier or diluent.

12. A compound according to any of claims 1-7, or a composition according to claim 11 as a pharmaceutical or diagnostic agent or imaging agent.

13. A compound according to claim 6 or a composition according to claim 11, wherein the composition comprises a compound according to claim 6 for use as a diagnostic agent or imaging agent, in particular for diseases of the central nervous system.

14. A compound according to any of the foregoing claims which is selected from the group consisting of compounds having the following structures 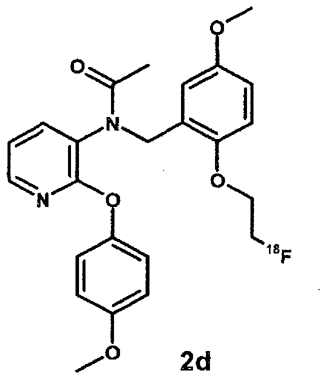 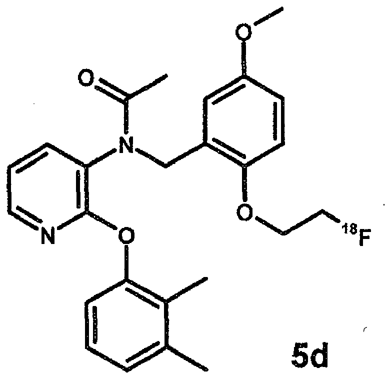 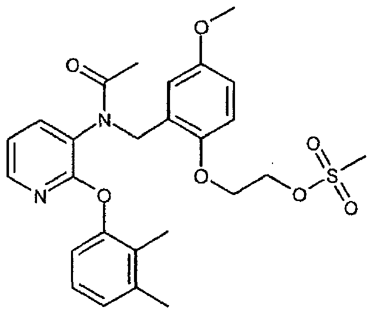 and

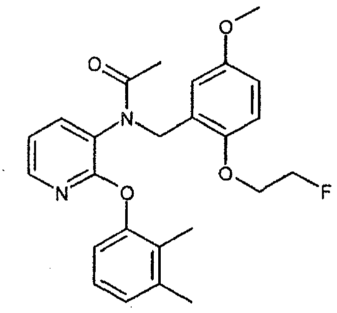

15. A compound according to any of the foregoing claims having the structure

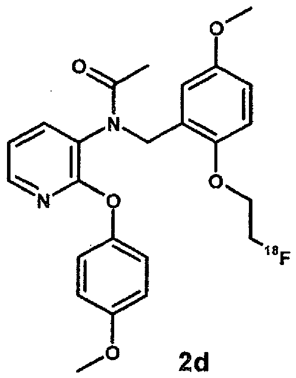

16. A compound according to any of the foregoing claims having the structure

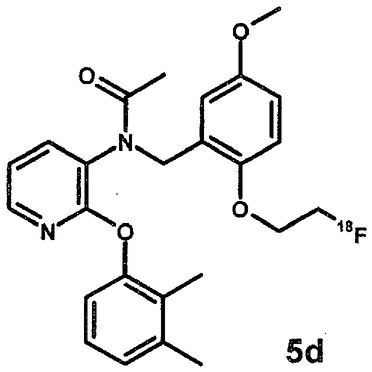

17. A pharmaceutical or diagnostic composition comprising a compound as defined by claims 15 or 16.

18. A kit, containing a sealed vial comprising a compound as defined by claim 14, 15 or 16 or a pharmaceutical composition according to claim 17.

## Patentansprüche

1. Verbindung der Formel 1 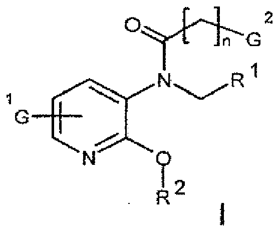 wobei
**R¹** und **R²** bei jedem Vorkommen unabhängig und einzeln ausgewählt werden aus der Gruppe bestehend aus (G³)Aryl, substituiertem (G³)Aryl, (G³-(C₁-C₈)Alkyl)aryl, (G³-(C₁-C₈)Alkoxy)aryl, (G³-(C₂-C₈)Alkynyl)aryl, (G³-(C₂-C₈)Alkenyl)aryl, substituiertem (G³-(C₁-C₈)Alkyl)aryl, substituiertem (G³-(C₁-C₈)Alkoxy)aryl, subsitutiertem (G³-(C₂-C₈)Alkynyl)aryl und subsitutiertem (G³-(C₂-C₈)Alkenyl)aryl;
**G¹, G²** und **G³** bei jedem Vorkommen unabhängig und einzeln ausgewählt werden aus der Gruppe bestehend aus Wasserstoff und **L,**
mit der Maßgabe, dass Verbindungen der Formel I genau ein **L** enthalten;
**L** ausgewählt wird aus der Gruppe bestehend aus **R³,** [¹⁸F]Fluor und [¹⁹F]Fluor;
**R³** eine Abgangsgruppe ist, die ausgewählt wird aus der Gruppe bestehend aus -I⁺(Aryl)(X⁻), -I⁺(Heteroaryl)(X⁻), Nitro, -N⁺(Me)₃(X⁻), Halogene, insbesondere Chlor, Brom und Iod, Mesyloxy, Tosyloxy, Trifluormethylsulfonyloxy, Nonafluorbutylsulfonyloxy, (4-Bromphenyl)sulfonyloxy, (4-Nitrophenyl)sulfonyloxy, (2-Nitrophenyl)sulfonyloxy, (4-Isopropylphenyl)sulfonyloxy, (2,4,6-Tri-lsopropylphenyl)sulfonyloxy, (2,4,6-Trimethylphenyl)sulfonyloxy, (4-*Tert*butylphenyl)sulfonyloxy und (4-Methoxy-phenyl)sulfonyloxy;
wobei **X⁻** ausgewählt wird aus der Gruppe bestehend aus einem Anion einer anorganischen Säure und einem Anion einer organischen Säure:
wobei n eine Ganzzahl von 0 bis 6 ist;
und tautomere, enantiomere, diastereoisomere Formen sowie racemische Mischungen davon;
und ein beliebiges pharmazeutisch verträgliches Salz davon.

2. Verbindung gemäß Anspruch 1, wobei **X⁻** ausgewählt wird aus der Gruppe bestehend aus CF₃S(O)₂C⁻, C₄F₉S(O)₂O⁻, CF₃COO⁻, H₃CCOO⁻, Iodid-Anion, Bomid-Anion, Chlorid-Anion, Perchlorat-Anion (ClO₄⁻) und Phosphat-Anion.

3. Verbindung gemäß einem der vorhergehenden Ansprüche, die ausgewählt wird aus der Gruppe von Verbindungen bestehend aus 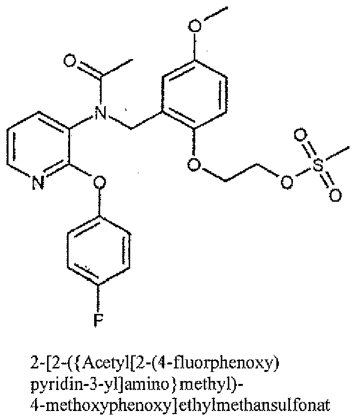 oder 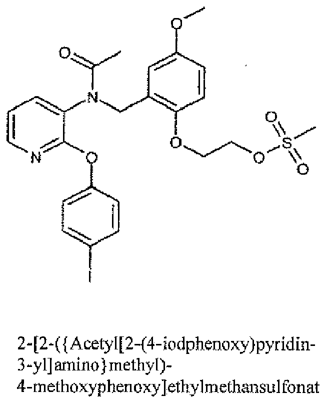 oder 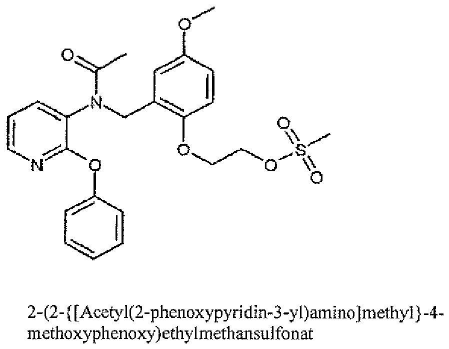 oder 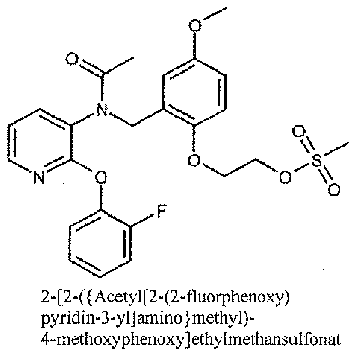 oder 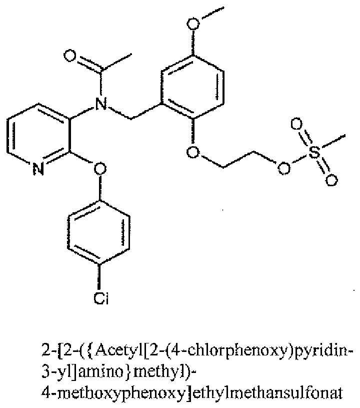 oder 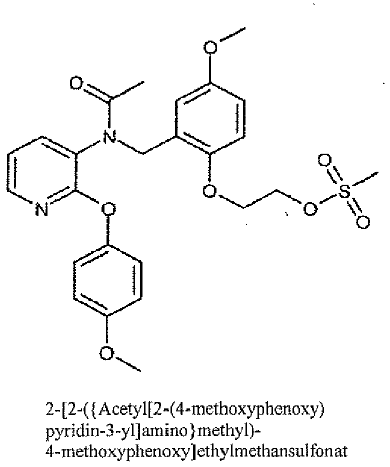 oder 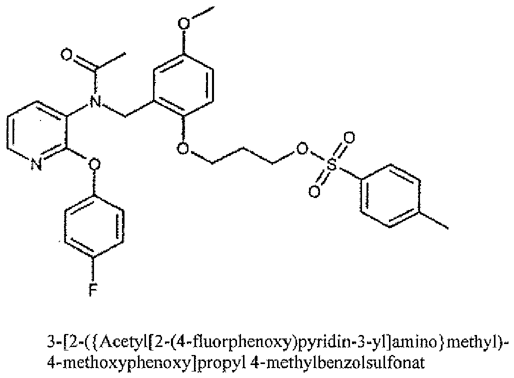 oder 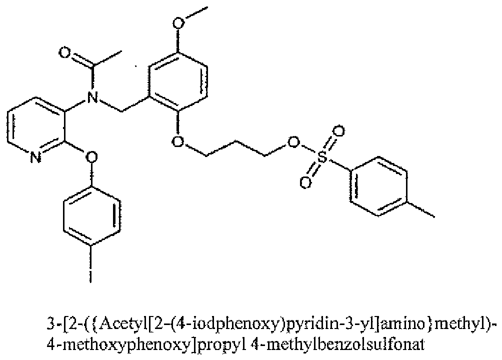 oder 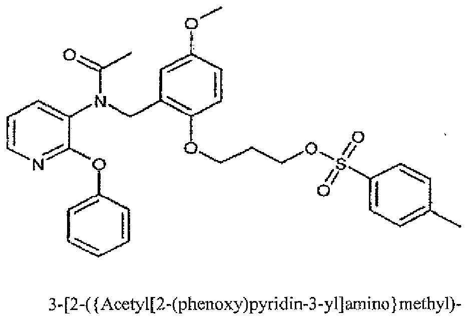 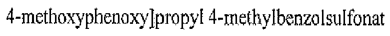 oder 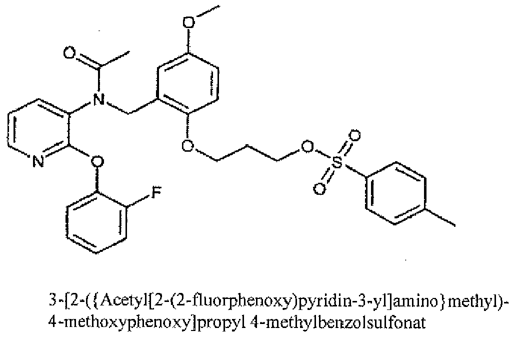 oder 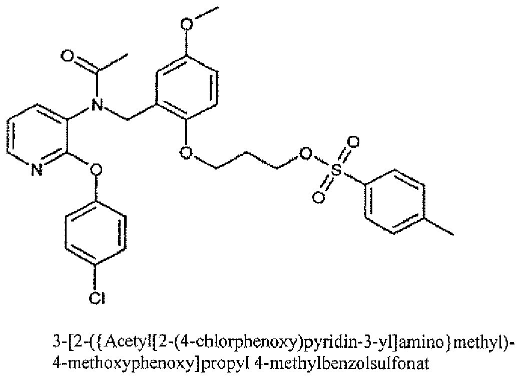 oder

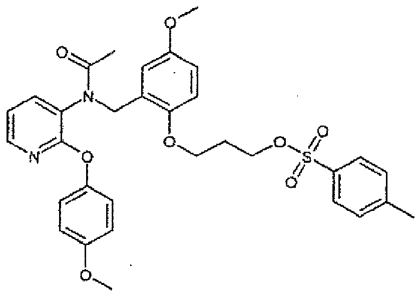

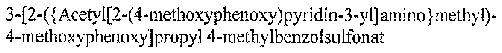

4. Verbindung gemäß einem der vorhergehenden Ansprüche mit der Formel

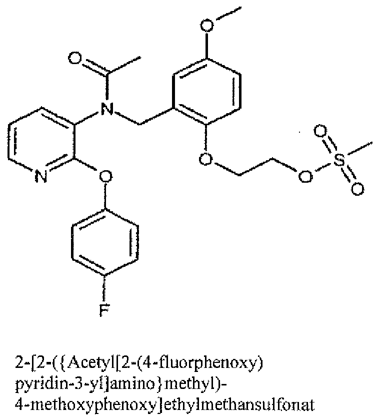

5. Verbindung gemäß einem der vorhergehenden Ansprüche mit der Formel

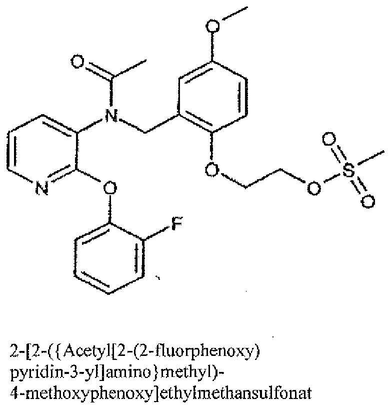

6. Verbindung gemäß einem der Ansprüche 1 bis 2, wobei L [¹⁸F]fluor oder eine Verbindung gemäß Anspruch 3, 4 oder 5 ist, wobei die Mesyloxy-Gruppe und die Tosyloxy-Gruppe durch [¹⁸F]fluor ersetzt wird.

7. Verbindung gemäß einem der Ansprüche 1 bis 2, wobei L [¹⁹F]fluor oder eine Verbindung gemäß Anspruch 3, 4 oder 5 ist, wobei die Mesyloxy-Gruppe und die Tosyloxy-Gruppe durch [¹⁹F]fluor ersetzt wird.

8. Syntheseverfahren einer Verbindung wie in Anspruch 6 oder 7 definiert, in welchem eine Verbindung gemäß Anspruch 1 bis 7 mit einem F-Fluorierungsmittel zur Reaktion gebracht wird, wobei F = ¹⁸F oder ¹⁹F, und wobei L R³ ist.

9. Verfahren gemäß Anspruch 8, wobei das F-Fluorierungsmittel eine Verbindung bestehend aus F-Anionen ist, vorzugsweise eine Verbindung ausgewählt aus der Gruppe bestehend aus 4, 7, 13, 16, 21, 24-Hexaoxa-1,10-diazabicyclo[8.8.8]-hexacosan K F, d.h. Kronenether-Salz Kryptofix KF, KF, HF, KH F₂, CsF, NaF und Tetraalkylammoniumsalze von F, wie N(Butyl)₄F (Tetrabutylammoniumfluorid), und wobei F = ¹⁸F oder ¹⁹F.

10. Verbindung der Formel VI 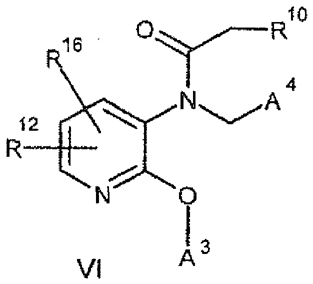 wobei
**R¹⁰** ausgewählt wird aus der Gruppe bestehend aus (C₁-C₆)Alkyl und Wasserstoff;
**R¹⁶** ausgewählt wird aus der Gruppe bestehend aus Wasserstoff, Halo, Trifluormethyl, (C₁-C₅)Alkyl, (C₂-C₅)Alkynyl), (C₂-C₅)Alkenyl und (C₁-C₅)Alkoxy;
**A³** und **A⁴** gleich oder verschieden sind und die Struktur (R¹²)(R⁴)(R⁵)Phenyl aufweisen;
**R¹²** ausgewählt wird aus der Gruppe bestehend aus **R¹³** und Wasserstoff;
**R¹³** Hydroxy ist,
mit der Maßgabe, dass Verbindungen der Formel VI genau ein **R¹³** enthalten;
**R⁴** und **R⁵** bei jedem Vorkommen unabhängig und einzeln ausgewählt werden aus der Gruppe bestehend aus Wasserstoff, Halo, Trifluormethyl, (C₁-C₅)Alkyl, (C₂-C₅)Alkynyl), (C₂-C₅)Alkenyl und (C₁-C₅)Alkoxy;
und tautomere, enantiomere, diastereoisomere Formen sowie racemische Mischungen davon, und ein beliebiges pharmazeutisch verträgliches Salz davon.

11. Zusammensetzung bestehend aus einer Verbindung gemäß einem der Ansprüche 1 bis 7 und einem pharmazeutisch verträglichen Träger oder Verdünnungsmittel.

12. Verbindung gemäß einem der Ansprüche 1 bis 7, oder eine Zusammensetzung gemäß Anspruch 11 als pharmazeutisches oder diagnostisches Mittel oder Bildgebungsmittel.

13. Verbindung gemäß Anspruch 6 oder eine Zusammensetzung gemäß Anspruch 11, wobei die Zusammensetzung eine Verbindung gemäß Anspruch 6 zur Verwendung als diagnostisches Mittel oder Bildgebungsmittel, insbesondere für Erkrankungen des zentralen Nervensystems, umfasst.

14. Verbindung gemäß einem der vorhergehenden Ansprüche, die ausgewählt wird aus der Gruppe bestehend aus Verbindungen mit den folgenden Strukturen 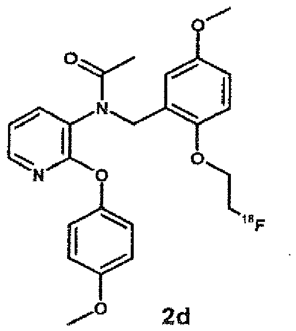 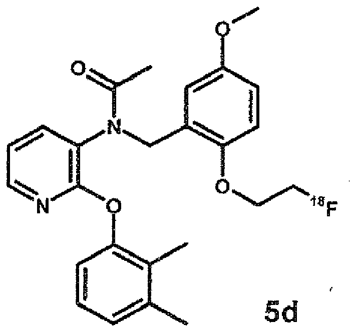 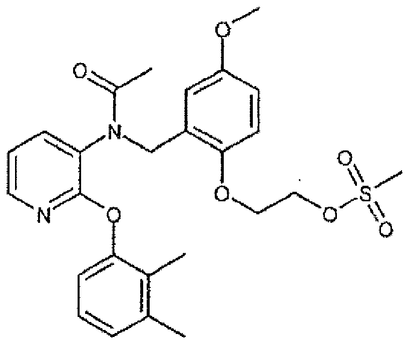 und

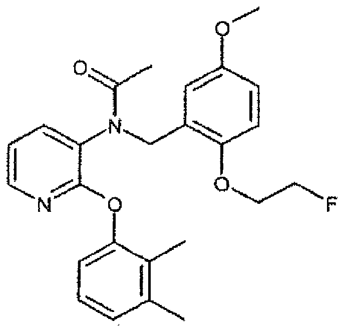

15. Verbindung gemäß einem der vorhergehenden Ansprüche mit der Struktur

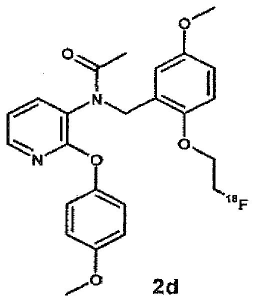

16. Verbindung gemäß einem der vorhergehenden Ansprüche mit der Struktur

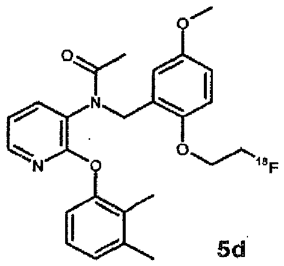

17. Pharmazeutische oder diagnostische Zusammensetzung, umfassend eine Verbindung wie durch Anspruch 15 oder 16 defniert.

18. Ein Kit, das ein versiegeltes Glasfläschchen enthält, umfassend eine Verbindung wie durch Anspuch 14, 15 oder 15 defniniert oder eine pharmazeutische Zusammensetzung gemäß Anspruch 17.

## Revendications

1. Composé de formule I 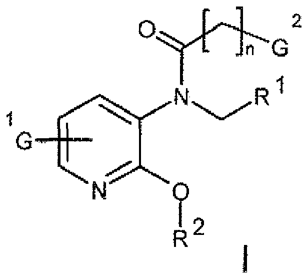 dans laquelle
R¹ et R² sont indépendamment et individuellement, à chaque occurrence, choisis dans le groupe constitué des groupes (G³) aryle, (G³) aryle substitué, (G³- (C₁-C₈) alkyl) aryle, (G³-(C₁-C₈)alcoxy)aryle, (G³-(C₂-C₈)alcynyl)aryle, (G³ - (C₂-C₈) alcényl) aryle, (G³ - (C₁-C₈)alkyl)aryle substitué, (G³-(C₁-C₈)alcoxy)aryle substitué, (G³- (C₂-C₈) alcynyl) aryle substitué et (G³-(C₂-C₈)alcényl)aryle substitué ;
G¹, G² et G³ sont indépendamment et individuellement, à chaque occurrence, choisis dans le groupe constitué de l'atome d'hydrogène et de L,
à condition que les composés de formule I contiennent exactement un L ;
L est choisi dans le groupe constitué des groupes R³, [¹⁸F] fluoro et [¹⁹F] fluoro ;
R³ représente un groupe partant choisi dans le groupe constitué des groupes -I⁺(aryl)(X⁻), - I⁺(hétéroaryl) (X⁻), nitro, -N⁺(Me)₃(X⁻), halogéno, en particulier chloro, bromo, et iodo, mésyloxy, tosyloxy, trifluorméthylsulfonyloxy, nona-fluorobutylsulfonyloxy, (4-bromo-phényl)sulfonyloxy, (4-nitro-phényl)sulfonyloxy, (2-nitro-phényl)sulfonyloxy, (4-isopropyl-phényl)sulfonyloxy, (2,4,6-tri-isopropyl-phényl)sulfonyloxy, (2,4,6-triméthyl-phényl)sulfonyloxy, (4-tertbutyl-phényl)sulfonyloxy et (4-méthoxy-phényl)sulfonyloxy ;
dans laquelle X⁻ est choisi dans le groupe constitué de l'anion d'un acide inorganique et de l'anion d'un acide organique ;
dans laquelle n représente un nombre entier de 0 à 6 ;
et les formes tautomères, énantiomères, diastéréoisomères ainsi que les mélanges racémiques de ceux-ci,
et tout sel pharmaceutiquement acceptable de ceux-ci.

2. Composé selon la revendication 1, dans lequel X⁻ est choisi dans le groupe constitué du CF₃S(O)₂O⁻, du C₄F₉S(O)₂O⁻, du CF₃COO⁻, du H₃CCOO⁻, d'un anion iodure, d'un anion bromure, d'un anion chlorure, d'un anion perchlorate (ClO₄⁻) et d'un anion phosphate.

3. Composé selon l'une quelconque des revendications précédentes qui est choisi dans le groupe de composés constitué de 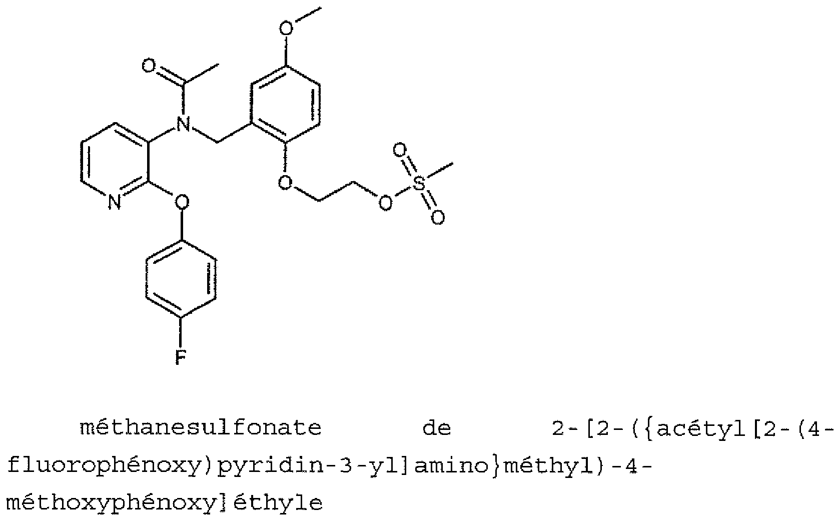 ou de 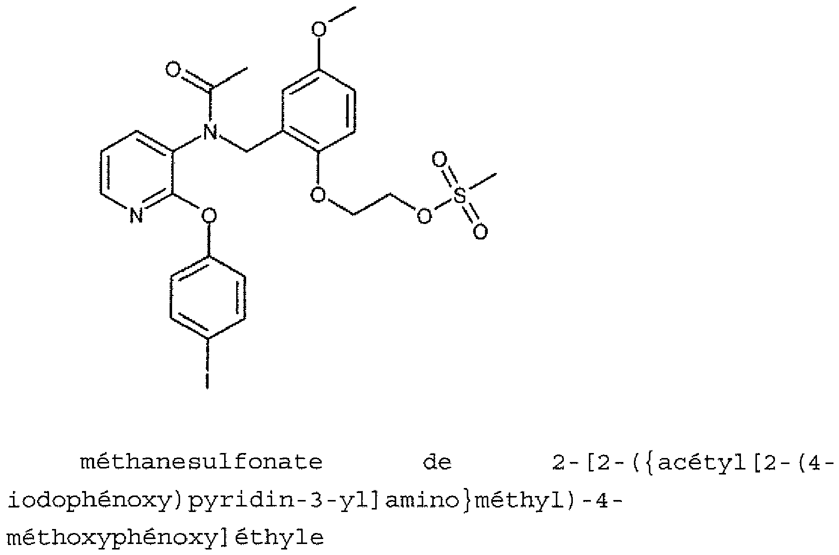 ou de 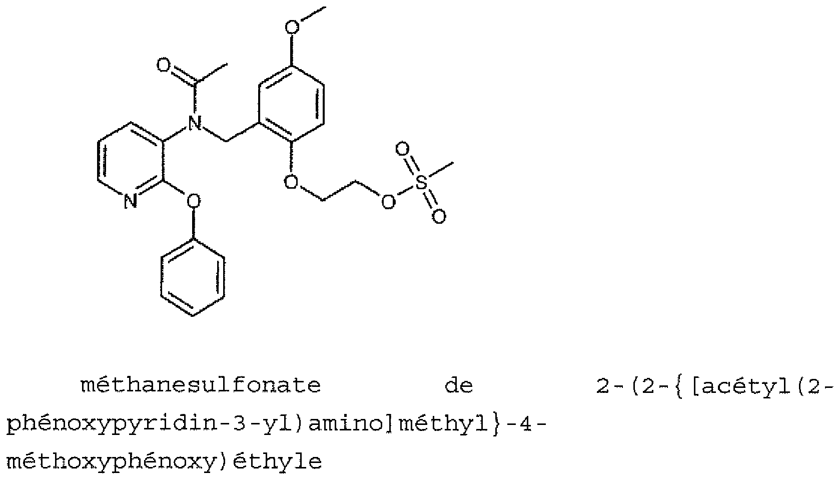 ou de 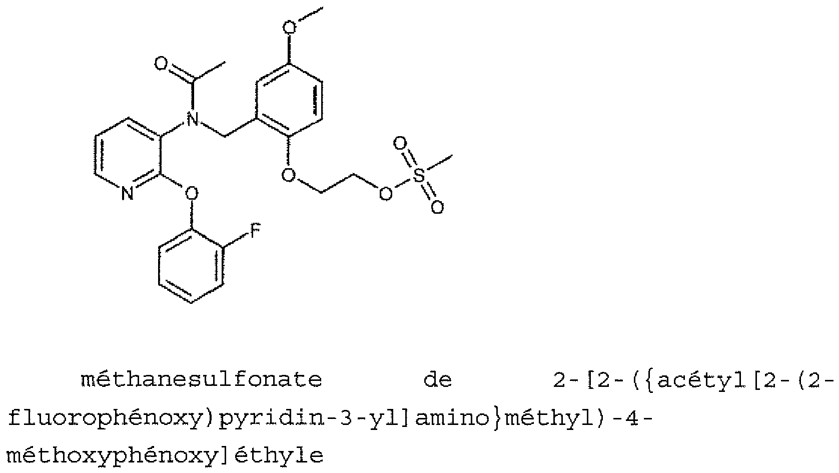 ou de 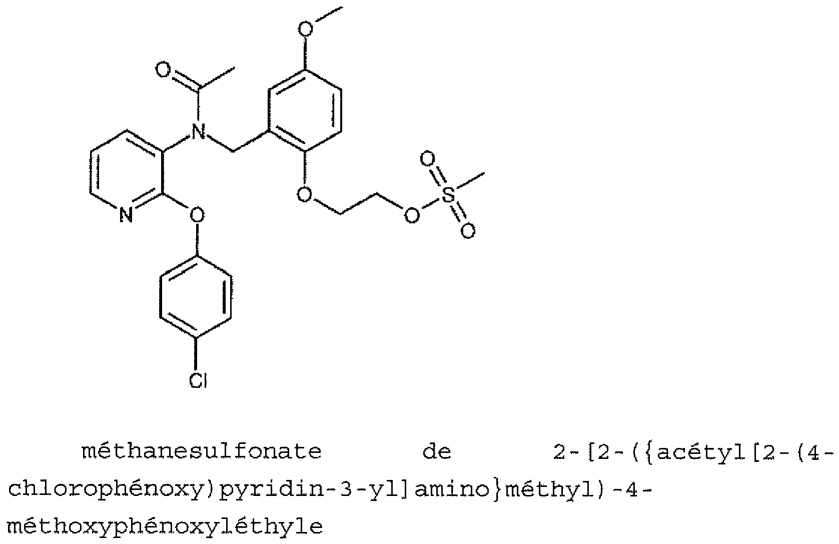 ou de 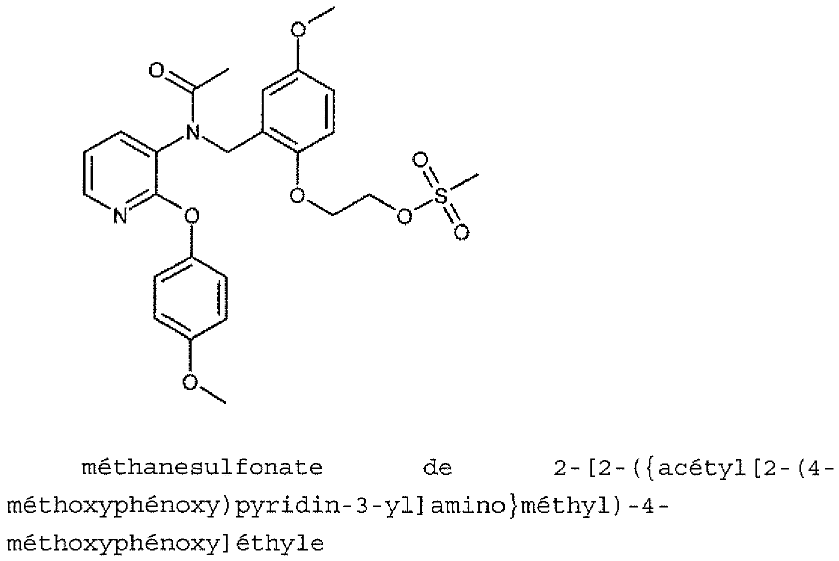 ou de 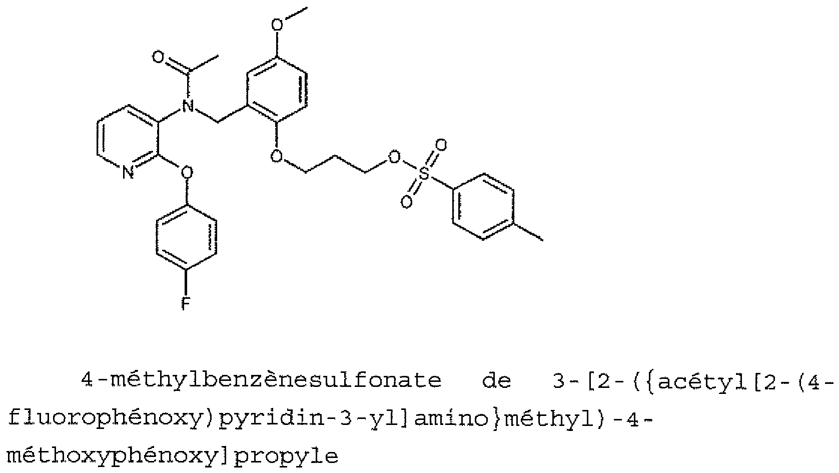 ou de 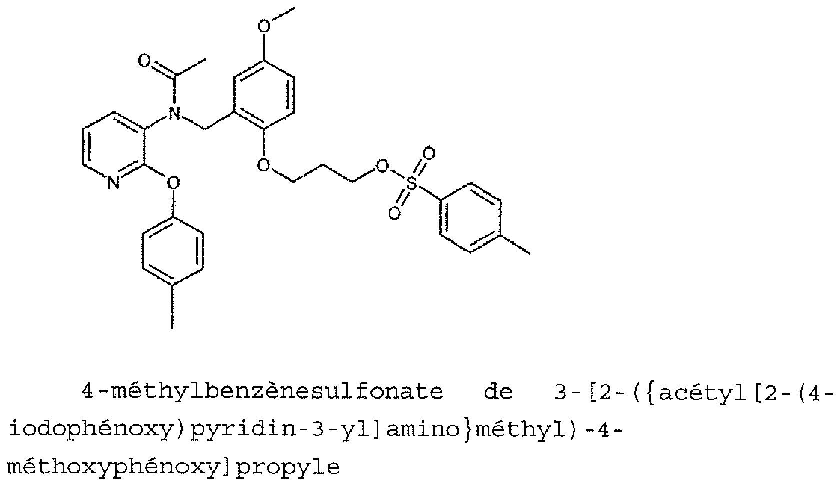 ou de 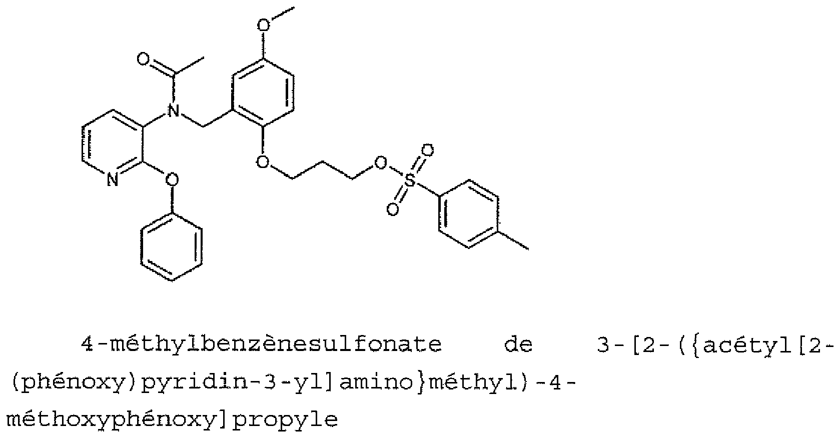 ou de 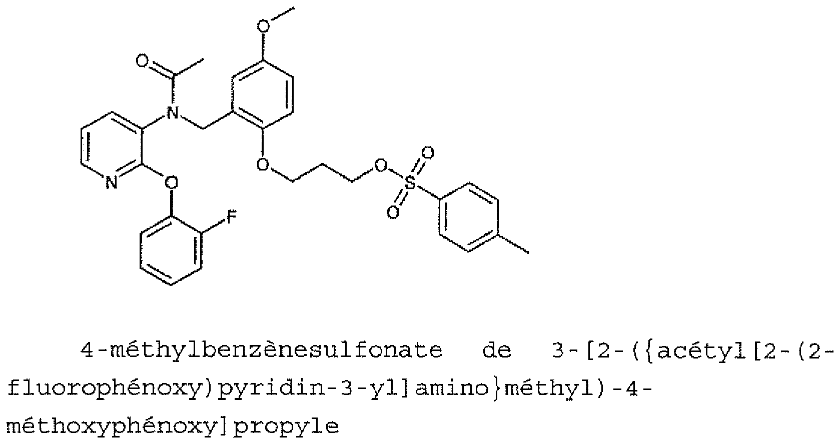 ou de 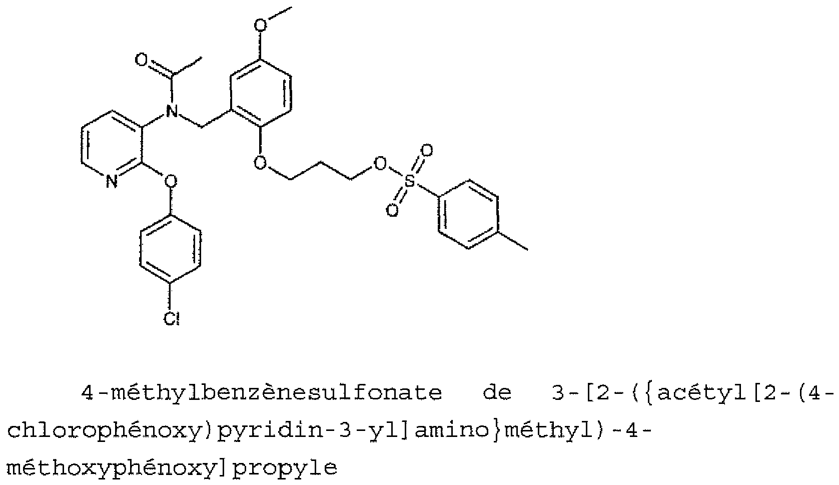 ou de

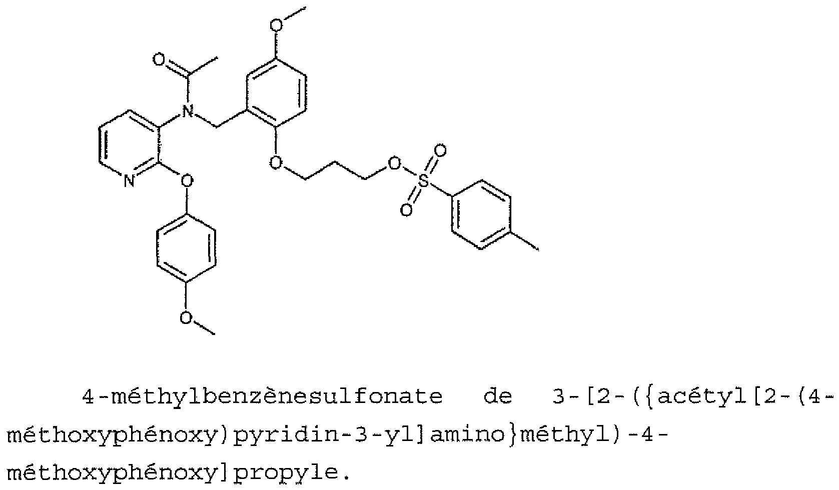

4. Composé selon l'une quelconque des revendications précédentes répondant à la formule

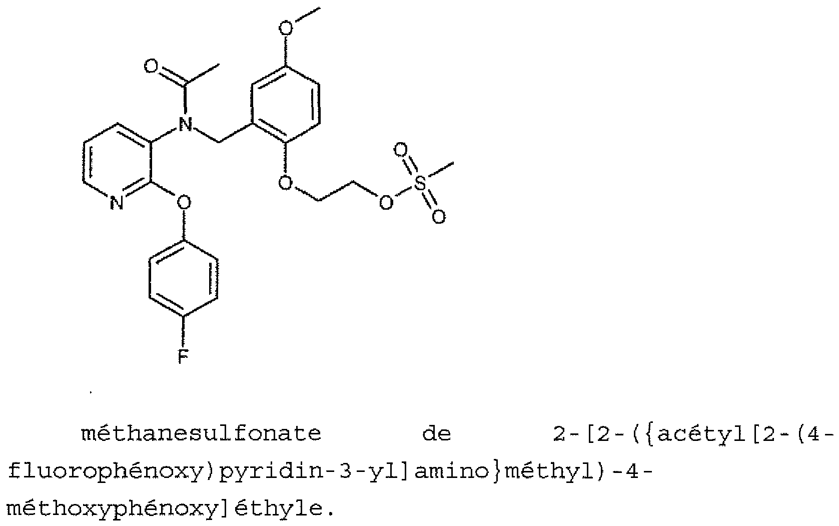

5. Composé selon l'une quelconque des revendications précédentes répondant à la formule

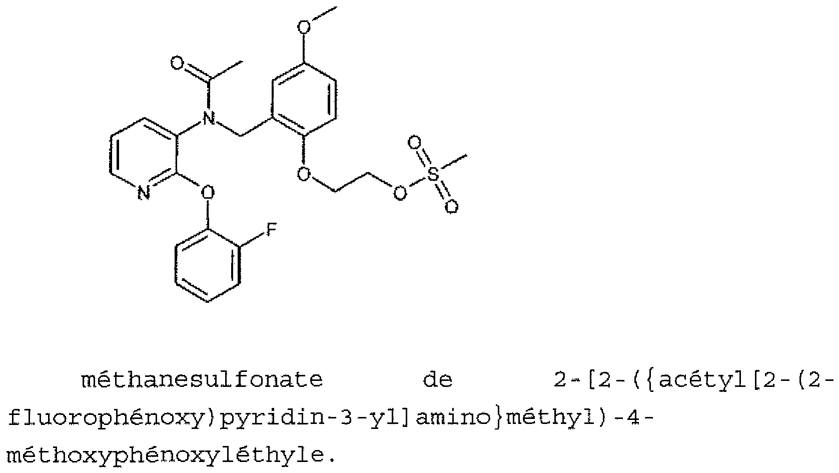

6. Composé selon l'une quelconque des revendications 1 à 2, dans lequel L représente un groupe [¹⁸F]fluoro ou composé selon la revendication 3, 4 ou 5 dans lequel le groupe mésyloxy et le groupe tosyloxy sont remplacés par un groupe [¹⁸F]fluoro.

7. Composé selon l'une quelconque des revendications 1 à 2, dans lequel L représente un groupe [¹⁹F]fluoro ou composé selon la revendication 3, 4 ou 5, dans lequel le groupe mésyloxy et le groupe tosyloxy sont remplacés par un groupe [¹⁹F]fluoro.

8. Procédé de synthèse d'un composé tel que défini dans la revendication 6 ou 7, dans lequel un composé selon l'une des revendications 1 à 7 est mis à réagir avec un agent de fluoration de F, dans lequel F = ¹⁸F ou ¹⁹F, et dans lequel L représente R³.

9. Procédé selon la revendication 8, dans lequel ledit agent de fluoration de F est un composé constitué d'anions de F, de préférence un composé choisi dans le groupe constitué du 4, 7, 13, 16, 21, 24-hexaoxa-1,10-diazabicyclo[8.8.8]-hexacosane K F, à savoir un sel d'éther-couronne Kryptofix KF, KF, HF, KH F₂, CsF, NaF et des sels de tétraalkylammonium de F, tels que le N(butyl)₄F (fluorure de tétrabutylammonium), et dans lequel F = ¹⁸F ou ¹⁹F.

10. Composé de formule VI 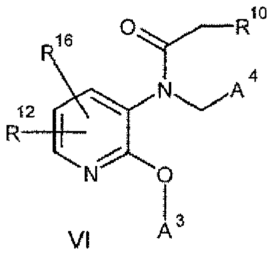 dans laquelle
R¹⁰ est choisi dans le groupe constitué d'un groupe (C₁-C₆)alkyle et de l'atome d'hydrogène ;
R¹⁶ est choisi dans le groupe constitué de l'atome d'hydrogène, des groupes halogéno, trifluorométhyle, (C₁-C₅) alkyle, (C₂-C₅) alcynyle, (C₂-C₅)alcényle et (C₁-C₅)alcoxy ;
A³ et A⁴ sont identiques ou différents et ont la structure (R¹²) (R⁴) (R⁵) phényle ;
R¹² est choisi dans le groupe constitué de R¹³ et de l'atome d'hydrogène ;
R¹³ représente un groupe hydroxy,
à condition que les composés de formule VI contiennent exactement un R¹³ ;
R⁴ et R⁵ sont indépendamment et individuellement, à chaque occurrence, choisis dans le groupe constitué de l'atome d'hydrogéne, des groupes halogéno, trifluorométhyle, (C₁-C₅)alkyle, (C₂-C₅)alcynyle, (C₂-C₅) alcényle et (C₁-C₅)alcoxy ;
et les formes tautomères, énantiomères, diastéréoisomères ainsi que les mélanges racémiques de ceux-ci,
et tout sel pharmaceutiquement acceptable de ceux-ci.

11. Composition constituée d'un composé selon l'une quelconque des revendications 1 à 7 et d'un vecteur ou diluant pharmaceutiquement acceptable.

12. Composé selon l'une quelconque des revendications 1 à 7, ou composition selon la revendication 11 en tant qu'agent pharmaceutique ou agent de diagnostique ou agent d'imagerie.

13. Composé selon la revendication 6 ou composition selon la revendication 11, dans lequel la composition comprend un composé selon la revendication 6 pour une utilisation en tant qu'agent de diagnostique ou agent d'imagerie, en particulier pour les maladies du système nerveux central.

14. Composé selon l'une quelconque des revendications précédentes qui est choisi dans le groupe constitué des composés présentant les structures suivantes 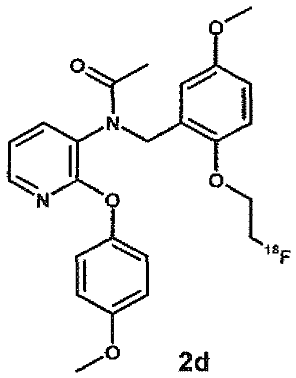 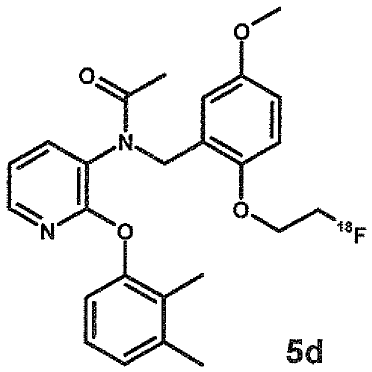 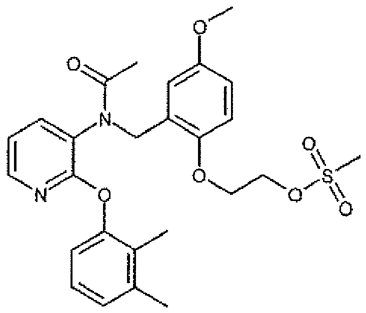 et

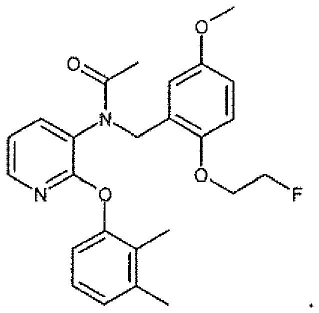

15. Composé selon l'une quelconque des revendications précédentes présentant la structure

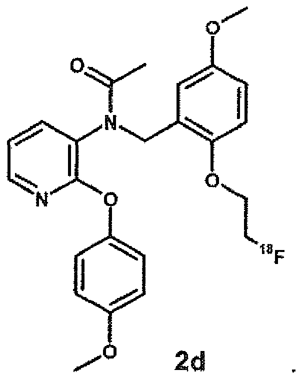

16. Composé selon l'une quelconque des revendications précédentes présentant la structure

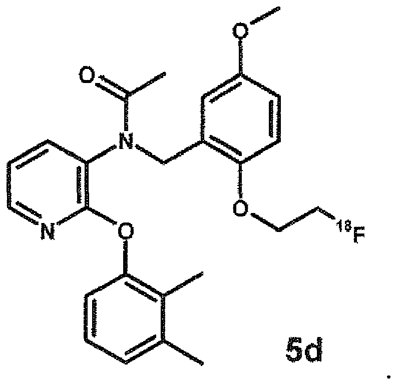

17. Composition pharmaceutique ou diagnostique comprenant un composé tel que défini par les revendications 15 ou 16.

18. Kit, contenant une fiole fermée hermétiquement comprenant un composé tel que défini par la revendication 14, 15 ou 16 ou une composition pharmaceutique selon la revendication 17.
